# EUROPÄISCHE PATENTSCHRIFT

(11) **EP 4 398 801 B1**
(45) Veröffentlichungstag und Bekanntmachung des Hinweises auf die Patenterteilung: **18.03.2026**
(21) Anmeldenummer: 22765582.6
(22) Anmeldetag: 05.09.2022
(51) Int. Cl.: A61B 5/271, A61B 5/259, A61B 5/00, A61B 5/18, A61B 5/12

(54) **ELEKTRODENANORDNUNG ZUR MESSUNG VON BIOPOTENTIALEN AN EINEM KOPF EINES MENSCHEN**
ELECTRODE ARRANGEMENT FOR MEASURING BIOPOTENTIALS ON A PERSON'S HEAD
ENSEMBLE D'ÉLECTRODES DESTINÉ À MESURER DES POTENTIELS BIOLOGIQUES SUR LA TÊTE D'UN ÊTRE HUMAIN

(30) Priorität: 06.09.2021 DE 102021209778
(43) Veröffentlichungstag der Anmeldung: 17.07.2024
(73) Patentinhaber: Fraunhofer-Gesellschaft zur Förderung der angewandten Forschung e.V., 80686 München (DE)
(72) Erfinder: WOLF, Karen Insa, 98693 Ilmenau (DE); PÄTZOLD, Wiebke, 98693 Ilmenau (DE); DA SILVA SOUTO, Carlos Filipe, 98693 Ilmenau (DE); DEBENER, Stefan, 98693 Ilmenau (DE); PAUL, Marina, 48703 Stadtlohn (DE)
(74) Vertreter: König, Andreas Rudolf
(86) Internationale Anmeldenummer: PCT/EP2022/074614
(87) Internationale Veröffentlichungsnummer: WO 2023/031457

(56) Entgegenhaltungen:
- EP-A1- 1 249 204
- EP-A1- 3 380 006
- EP-B1- 2 200 342
- EP-B1- 2 997 893
- EP-B1- 3 417 775
- WO-A1-2018/153060
- US-A1- 2010 041 962
- US-A1- 2015 238 106
- US-A1- 2018 289 274
- US-A1- 2019 223 749
- US-A1- 2020 196 900
- US-A1- 2020 383 594
- US-B1- 6 453 186
- US-B2- 8 244 340
- US-B2- 9 770 184
- YU MIKE CHI ET AL: "Dry-Contact and Noncontact Biopotential Electrodes: Methodological Review", IEEE REVIEWS IN BIOMEDICAL ENGINEERING, IEEE, USA, vol. 3, 1 January 2010 (2010-01-01), pages 106 - 119, XP011339892, ISSN: 1937-3333, DOI: 10.1109/RBME.2010.2084078
- PAUL GORDON MARK ET AL: "A Smart Textile Based Facial EMG and EOG Computer Interface", IEEE SENSORS JOURNAL, IEEE, USA, vol. 14, no. 2, 1 February 2014 (2014-02-01), pages 393 - 400, XP011532660, ISSN: 1530-437X, [retrieved on 20131126], DOI: 10.1109/JSEN.2013.2283424
- DASGUPTA ANIRBAN ET AL: "Active Sensors for the Acquisition of Physiological Signals", 2018 IEEE SENSORS, IEEE, 28 October 2018 (2018-10-28), pages 1 - 3, XP033477594, [retrieved on 20181226], DOI: 10.1109/ICSENS.2018.8589865
- SALVO P ET AL: "A 3D printed dry electrode for ECG/EEG recording", SENSORS AND ACTUATORS A: PHYSICAL, ELSEVIER BV, NL, vol. 174, 8 December 2011 (2011-12-08), pages 96 - 102, XP028443429, ISSN: 0924-4247, [retrieved on 20111217], DOI: 10.1016/J.SNA.2011.12.017
- JIANG YIZHOU ET AL: "Flexible and Stretchable Dry Active Electrodes With PDMS and Silver Flakes for Bio-Potentials Sensing Systems", IEEE SENSORS JOURNAL, IEEE, USA, vol. 21, no. 10, 24 February 2021 (2021-02-24), pages 12255 - 12268, XP011850537, ISSN: 1530-437X, [retrieved on 20210416], DOI: 10.1109/JSEN.2021.3061949
- CECOTTI HUBERT ET AL: "Single-Trial Classification of Event-Related Potentials in Rapid Serial Visual Presentation Tasks Using Supervised Spatial Filtering", IEEE TRANSACTIONS ON NEURAL NETWORKS AND LEARNING SYSTEMS, IEEE, USA, vol. 25, no. 11, 1 November 2014 (2014-11-01), pages 2030 - 2042, XP011561363, ISSN: 2162-237X, [retrieved on 20141015], DOI: 10.1109/TNNLS.2014.2302898
- MATHEWSON KYLE E. ET AL: "High and dry? Comparing active dry EEG electrodes to active and passive wet electrodes : Active dry vs. active & passive wet EEG electrodes", PSYCHOPHYSIOLOGY., vol. 54, no. 1, 20 December 2016 (2016-12-20), US, pages 74 - 82, XP093033378, ISSN: 0048-5772, Retrieved from the Internet <URL:https://api.wiley.com/onlinelibrary/tdm/v1/articles/10.1111%2Fpsyp.12536> DOI: 10.1111/psyp.12536
- KAM JULIA W.Y. ET AL: "Systematic comparison between a wireless EEG system with dry electrodes and a wired EEG system with wet electrodes", NEUROIMAGE, vol. 184, 1 January 2019 (2019-01-01), AMSTERDAM, NL, pages 119 - 129, XP093033387, ISSN: 1053-8119, DOI: 10.1016/j.neuroimage.2018.09.012

## Beschreibung

### Technisches Gebiet

Ausführungsbeispiele gemäß der Erfindung beziehen sich auf eine Vorrichtung mit einer Elektrodenanordnung, sowie ein Verfahren zur Verwendung der Vorrichtung. Die Elektrodenanordnung ist ausgebildet, um elektrische Signale ar einem Kopf eines Menschen zu messen.

Ausführungsbeispiele gemäß der Erfindung betreffen unter anderem selbstanlegbare Elektrodengridkonfigurationen, d. h. Trägerstrukturen mit Elektrodenanordnung, zur noninvasiven Langzeit-Messung von Biopotentialen am Menschen.

### Hintergrund der Erfindung

Für die Erhebung elektrophysiologischer Signale, insbesondere Elektroenzephalogramm (EEG), Elektrookulogramm (EOG), Elektrokardiogramm (EKG) und Elektromyogramm (EMG) werden Elektroden über ein Gel (Nasselektroden) oder auch ohne Gel (Trockenelektroden) in Verbindung mit der Haut eines Menschen gebracht, um eine von Gehirn und Muskelzellen generierte elektrische Aktivität zu messen. Das Aufbringen der Elektroden, insbesondere der EEG-Elektroden am Kopf, in Verbindung mit einer ausreichend hohen Signalqualität ist bisher aufwendig, insbesondere bei Nasselektroden, welche somit für eine Selbstapplikation durch einen Laien ungeeignet sind. Trockenelektroden sind für die EEG-Messung eher ungeeignet, da sie eine hohe Übergangsimpedanz haben und der Druck auf die Kopfhaut Missempfindungen und Kopfschmerzen verursacht. Im EKG-Bereich gibt es sogenannte Festgelelektroden als Nasselektrode, die wie ein Pflaster jeweils als Einzelelektrode auf der Haut leicht angebracht werden können. Die moderat viskose Gellinse hat neben der Elektrolyt-Wirkung klebende Eigenschaften, so dass die Elektrode besser an der Haut haftet als bei Nasselektroden mit Gelauftrag oder Trockenelektroden. Für die Erfassung des EEG und des EOG müssen Positionen am Kopf/im Gesicht genutzt werden (in der Nähe der Augen bzw. in der Nähe des Gehirns als Signalquelle), wobei die Positionen dabei mit einer Toleranz von wenigen cm eingehalten werden sollten, um spezifische Signal der Hirnaktivität und der Augenbewegung aufnehmen zu können.

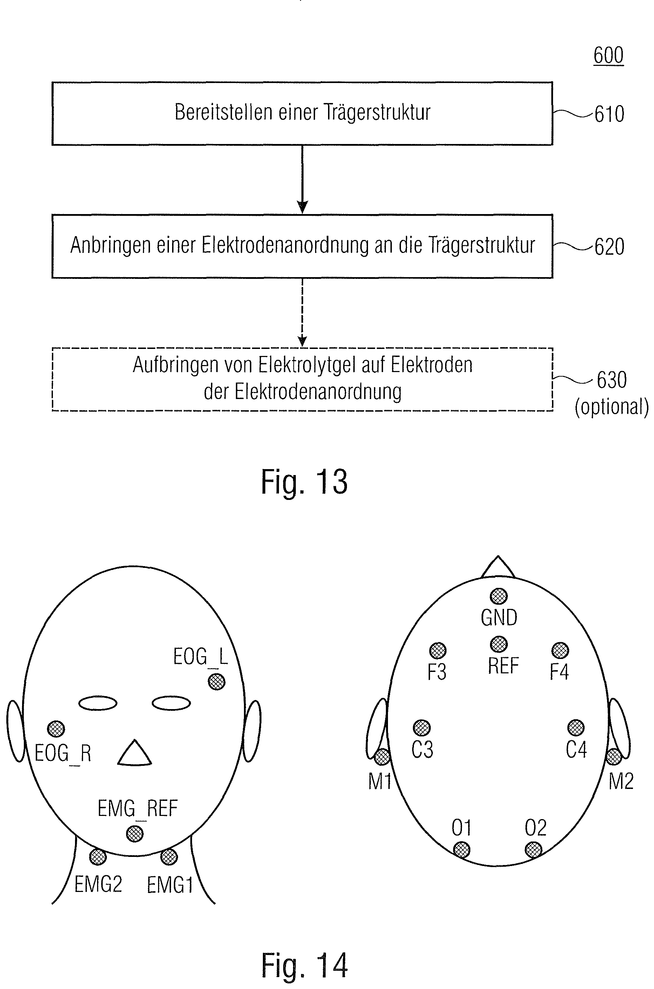

Für die Erkennung von Schlafphasen ist die Polysomnographie (PSG) das diagnostische Standardverfahren. Es werden verschiedene Biosignale kontinuierlich während des Schlafes erfasst, in der Regel stationär über Nacht in einem Schlaflabor. Es gehören dazu die Erhebung von EEG, EOG, EMG, EKG, Atemfluss, Atmungsanstrengung, Sauerstoffsättigung, Körperlage und Video. Als Elektrodenpositionen zur Erfassung von EEG, EOG und EMG werden in der Regel Positionen, wie Sie in Fig. 14 dargestellt sind, verwendet. Kleinere Abweichungen in der Konfiguration sind möglich. Fig. 14 zeigt somit eine herkömmliche Elektrodenplatzierung bei einer Polysomnographie.

EOG_R und EOG_L erfassen die Augenbewegungen. GND und REF sind notwendig zur Messung des EEG mit einem Differenzverstärker, bei dem Potentialdifferenzen in Bezug auf REF und GND ermittelt werden. F4, F3, C4, C3, O1, O2 sind Positionen der EEG-Elektroden, angelehnt an das im EEG Bereich übliche 10-20-System, wobei jeweils eine occipitale, eine zentrale und eine frontale Position mindestens zur Bestimmung der Schlafphasen notwendig sind und bei der Messung durch jeweils eine Alternativposition ergänzt werden. M1 und M2 werden zur klassischen Re-Referenzierung zum Mastoid genutzt. Die Elektroden am Kinn (EMG1, EMG_REF, EMG2) sind zur Erfassung von Muskelbewegungen bzw. Muskelspannung notwendig. Die Signale der Elektroden F4, F3, C4, C3, O1, O2 werden in Kombination und in Bezug auf REF und GND als sogenannte EEG-Kanäle für die Bestimmung der Schlafphasen verwendet. Die Platzierung aller Elektroden ist sehr zeitaufwendig und nur durch geschultes Personal möglich.

Für das EEG-Monitoring außerhalb von Klinik und Forschungslabor, bei dem sich Personen Elektroden selbst applizieren, gibt es bisher für feste Elektrodenkonfigurationen nur Trockenelektrodensysteme. Im Bereich des Schlafmonitorings gibt es Trockenelektrodenlösungen, die aber kein EOG oder EMG in der hier vorgeschlagenen Form abbilden (siehe z. B. https://dreem.com/, 22.7.2021 [Arnal, P. J., Thorey, V., Ballard, M. E., Hernandez, A. B., Guillot, A., Jourde, H., & Sauvet, F. (2019); "The Dreem headband as an alternative to polysomnography for EEG signal acquisition and sleep staging."; BioRxiv, 662734] oder https://www.philips.de/c-e/hs/sleep-solutions/smartsleep-advocacy, 22.7.2021). Nasselektroden für hohe Signalqualität werden derzeit ausschließlich von geschultem Personal angebracht und nicht von ungeschulten Personen allein. Es gibt noch Helm-/Kopfhörerlösungen, die mit feucht zu haltenden Schwämmen arbeiten.

Auf dem Markt gibt es gedruckte Elektrodensysteme:
(1) cEEGrid
   Bei diesen Elektroden muss das Gel noch direkt vor der Messung vorappliziert werden, es ist nicht möglich, sich das Grid selbst zu applizieren, dazu bedarf es einer geschulten Person. Das Trägermaterial der gedruckten Elektrode ist nur in eine Richtung flexibel, so dass es sich nicht gut einer dreidimensionalen Form (z.B. Knochenauswölbung am Kopf) anpasst. An den Rändern ist es scharfkantig, dies kann im Ohrbereich zur Beeinträchtigung des Tragekomforts führen.
   (siehe https://tmsi.wiljekoffie.dev/product/ceegrid (21.7.2021);
   Debener, S., Emkes, R., De Vos, M. & Bleichner, M.; "Unobtrusive ambulatory EEG using a smartphone and flexible printed electrodes around the ear."; Sci. Rep. 5, 16743 (2015); und
   Bleichner, M.G. & Debener, S.; "Concealed, unobtrusive ear-centered EEG acquisition: cEEGrids for transparent EEG."; Front Hum Neurosci. 11, (2017))
(2) xTrodes
   Es handelt sich um Trockenelektroden und es ist nicht bekannt, ob oder inwieweit sich das Grid selbst applizieren lässt.
   (siehe https://xtrodes.com/; WO 2017/090050 A1; und "Home monitoring of sleep with a temporary-tattoo EEG, EOG and EMG electrode array: a feasibility study." by Shustak, S., Inzelberg, L., Steinberg, S., Rand, D., Pur, M. D., Hillel, I., ... & Hanein, Y. (2019), Journal of neural engineering, 16(2), 026024)
(3) Bittium
   Das Elektrodensystem ist für die Anwendung in der medizinischen Notfallversorgung in der Produktausführung konzipiert und daher nicht unter dem Aspekt, dass eine Person sich dies selbst anlegen kann. In der unten aufgeführten Patentschrift werden aber auch weitere Anwendungen, wie das Schlafmonitoring benannt. Es wird aber nicht davon gesprochen, dass das Grid durch den Nutzer selbst applizierbar ist. Es ist eine weitere Person, wie z. B. eine geschulte Krankenschwester, involviert.
   (siehe https://shop.bittium.com/product/28/bittium-brainstatus-eeg-electrode-10pcs (21.7.2021) und US2015238106A)

In der Publikation [V. Toral et al., "Cost-Effective Printed Electrodes Based on Emerging Materials Applied to Biosignal Acquisition," in IEEE Access, vol. 8, pp. 127789-127800, 2020, doi: 10.1109/ACCESS.2020.3008945] werden Verfahrenswege mit verschiedenen Materialien zur Erstellung von gedruckten Elektrodensystemen insbesondere für Anwendung von EKG, EMG und EOG vorgestellt.

Das Dokument US 2010/041962 A1 offenbart ein Headset zur Erfassung der elektrischen Aktivität des Gehirns mit einem flexiblen Band, das mindestens einen dehnbaren Bereich aufweist, der es ermöglicht, den Abstand zwischen einem ersten Ende und einem zweiten Ende selektiv zu verändern. Das Headset kann auch flexible Schaltkreise in dem flexiblen Band aufweisen.

Das Dokument US 2020/383594 A1 offenbart Elektrodenträger für elektrophysiologische Messungen, umfassend ein Substrat und eine Vielzahl von Kontaktstellen, die an einer Substratoberfläche angebracht sind. Das Substrat hat mindestens zwei nicht dehnbare Abschnitte zur Aufnahme der Kontaktstellen, wobei die Abschnitte durch einen dehnbaren Abschnitt miteinander verbunden sind.

In Anbetracht dessen besteht ein Bedarf an einem Konzept, das eine selbst anlegbare Elektrodenanordnung ermöglicht und einen besseren Kompromiss zwischen einfacher Handhabung, hoher Positionsgenauigkeit der Elektroden an einem Kopf eines Nutzers, hoher Haftung der Elektrodenanordnung an dem Kopf des Nutzers, hoher Signalqualität und angenehmen Tragekomfort über mehrere Stunden ermöglicht.

Diese Aufgabe wird durch die unabhängigen Patentansprüche gelöst.

Erfindungsgemäße Weiterbildungen sind in den Unteransprüchen definiert.

### Zusammenfassung der Erfindung

Gemäß einem ersten Aspekt der vorliegenden Erfindung haben die Erfinder erkannt, dass ein Problem, das bei einem selbstständigen Anlegen einer Elektrodenanordnung auftritt, aus der Tatsache resultiert, dass bisher die Elektroden nicht einfach und effizient richtig von dem Nutzer an sich selbst positioniert werden können. Gemäß dem ersten Aspekt wird diese Schwierigkeit dadurch überwunden, dass für die Elektrodenanordnung eine Trägerstruktur bereitgestellt wird, die die einzelnen Elektroden der Elektrodenanordnung miteinander verbindet. Durch die Trägerstruktur werden Abstände zwischen den Elektroden und/oder Positionen der Elektroden relativ zueinander vordefiniert. Die Trägerstruktur spannt z. B. eine Matrix auf, auf der die Elektroden der Elektrodenanordnung an vordefinierten Positionen angeordnet sind. Dadurch wird eine einfache, effiziente und präzise Positionierung der Elektroden durch den Nutzer an sich selbst ermöglicht, da die Trägerstruktur eine Hilfestellung darstellt. Durch ein Anbringen der Trägerstruktur an einem Kopf oder Gesicht des Nutzers werden die an vordefinierten Positionen auf der Trägerstruktur angeordneten Elektroden automatisch innerhalb eines Toleranzbereichs richtig an dem Kopf bzw. an dem Gesicht des Nutzers positioniert. Durch das spezielle Design der Trägerstruktur, die die Elektroden der Elektrodenanordnung miteinander verbindet, wird ein hoher Tragekomfort und eine gute Handhabbarkeit erzielt. Besonders vorteilhaft ist die Vorrichtung in einem Vergleich zu einer Platzierung von Einzelelektroden, da dies mehr Aufwand bei der Installation und eine geringere Sicherheit in der Reproduzierbarkeit der Konfiguration der Elektroden bietet, als bei der hierin vorgestellten Vorrichtung. Die vorgeschlagene Lösung soll insbesondere hier Abhilfe schaffen und eine schnellere und einfachere Installation der Elektroden erlauben, insbesondere durch die Person selbst, so dass keine weitere (geschulte) Person anwesend sein muss.

Ferner haben die Erfinder herausgefunden, dass es vorteilhaft ist, wenn die Trägerstruktur eine Flexibilität aufweist, die abhängig davon ist, ob der Nutzer eine Kraft auf die Trägerstruktur ausübt oder nicht. Diese unterschiedliche Flexibilität ermöglicht eine einfache, effiziente und präzise Positionierung der Elektroden an Gesichtsstrukturen verschiedener Nutzer anpassen zu können.

So sollte die Trägerstruktur beispielsweise ohne Krafteinwirkung des Nutzers derart formstabil sein, dass sich diese unter Einwirkung der Schwerkraft nicht oder nur kaum verbiegt bzw. der Schwerkraft nicht oder nur kaum nachgibt. Die Formstabilität kann z. B. dadurch erreicht werden, dass die Trägerstruktur ein Material, wie z. B. Polyurethanschaummaterial, und eine bestimmte Dicke, z. B. in einem Bereich von 0.5 mm bis 2 mm, aufweist. Alternativ kann die Trägerstruktur ein lösbares Versteifungselement aufweisen, das ausgebildet ist, um die Form der Trägerstruktur zu stabilisieren. Die Trägerstruktur kann dabei z. B. durch das lösbare Versteifungselement für den Vorgang der Applizierung der Trägerstruktur durch den Nutzer verstärkt werden. Dies wird im Folgenden zusammenfassend auch als Trägerstruktur bezeichnet. Die Trägerstruktur spannt beispielsweise eine Matrix auf, die bei einer Ausrichtung in einer Fläche senkrecht zu der Schwerkraft sich auf beispielsweise eine Länge von 10 cm maximal um 15 cm, 10 cm, 8 cm oder 6 cm in Richtung der Schwerkraft verbiegt. Die Trägerstruktur weist somit z. B. eine hohe Steifigkeit bzw. eine geringe Nachgiebigkeit bezüglich der Schwerkraft auf. Dies basiert auf der Idee, dass die Trägerstruktur durch die Steifigkeit einfach und effizient präzise von dem Nutzer an dessen Kopf bzw. Gesicht platziert werden kann, ohne dass die Struktur in sich zusammenfällt und das Anbringen erschwert. Die Form der Trägerstruktur bzw. die relative Anordnung der Elektroden zueinander weicht unter Einwirkung der Schwerkraft auf die senkrecht zu der Schwerkraft ausgerichtete Trägerstruktur von der Form der Trägerstruktur bzw. der relativen Anordnung der Elektroden zueinander bei einer auf die Trägerstruktur wirkenden resultierenden Kraft von Null Newton (die Form der Trägerstruktur bzw. der relativen Anordnung der Elektroden bei einem Kräftegleichgewicht) maximal um 2 cm oder maximal um 3 cm ab. Ist die Trägerstruktur hingegen parallel zu der Schwerkraft ausgerichtet, so beträgt die Abweichung maximal 2 cm oder maximal 1 cm. Diese Formstabilität der Trägerstruktur ist insbesondere relevant solange die Vorrichtung nicht auf einer Kopfoberfläche des Nutzers, d. h. auf einer Gesichtsoberfläche des Nutzers und hinter einem Ohr des Nutzers, angebracht worden ist.

Unter äußerer Krafteinwirkung des Nutzers hingegen sollte die Trägerstruktur deformierbar bzw. verformbar sein, so dass die Trägerstruktur je nach Gesichtsstruktur des Nutzers angepasst werden kann, so dass z. B. auch durch Knochenstrukturen ausgebildete Unebenheiten flächig beklebt werden können und eine gute Haftung ermöglicht wird. Außerdem kann so eine Elektrodenposition einer ersten Elektrode auf einem Gesicht eines ersten Nutzers im Wesentlichen an eine Elektrodenposition der ersten Elektrode auf einem Gesicht eines zweiten Nutzers angeglichen werden. Dadurch wird sichergestellt, dass die Elektroden trotz unterschiedlicher Kopfumfänge von Nutzern an deren Gesichtern an vorbestimmten Positionen durch den jeweiligen Nutzer mit einem Toleranzbereich von bis zu 1 cm oder bis zu 2 cm angebracht werden können. Die Trägerstruktur ist z. B. derart ausgebildet, dass eine Deformation in einem Bereich von z. B. mindestens 2% bis z. B. maximal 10 % bis 20 % je nach Materialart möglich ist. Dies basiert auf der Idee, dass die Trägerstruktur dem Nutzer eine Hilfestellung bei der Positionierung auf dem Gesicht bzw. an dem Kopf des Nutzers bietet und die Trägerstruktur durch die geringe Deformierbarkeit die Positionen der Elektroden auf dem Gesicht bzw. an dem Kopf des Nutzers vorgibt und die Form der Trägerstruktur durch den Nutzer nur minimal an dessen spezifische Gesichtsstruktur angepasst werden kann. Die Trägerstruktur ist somit z. B. ausgebildet, um an eine Kopfanatomie bzw. Gesichtsanatomie des Nutzers angepasst zu werden, so dass vordefinierte Positionen der Elektroden auf der Trägerstruktur mit vordefinierten Punkten an dem Kopf bzw. an dem Gesicht des Nutzers übereinstimmen.

Dementsprechend weist eine Vorrichtung nach einem ersten Aspekt der vorliegenden Erfindung eine Trägerstruktur und eine Elektrodenanordnung mit einer Vielzahl von Elektroden zur Messung von elektrischen Signalen an einer Gesichtsoberfläche eines Kopfes eines Nutzers und ferner zur Messung von elektrischen Signalen hinter einem Ohr des Nutzers auf. Die Trägerstruktur definiert zumindest teilweise eine Relativposition der Elektroden zueinander. Die Trägerstruktur ist ohne äußere Krafteinwirkung eines Nutzers innerhalb eines Toleranzbereichs formstabil und unter äußerer Krafteinwirkung des Nutzers deformierbar, um einen Abstand zwischen zwei benachbart angeordneten Elektroden, d.h. zwei Elektroden, die über einen Steg der Trägerstruktur miteinander verbunden sind, zu verändern und sich Gesichts- und Kopfstrukturen flächendeckend, d.h. flächig aufliegend auf der jeweiligen Oberfläche, anzupassen. Ein Abstand zwischen zwei benachbart angeordneten Elektroden ist z. B. anpassbar, indem der Steg der Trägerstruktur, der die beiden Elektroden miteinander verbindet, gedehnt, gestaucht oder verdreht wird. Besonders vorteilhaft ist, dass der Nutzer durch die Deformierbarkeit der Trägerstruktur, z. B. die Trägerstruktur an Wölbungen auf dessen Gesichtsoberfläche bzw. Kopfoberfläche anpassen kann. Dadurch kann die Trägerstruktur flächig auf der jeweiligen Oberfläche angebracht werden, wodurch z. B. eine robuste Klebeverbindung zwischen der Trägerstruktur und der Gesichts- bzw. Kopfoberfläche gewährleistet wird. In anderen Worten, kann die Trägerstruktur ohne äußere Krafteinwirkung eines Nutzers innerhalb eines Toleranzbereichs formstabil sein und unter äußerer Krafteinwirkung des Nutzers deformierbar sein, um sich Gesichts- und/oder Kopfstrukturen flächendeckend anzupassen, so dass die Trägerstruktur flächig auf der Gesichtsoberfläche und/oder der Kopfoberfläche aufliegt. Die Trägerstruktur ist beispielsweise zwischen den einzelnen Elektroden der Elektrodenanordnung, z. B. flächig, in Kontakt mit der jeweiligen Oberfläche. Die Trägerstruktur ist ausgebildet, um zumindest einen Teil der Elektrodenanordnung an der Gesichtsoberfläche und einen weiteren Teil der Trägerstruktur hinter dem Ohr des Nutzers zu positionieren.

Die Elektrodenanordnung weist Nasselektroden, wie z. B. Festgelelektroden, auf. Somit handelt es sich bei den Elektroden der Vielzahl von Elektroden um Nasselektroden. In anderen Worten weist die Elektrodenanordnung eine Vielzahl von Nasselektroden auf. Nasselektroden bieten eine hochwertigere Signalqualität als Trockenelektroden, da hochviskoses Leitgel die Hautschichten besser durchdringt und somit die Impedanz reduziert. Bei der Verwendung von Nasselektroden ist das Gel, z. B. Leitgel oder Festgel, beispielsweise bereits vorappliziert, z. B. als Leitgellinse oder Festgellinse. Das vorapplizierte Leitgel oder Festgel wird beispielsweise durch ein lösbares Schutzelement, wie eine Schutzfolie, geschützt. Durch die Vorapplizierung des Gels muss dieses nicht von dem Nutzer selbst aufgetragen werden und das Anlegen der Vorrichtung durch den Nutzer selbst wird erleichtert. Ferner haben die Erfinder erkannt, dass durch die Verwendung von Nasselektroden der Tragekomfort gegenüber Trockenelektroden verbessert werden kann, da kein Druck zur Verbindung von Elektrode und Haut aufgebracht werden muss und ein deutlich niedrigeres Elektrodengewicht erreicht wird. Bei der Verwendung von Trockenelektroden, müssen diese mit einer Kraft von zumindest 1 N bis 4 N (1 Newton bis 4 Newton) oder mehr auf die Haut gedrückt werden, um Signale mit einer Impedanz im Bereich 250 bis 50 kOhm messen zu können. Bei Nasselektroden hingegen, wird die Verbindung zwischen der jeweiligen Elektrode und der Haut über ein Elektrolytgel, z. B. ein Festgel, erreicht, wodurch die Trägerstruktur ausgebildet sein kann, um die Elektroden ohne Krafteinwirkung oder nur unter geringer Krafteinwirkung auf die Haut zu drücken. Das heißt eine Kraft von maximal 0.2 N reicht aus, um Signale mit einer Impedanz unter 50 kOhm oder sehr viel kleiner aufzunehmen. Diese geringe Kraft kann beispielsweise bereits durch ein Aufkleben der Trägerstruktur mit der Elektrodenanordnung auf der Gesichtsoberfläche des Nutzers erzielt werden. Somit können mit Nasselektroden Signale mit einem besseren Signal-Rauschverhältnis als mit Trockenelektroden aufgenommen werden. Bei einer weiteren vorteilhaften Ausgestaltung sollte die Elektrodenfläche einer Elektrode der Vielzahl von Elektroden im Durchmesser kleiner als 1 cm sein, um die Vielzahl von Elektroden im Gesicht und am Kopf überhaupt platzieren zu können. Dies ist für Langzeitanwendungen über mehrere Stunden, über Nacht oder mehrere Tage vorteilhaft.

Gemäß einem Ausführungsbeispiel folgt die Trägerstruktur der Gesichtsoberfläche als einer menschlichen Gesichtsform und die Vielzahl von Elektroden sind auf der Trägerstruktur angeordnet. Die Trägerstruktur folgt z. B. der Gesichtsoberfläche im Sinne dessen, dass die Trägerstruktur eine Matrix aufspannt, die markanten Gesichtslinien folgt. Markante Gesichtslinien stellen z. B. die Augenbraue, der Backenknochen (d.h. das Jochbein), die Rundung hinter dem Ohr und der Unterkiefer dar. Die Trägerstruktur folgt z. B. der Gesichtsoberfläche im Sinne dessen, dass die Trägerstruktur eine Matrix aufspannt, die der Form der Augenbraue des Nutzers folgt und hinter dem Ohr des Nutzers der Form des Ohres folgt. Die Trägerstruktur ist z. B. ausgebildet, um die Vielzahl von Elektroden an markanten Punkten auf der Gesichtsoberfläche des Nutzers zu positionieren. Ein oberer Wangenknochen, ein Punkt vor dem Tragus des Ohres, ein Punkt auf der Stirn senkrecht oberhalb des Nasions, das Kinn und/oder das Mastoid hinter dem Ohr stellen beispielsweise markante Punkt dar. Eine erste Elektrodenposition, die einer ersten Elektrode der Vielzahl von Elektroden zugeordnet ist, stimmt z. B. mit einer Position eines ersten markanten Punktes der Gesichtsoberfläche überein; und eine zweite Elektrodenposition, die einer zweiten Elektrode der Vielzahl von Elektroden zugeordnet ist, stimmt z. B. mit einer Position eines zweiten markanten Punktes der Gesichtsoberfläche überein; und eine dritte Elektrodenposition, die einer dritten Elektrode der Vielzahl von Elektroden zugeordnet ist, ergibt sich ausgehend von der ersten Elektrodenposition und der zweiten Elektrodenposition an der Gesichtsoberfläche, z. B. an einer vorbestimmten Position, innerhalb eines durch die Trägerstruktur bestimmten Toleranzbereichs. Die erste Elektrodenposition und die zweite Elektrodenposition stellen beispielsweise Referenzpositionen für die Positionierung weiterer Elektroden der Vielzahl von Elektroden dar. Der Toleranzbereich ergibt sich beispielsweise, wie bereits oben ausgeführt, aus der Deformierbarkeit der Trägerstruktur unter Krafteinwirkung eines Nutzers. Durch die Deformierbarkeit durch den Nutzer wird ein Toleranzbereich von maximal 2 cm oder maximal 1 cm um eine Position auf der Gesichtsoberfläche der dritten Elektrode erreicht. Die Trägerstruktur ist z. B. ausgebildet, um die Vielzahl von Elektroden auf einer korrespondierenden Vielzahl von vorbestimmten Positionen, z. B. den markanten Punkten, auf der Kopfoberfläche des Nutzers, d. h. auf der Gesichtsoberfläche und/oder hinter dem Ohr, zu positionieren. Der Toleranzbereich definiert z. B. einen Bereich von maximal 2 cm oder von maximal 1 cm um eine vorbestimmte Position der jeweiligen Elektrode auf der Kopfoberfläche. Der Toleranzbereich gibt z. B. an wie reproduzierbar die Trägerstruktur die Elektroden der Elektrodenanordnung auf verschiedenen Köpfen, z. B. mit unterschiedlichen Kopfumfängen, an markanten Punkten positioniert bzw. wie genau diese markanten Punkte erreicht werden. Noch genauer muss die Elektrodenanordnung nicht positioniert werden. Innerhalb dieses Toleranzbereichs wird eine hohe Signalqualität erzielt. Die Trägerstruktur ist z. B. ausgebildet, um die dritte Elektrode mit der ersten und der zweiten Elektrode über einen Verbindungssteg zu verbinden, wobei eine Form und/oder eine Länge des Verbindungsstegs abhängig von der ersten und der zweiten Elektrodenposition ist. Optional hängt die Form und/oder die Länge des Verbindungsstegs ferner von der Gesichtsform ab. Dadurch wird bei der Form der Trägerstruktur beachtet, dass der Verbindungssteg z. B. nicht über ein Auge, ein Ohr eine Nase oder einen Mund eines Nutzers verläuft. Durch die speziell geformte Trägerstruktur und die spezielle Anordnung der Vielzahl an Elektroden auf der Trägerstruktur wird ermöglicht, dass die Elektroden mit hohem Tragekomfort und hoher Präzision auf dem Gesicht des Nutzers angeordnet werden können. Durch die Präzision bei der Positionierung wird eine hohe Signalqualität von spezifisch für den Anwendungsfall definierten Positionen an dem Kopf erreicht.

Gemäß einem Ausführungsbeispiel entspricht der erste markante Punkt der Gesichtsoberfläche einem oberen Wangenknochen und der zweite markante Punkt einer Position vor dem Tragus eines Ohrs des Nutzers. Die Wahl dieser beiden Punkte als Referenzpositionen für die Positionierung weiterer Elektroden, wie die dritte Elektrode, begründet sich darauf, dass diese beiden Punkt leicht auf der Gesichtsoberfläche zu erfühlen sind und somit durch den Nutzer sehr exakt positioniert werden können. Die Erfinder erkannten, dass mittels dieser beiden Punkte die Trägerstruktur mit der Elektrodenanordnung sehr präzise auf der Gesichtsoberfläche des Nutzers ausgerichtet werden kann. Dadurch, dass die Referenzpositionen (d.h. die erste Elektrodenposition und die zweite Elektrodenposition) charakteristischen Punkten in dem Gesicht des Nutzers entsprechen, können z. B. durch die Form der Trägerstruktur relativ zu den Referenzpunkten auf der Trägerstruktur angeordnete Elektroden der Vielzahl von Elektroden leicht auf vorbestimmten Positionen auf der Gesichtsoberfläche positioniert werden. Die restlichen Elektroden (z. B. die vierte Elektrode bis zur n-ten Elektrode, wobei die Vielzahl von Elektroden n Elektroden aufweist) der Vielzahl von Elektroden sind z. B. auf der Trägerstruktur relativ zu der ersten Elektrode und der zweiten Elektrode angeordnet. Dadurch kann die Elektrodenanordnung durch den Nutzer sehr einfach und effizient präzise auf dessen Gesichtsoberfläche angebracht werden, wodurch eine hohe Signalqualität erreicht wird.

Gemäß einem Ausführungsbeispiel umfasst die Trägerstruktur einen Verbindungssteg (z. B. einen ersten Verbindungssteg), der die erste Elektrodenposition und/oder die zweite Elektrodenposition einerseits mit der dritten Elektrodenposition andererseits zumindest teilweise verbindet und wobei die dritte Elektrodenposition innerhalb eines durch die Trägerstruktur bestimmten Toleranzbereichs von 1 bis 2 cm mit einer Position auf einer Stirn der Gesichtsoberfläche in einem Bereich von 1 cm bis 3 cm oberhalb der Augenbrauenlinie senkrecht oberhalb eines Nasions des Nutzers übereinstimmt. Die dritte Elektrodenposition sollte somit einer Position in dem Bereich von 1 cm bis 3 cm oberhalb der Augenbrauenlinie senkrecht oberhalb des Nasions des Nutzers entsprechen. Die Erfinder erkannten aber, dass ebenfalls eine hohe Signalqualität erreicht werden kann, wenn die dritte Elektrodenposition innerhalb des Toleranzbereichs von 1 bis 2 cm auf beiden Seiten senkrecht zu dem Bereich liegt. Der Toleranzbereich erstreckt sich senkrecht zu dem Bereich senkrecht oberhalb eines Nasions bzw. senkrecht zu einer Verlängerungslinie der Nasenspitze zu dem Nasion und beträgt z. B. maximal 1 cm oder maximal 2 cm. Eine optimale Signalqualität wird erreicht, wenn die dritte Elektrodenposition 2 cm oberhalb der Augenbrauenlinie senkrecht oberhalb des Nasions des Nutzers angeordnet ist. Durch den Verbindungssteg der Trägerstruktur wird die dritte Elektrodenposition vorgegeben, wodurch der Nutzer die dritte Elektrode sehr präzise auf dessen Gesichtsoberfläche anbringen kann. Dabei ist der Verbindungssteg zwar ausgelegt, um einen gewissen Toleranzbereich zuzulassen, aber dieser vermeidet gleichzeitig, dass die Elektrode von dem Nutzer außerhalb eines vorbestimmten Bereichs auf der Gesichtsoberfläche anbringt. Dadurch wird sichergestellt, dass der Nutzer die dritte Elektrode der Elektrodenanordnung einfach aufbringen kann, da der Verbindungssteg der Trägerstruktur sicherstellt, dass die dritte Elektrode so auf der Gesichtsoberfläche des Nutzers angeordnet wird, dass eine gute Signalqualität von einem spezifisch definierten Ort für das mit der dritten Elektrode gemessene elektrische Signal erzielt wird.

Gemäß einem Ausführungsbeispiel umfasst die Trägerstruktur einen Verbindungssteg (z. B. einen dritten Verbindungssteg), der die erste Elektrodenposition und/oder die zweite Elektrodenposition einerseits mit einer vierten Elektrodenposition, die einer vierten Elektrode der Vielzahl von Elektroden zugeordnet ist, andererseits zumindest teilweise verbindet und wobei die vierte Elektrodenposition innerhalb eines durch die Trägerstruktur bestimmten Toleranzbereichs mit einer Position hinter dem Ohr, wie z. B. mit einer vorbestimmten Position, wie einem Mastoid des Nutzers, übereinstimmt. Der Toleranzbereich definiert beispielsweise einen Bereich von maximal 1 cm oder maximal 2 cm um eine vorgegebene bzw. vorbestimmte Position hinter dem Ohr. Optional sind auf dem Verbindungssteg weitere Elektroden der Vielzahl von Elektroden, wie z. B. eine sechste, eine siebte, eine achte und eine neunte Elektrode, angeordnet. Die Positionen der weiteren Elektroden stimmen innerhalb des durch die Trägerstruktur bestimmten Toleranzbereichs mit einer korrespondierenden Anzahl von Position hinter dem Ohr des Nutzers überein. Die sechsten, siebten, achten und neunten Elektroden sind z. B. auf dem Verbindungssteg relativ zu der vierten Elektrodenposition angeordnet. Eine Form und/oder eine Länge des Verbindungsstegs ist z. B. abhängig von der ersten und der zweiten Elektrodenposition. Der Verbindungssteg ist z. B. ausgelegt, um die vierte Elektrodenposition relativ zu der ersten und/oder zweiten Elektrodenposition zu definieren. Der Verbindungssteg führt z. B. von einem Gesicht des Nutzers oberhalb des Ohrs des Nutzers hinter das Ohr, wobei der Verbindungssteg hinter dem Ohr der Form des Ohres folgt. Optional weisen der dritte Verbindungssteg und der erste Verbindungssteg ein gemeinsames Teilstück auf. Das gemeinsame Teilstück verzweigt sich z. B. zu dem ersten Verbindungssteg und dem dritten Verbindungssteg. An dem Punkt der Verzweigung ist z. B. eine weitere Elektrode der Vielzahl von Elektroden angeordnet.

Gemäß einem Ausführungsbeispiel umfasst die Trägerstruktur einen Verbindungssteg (z. B. einen zweiten Verbindungssteg), der die erste Elektrodenposition und/oder die zweite Elektrodenposition einerseits mit einer fünften Elektrodenposition, die einer fünften Elektrode der Vielzahl von Elektroden zugeordnet ist, andererseits zumindest teilweise verbindet und wobei die fünfte Elektrodenposition innerhalb eines durch die Trägerstruktur bestimmten Toleranzbereichs mit einer Position, z. B. einer vorbestimmten Position, auf einem Kinn der Gesichtsoberfläche des Nutzers übereinstimmt. Der Toleranzbereich definiert beispielsweise einen Bereich von maximal 1 cm oder maximal 2 cm um eine vorgegebene bzw. vorbestimmte Position auf dem Kinn. Der Verbindungssteg ist ausgebildet, um von dem Nutzer deformiert zu werden, um die fünfte Elektrodenposition an die Gesichtsstruktur des Nutzers anpassen zu können, wobei sichergestellt wird, dass die fünfte Elektrodenposition einer Position innerhalb des Toleranzbereichs um das Kinn des Nutzers entspricht. Eine Form und/oder eine Länge des Verbindungsstegs ist z. B. abhängig von der ersten und der zweiten Elektrodenposition. Der Verbindungssteg ist z. B. ausgelegt, um die fünfte Elektrodenposition relativ zu der ersten und/oder zweiten Elektrodenposition zu definieren.

Gemäß einem Ausführungsbeispiel weist die Trägerstruktur ein Klebematerial zur Fixierung der Vielzahl von Elektroden und der Trägerstruktur an der Gesichtsoberfläche auf. Optional ist das Klebematerial von einer lösbaren Schutzfolie bedeckt. Gemäß einem Ausführungsbeispiel weisen ferner die Vielzahl von Elektroden ein klebendes Elektrolytgel zur Fixierung der Vielzahl von Elektroden und der Trägerstruktur an der Gesichtsoberfläche auf. Das klebende Elektrolytgel ist zumindest bereichsweise von einer lösbaren Schutzfolie bedeckt. Die Elektroden mit dem klebenden Elektrolytgel und das Klebematerial können z. B. eine gemeinsame lösbare Schutzfolie oder voneinander getrennte Schutzfolien aufweisen. Bei den Elektroden mit dem klebenden Elektrolytgel handelt es sich um sogenannte Nasselektroden. Das Klebematerial ist z. B. auf der Seite der Trägerstruktur angeordnet, auf der auch die Elektrodenanordnung angeordnet ist.

Gemäß einem Ausführungsbeispiel weist die Schutzfolie, die das Klebematerial und optional ferner das klebende Elektrolytgel bedeckt, eine Mehrzahl von Schutzfolienabschnitte auf, die unabhängig voneinander von der Trägerstruktur und der Vielzahl von Elektroden lösbar sind. Dadurch wird ermöglicht, dass einzelne Abschnitte der Trägerstruktur und der Elektrodenanordnung nacheinander einfach und effizient von dem Nutzer an dessen Gesichtsoberfläche angebracht werden können, da vermieden wird, dass Teile der Trägerstruktur, die erst zu einem späteren Zeitpunkt appliziert werden, nicht miteinander verkleben. Optional bedeckt ein erster Schutzfolienabschnitt der Mehrzahl von Schutzfolienabschnitte eine erste Teilmenge der Vielzahl von Elektroden und der Trägerstruktur und ein zweiter Schutzfolienabschnitt der Mehrzahl von Schutzfolienabschnitte bedeckt eine disjunkte zweite Teilmenge der Vielzahl von Elektroden und der Trägerstruktur. Der erste Schutzfolienabschnitt bedeckt z. B. einen ersten Teilbereich der Trägerstruktur und der zweite Schutzfolienabschnitt bedeckt z. B. einen zweiten Teilbereich der Trägerstruktur, wobei sich der erste Teilbereich und der zweite Teilbereich nicht überschneiden. Die erste Teilmenge der Vielzahl von Elektroden ist z. B. in dem ersten Teilbereich der Trägerstruktur angeordnet und die zweite Teilmenge der Vielzahl von Elektroden ist z. B. in dem zweiten Teilbereich der Trägerstruktur angeordnet. Optional können auch bestimmte Teilmengen der Trägerstruktur als nichtklebend ausgeführt sein, beispielsweise für den Verlauf oberhalb des Ohrs. So kann beispielsweise ein Bereich der Trägerstruktur, der sich auf der Seite befindet, an der die Elektrodenanordnung angeordnet ist, kein Klebematerial aufweisen.

Die Trägerstruktur ist erfindungsgemäß in Form einer Netzstruktur, z. B. einer Matrix, mit sich verzweigenden Verbindungsstegen ausgebildet, die einem Verlauf der Gesichtsoberfläche folgen. Die sich verzweigenden Verbindungsstege führen z. B. entlang markanter Linien und/oder Formen der menschlichen Gesichtsform. Die sich verzweigenden Verbindungsstege folgen z. B. dem Verlauf der Gesichtsoberfläche im Sinne dessen, dass die Verbindungsstege der Form der Augenbraue des Nutzers folgen, z. B. entlang der Augenbraue und entlang des Wangenknochens um das Auge herum, und hinter dem Ohr des Nutzers der Form des Ohres folgen. Optional können die Verbindungsstege ferner entlang des Unterkiefers zu einem Kinn des Nutzers verlaufen. Dadurch, dass die Verbindungsstege dem Verlauf der Gesichtsoberfläche folgen und mit Krafteinwirkung deformierbar sind, entsteht ein hoher Tragekomfort für den Nutzer.

Die verzweigten Verbindungsstege weisen eine Stegbreite auf, die erfindungsgemäß höchstens einem dreifachen und in weiteren Ausführungsbeispielen höchstens einem 1,5fachen oder einem zweifachen Elektrodendurchmesser einer Elektrode der Vielzahl von Elektroden entspricht. Durch die schmale Ausführung der Verbindungsstege wird der Tragekomfort weiter erhöht, da nur ein sehr geringer Teil der Gesichtsoberfläche mit der Trägerstruktur in Verbindung ist. Durch den erhöhten Tragekomfort kann die Vorrichtung für einen langen Zeitraum, wie z. B. über Nacht, an dem Nutzer angewendet werden. Ferner erkannten die Erfinder, dass die schmalen Verbindungsstege eine Anpassung an nutzerabhängige Gesichtsformen vereinfacht.

Gemäß einem Ausführungsbeispiel ist ein Verbindungssteg der sich verzweigenden Verbindungsstege an einem Ende gebogen, so dass die Trägerstruktur ein gebogenes Endstück aufweist, das ausgebildet ist, um hinter einem Ohr des Nutzers angebracht zu werden und wobei der Verbindungssteg ausgebildet ist, um von dem Gesicht des Nutzers oberhalb des Ohrs zu dem gebogenen Endstück zu verlaufen. An dem gebogenen Endstück ist zumindest eine Elektrode der Vielzahl von Elektroden angeordnet. Die Erfinder erkannten, dass der Verlauf des Verbindungsstückes oberhalb des Ohres das selbstständige Anbringen der Vorrichtung auf der Gesichtsoberfläche des Nutzers erleichtert, da der Verbindungssteg ausgebildet ist, um z. B. während der Applikation auf dem oberen Ohransatz des Nutzers abgelegt zu werden, um die korrekte Ausrichtung zu erleichtern. Die Vorrichtung ist über den Verbindungssteg auf dem Ohr abgestützt, was ein effizientes Anordnen und Anbringen der restlichen sich verzweigenden Verbindungsstege auf der Gesichtsoberfläche ermöglicht, da nicht mit einer Hand der Verbindungssteg zu dem Ohr des Nutzers gestützt werden muss. Das gebogene Endstück, basiert auf der Idee, dass dieses von dem Nutzer hinter dem Ohr eingehakt werden kann und dadurch die Vorrichtung bei einem selbstständigen Anbringen auf der Gesichtsoberfläche des Nutzers stabilisiert bzw. bereits zumindest teilwiese in die richtige Zielstellung auf der Gesichtsoberfläche orientiert. Somit wird mittels des Verbindungssteges ein selbstständiges Anbringen der Vorrichtung durch den Nutzer erleichtert und ein Zeitaufwand für das Aufbringen reduziert.

In dem Bereich des Verbindungssteges, der oberhalb des Ohres verläuft, ist beispielsweise kein Klebematerial angebracht oder eine nichtlösbare Schutzfolie angeordnet, die in diesem Bereich das angebrachte Klebematerial bedeckt. Hierdurch wird eine Verbindung der Vorrichtung mit Haaren vermieden und somit der Tragekomfort optimiert. Die Erfinder erkannten, dass der Verbindungssteg stabil auf dem oberen Ohransatz aufgesetzt werden kann, wodurch das selbstständige Anbringen der Vorrichtung auf der Gesichtsoberfläche des Nutzers erleichtert wird.

Gemäß einem Ausführungsbeispiel weist die Trägerstruktur einen Ohr-Bereich auf, der ausgebildet ist, um oberhalb eines Ohres des Nutzers zu verlaufen. In anderen Worten, verläuft ein Teil der Trägerstruktur oberhalb des Ohres des Nutzers. Dieser oberhalb des Ohres verlaufende Teil kann als Ohr-Bereich angesehen werden. Die Erfinder erkannten, dass ein Verlauf der Trägerstruktur oberhalb des Ohres das selbstständige Anbringen der Vorrichtung auf der Gesichtsoberfläche des Nutzers erleichtert, da der Ohr-Bereich ausgebildet ist, um z. B. auf dem oberen Ohransatz des Nutzers abgelegt zu werden. Die Vorrichtung ist über den Ohr-Bereich auf dem Ohr abgestützt, was ein effizientes Anordnen und Anbringen der restlichen Trägerstruktur auf der Gesichtsoberfläche ermöglicht.

Gemäß einem Ausführungsbeispiel ist der Ohr-Bereich der Trägerstruktur Teil eines Verbindungssteges, der ausgehend von zumindest einer ersten Elektrodenposition, die einer ersten Elektrode der Vielzahl von Elektroden zugeordnet ist, und/oder einer zweiten Elektrodenposition, die einer zweiten Elektrode der Vielzahl von Elektroden zugeordnet ist, eine vierte Elektrodenposition, die einer vierten Elektrode der Vielzahl von Elektroden zugeordnet ist, festlegt. Die vierte Elektrodenposition stimmt mit einer Position hinter dem Ohr des Nutzers überein. Optional stellt die vierte Elektrode eine Referenzelektrode (REF) oder eine Masseelektrode (d. h. eine Ground-Elektrode) für die Vielzahl von Elektroden dar. Alternativ kann z. B. die vierte Elektrode eine Referenzelektrode (REF) und eine zu dieser Elektrode benachbart angeordnete Elektrode, z. B. die Elektrode 110₅, eine Masseelektrode (GND) darstellen.

Gemäß einem Ausführungsbeispiel ist die Trägerstruktur oder eine Form der Trägerstruktur ausgebildet, um die Vielzahl von Elektroden an einer korrespondierenden Vielzahl von Positionen an der Gesichtsoberfläche zu positionieren. Die spezielle Formgebung der Trägerstruktur definiert z. B. die Positionen der an der Trägerstruktur angeordneten Elektroden auf der Gesichtsoberfläche. Die Vielzahl von Positionen ist für eine Erfassung der elektrischen Signale an der Gesichtsoberfläche für zumindest eine Linearkombination der elektrischen Signale zum Abbilden von Signalen aus einer vorbestimmten Kopfregion des Nutzers eingerichtet. Die Form der Trägerstruktur sowie z. B. die Positionierung der Elektroden der Elektrodenanordnung auf der Trägerstruktur ermöglicht eine Erfassung von elektrischen Signalen aus verschiedenen Kopfregionen des Nutzers, wie z. B. verschiedenen Hirn- und Muskelregionen. Die Anordnung der Elektroden auf der Trägerstruktur ist ausgelegt, um z. B. basierend auf Linearkombinationen der mittels der Elektroden gemessenen elektrischen Signalen ein Elektroenzephalogramm für verschiedene Hirnregionen [z. B. in einem Bereich Präfrontal (d. h. in einem vorderen Bereich des Stirnlappens (Fp)), Frontal (d. h. stirnseitig bzw. in dem Stirnlappen (F)), schläfenseitig (z. B. temporal (T)), parietal (P), in einem Bereich des Hinterkopfes (d. h. occipital (O)), und in einer zentralen Hirnregion (d.h. central (C))] abzuleiten, ein Elektrookulogramm (vertikal, horizontal und diagonal) abzuleiten und/oder ein Elektromyogram insbesondere z. B. in einem Bereich des Kinns abzuleiten. Dadurch, dass die Trägerstruktur so geformt ist, dass die auf der Trägerstruktur angeordneten Elektroden mit vorbestimmten Positionen auf der Gesichtsoberfläche des Nutzers übereinstimmen, können durch die Linearkombination der gemessenen Signale auch Kopfregionen bzw. Hirn- und Muskelregionen des Nutzers abgebildet werden, an denen keine Elektrode direkt ein Signal misst. Dies ermöglicht die Zahl der Elektroden zu minimieren und somit Kosten zu reduzieren aber auch den Tragekomfort für den Nutzer zu erhöhen, da weniger Bereiche der Gesichtsoberfläche von der Vorrichtung bedeckt sind, möglichst nur haarfreie Hautbereiche verwendet werden und die geringere Anzahl zudem das Gewicht der Vorrichtung reduziert. Schließlich ist im derart erfassten Signal auch Information über die Herzaktivität enthalten, so dass auch ein Elektrokardiogramm (EKG) aus den Daten über eine entsprechende Signalanalyse ableitbar ist.

Gemäß einem Ausführungsbeispiel weist die Vorrichtung einen Signalausgang auf, der ausgebildet ist, um ein auf den von der Vielzahl von Elektroden gemessenen elektrischen Signalen basierendes Ausgangssignal bereitzustellen. Der Signalausgang ist z. B. über elektrische Leitungen mit den Elektroden der Elektrodenanordnung der Vorrichtung gekoppelt. Optional weist die Vorrichtung ferner einen Signalverstärker auf, der ausgebildet ist, um von dem Signalausgang die gemessenen elektrischen Signale zu erhalten. Die Vorrichtung weist z. B. ein Befestigungsmittel auf, das eine Position des Signalverstärkers an einem Hinterkopf, einem Arm, einer Schulter, einer Brust oder einem Hals des Nutzers definiert.

Gemäß einem Ausführungsbeispiel ist das Befestigungsmittel ein Halsband oder Schal, das/der ausgebildet ist, um den Signalverstärker an dem Hals des Nutzers unterhalb eines knöchernden Vorsprungs processus mastoideus und zwischen den beiden Muskeln sternocleidomastoideus und trapezius anzubringen. Dies basiert auf der Erkenntnis, dass diese Position des Signalverstärkers in einem hohen Tragekomfort des Nutzers resultiert. Bei dieser Position ist auch ein Schlafen mit der Vorrichtung komfortabel. Somit verbessert diese Positionierung des Signalverstärkers eine Nutzung der Vorrichtung über mehrere Stunden, wie z. B. über Nacht, oder sogar über mehrere Tage, um ein Langzeitscreening durchzuführen.

Gemäß einem Ausführungsbeispiel, weist die Trägerstruktur als Trägermaterial Polyurethanmaterial (wie z. B. Polyurethanfolienmaterial), Polymermaterial, und/oder Silikonmaterial auf. Das Trägermaterial ist dehnbar oder elastisch und z. B. biegeschlaff. Die Biegeschlaffheit erlaubt die flächige Anpassung an Unebenheiten von Gesicht- und Kopfstrukturen. Wird die Struktur (z. B. die Trägerstruktur mit einer maximalen Dicke von 0.1 mm, 0.3 mm oder 0.5 mm, wie z. B. 0.2 mm) auf eine radiale Erhebung (als Zylinder hier angenommen) mit einem Durchmesser von z.B. 0.5 cm und einer Höhe von 1 cm gelegt, kann das Trägermaterial gleichzeitig durch das Eigengewicht der Schwerkraft folgend oder durch geringe Kraftaufbringung (<1 N) jeweils auf der Kreisfläche und auf vier Stellen der Zylindermantelfläche flächig zum Liegen kommen, wovon jeweils zwei der Bereiche auf der Mantelfläche sich gegenüberliegen und die Verbindungslinien der jeweils gegenüberliegenden Flächen zueinander senkrecht stehen, ohne dass dabei die Trägerstruktur beschädigt wird (reißen oder brechen). Dabei folgt die Struktur der 90° Grad Ecke von Kreisfläche auf Zylindermantelfläche mit einem eng anliegenden Radius von maximal 1mm. Unter Biegeschlaffheit kann z. B. verstanden werden, wenn eine Struktur gleichzeitig entlang zwei zueinander senkrecht stehende laterale Richtungen in einer Dickenrichtung der Struktur verformbar ist ohne beschädigt zu werden und/oder wenn die Struktur mehrfach ineinander verdrehbar ist ohne beschädigt zu werden, d.h. wenn beispielsweise ein Torsionsmoment auf die Struktur wirkt, um diese mehrfach um ein Achse senkrecht zu der Dickenrichtung zu verdrehen.

Gemäß einem Ausführungsbeispiel, weist die Trägerstruktur als Trägermaterial Polyurethanmaterial (wie z. B. Polyurethanfolienmaterial), Polymermaterial, und/oder Silikonmaterial auf. Das Trägermaterial ist dehnbar oder elastisch und z. B. biegeschlaff. Die Elektroden sind mit Leiterbahnen verbunden. Die Leiterbahnen weisen einen geschlungenen Verlauf an oder in der Trägerstruktur auf, um sich bei Dehnung der Trägerstruktur korrespondierend zu längen. Dadurch, dass die Leiterbahnen den geschlungenen Verlauf, wie z. B. wellenförmig, mäanderförmig oder gezackt, aufweisen, wird ermöglicht, dass die Vorrichtung an voneinander abweichende Gesichtsformen von Nutzern angepasst werden kann, da die Leiterbahnen bei einer Deformation der Trägerstruktur durch äußere Krafteinwirkung des Nutzers nicht reißen. Die Leiterbahnen sind z. B. in das Trägermaterial integriert oder auf einer Oberfläche der Trägerstruktur angeordnet. Die Leiterbahnen können mittels Verfahren, die auch bei der Leiterplattenherstellung verwendet werden, an oder in der Trägerstruktur gefertigt werden. So können die Leiterbahnen beispielsweise aufgedruckt oder aufgeätzt werden.

Gemäß einem Ausführungsbeispiel ist die Trägerstruktur ausgebildet, um einen Abstand zwischen zwei Elektroden der Elektrodenanordnung um maximal 20%, 15 %, 10 % oder 5 % zu verlängern. Bei dem Abstand handelt es sich um einen Abstand zwischen zwei benachbarten Elektroden, zwischen denen auf der Trägerstruktur keine weitere Elektrode angeordnet ist. Die Verlängerung wird z. B. durch eine äußere Krafteinwirkung des Nutzers auf die Trägerstruktur bewirkt. Dafür verantwortlich, dass der Abstand um maximal 20 %, 15 %, 10 % oder 5 % verlängert werden kann, sind unter anderem das dehnbare oder elastische Trägermaterial und die geschlungenen Leiterbahnen. Es ist vorteilhaft, wenn die Trägerstruktur ausgebildet ist, um den Abstand zwischen zwei Elektroden z. B. zumindest um 1%, 3 % oder 5 % zu längen. Besonders vorteilhaft ist es, wenn die Trägerstruktur ausgebildet ist, um den Abstand zwischen zwei Elektroden z. B. in einem Bereich von 3 % bis 10 % oder 1 % bis 5 % zu längen. Durch die Mindestanforderung an die Trägerstruktur, dass diese ausgebildet sein sollte den Abstand zumindest um 1%, 3 % oder 5 % zu längen, wird erreicht, dass die Vorrichtung für Nutzer mit hohem Tragekomfort verbunden ist, sich Unebenheiten gut anpasst und für unterschiedliche Gesichtsformen geeignet ist. Durch die Maximalgrenze, dass die Trägerstruktur ausgebildet sein sollte, den Abstand maximal um 20%,15 %, 10 % oder 5 % zu längen, wird verhindert, dass der Nutzer durch äußere Krafteinwirkung die Elektroden, der auf der Trägerstruktur angeordneten Elektrodenanordnung, an Positionen auf der Gesichtsoberfläche zu positionieren, die von den von der Trägerstruktur vorgegebenen Elektrodenpositionen auf der Gesichtsoberfläche mehr als ein gewisser Toleranzbereich abweichen. Bezüglich des Toleranzbereichs wird auf die obigen Ausführungen verwiesen. Begrenzt wird die mögliche Verlängerung des Abstandes unter anderem durch das bei den Leiterbahnen erzeugte Spiel durch die geschwungene Führung in oder an der Trägerstruktur und durch die Dehnbarkeit und/oder Elastizität des Trägermaterials.

Gemäß einem Ausführungsbeispiel weist die Trägerstruktur als Trägermaterial Polyurethanschaum und optional zusätzlich eine Polyurethanfolie (z. B. als Versteifungselement) auf. Das Trägermaterial ist dehnbar oder elastisch und z. B. biegeschlaff. Die Biegeschlaffheit erlaubt die flächige Anpassung an Unebenheiten von Gesicht- und Kopfstrukturen. Wird die Struktur (z. B. die Trägerstruktur mit einer Dicke in einem Bereich von 0.5 mm bis 2 mm) auf eine radiale Erhebung (als Zylinder hier angenommen) mit einem Durchmesser von z.B. 0.5 cm und einer Höhe von 1 cm gelegt, kann das Trägermaterial gleichzeitig durch geringe Kraftaufbringung (<1 N) auf der Kreisfläche und auf vier Stellen der Zylindermantelfläche flächig zum Liegen kommen, wovon jeweils zwei der Bereich auf der Mantelfläche sich gegenüberliegen und die Verbindungslinien der jeweils gegenüberliegenden Flächen zueinander senkrecht stehen, ohne dass dabei die Trägerstruktur beschädigt wird (reißen oder brechen). Dabei folgt die Struktur der 90° Grad Ecke von Kreisfläche auf Zylindermantelfläche mit einem eng anliegenden Radius von maximal 1mm. Unter Biegeschlaffheit kann z. B. verstanden werden, wenn eine Struktur gleichzeitig entlang zwei zueinander senkrecht stehende laterale Richtungen in einer Dickenrichtung der Struktur verformbar ist ohne beschädigt zu werden und/oder wenn die Struktur mehrfach ineinander verdrehbar ist ohne beschädigt zu werden, d.h. wenn beispielsweise ein Torsionsmoment auf die Struktur wirkt, um diese mehrfach um ein Achse senkrecht zu der Dickenrichtung zu verdrehen.

Gemäß einem Ausführungsbeispiel weist die Trägerstruktur als Trägermaterial Polyurethanschaum und optional zusätzlich eine Polyurethanfolie (z. B. als Versteifungselement) auf. Das Trägermaterial ist dehnbar oder elastisch und z. B. biegeschlaff. Die Elektroden sind mit Kabeln verbunden. Die Kabel sind an einer der Elektrodenanordnung abgewandten Oberfläche der Trägerstruktur mit Spiel entlanggeführt. Das Spiel begrenzt eine Kraftübertragung einer Dehnung der Trägerstruktur auf die Kabel. Das Spiel entspricht beispielsweise einem gewundenen oder losen Verlauf der Kabel an der Trägerstruktur. Die Trägerstruktur kann z. B. voneinander beabstandete Klemmstellen aufweisen, die ausgebildet sind, um die Kabel an der Trägerstruktur zu fixieren, d.h. festzuklemmen, wobei die Kabel zwischen den Klemmstellen nicht an der Trägerstruktur fixiert sind und eine größere Länge als der Abstand zwischen den jeweiligen Klemmstellen aufweisen. Alternativ kann die Trägerstruktur z. B. voneinander beabstandete Führungsringe aufweisen, die ausgebildet sind, um die Kabel entlang der Trägerstruktur zu führen, wobei die Kabel lose durch die Führungsringe verlaufen und die Kabel zwischen den Führungsringen eine größere Länge als der Abstand zwischen den jeweiligen Führungsringen aufweisen. Ähnlich wie bereits oben für die Leiterbahnen ausgeführt, ermöglichen das elastische Trägermaterial sowie das Spiel bei der Führung der Kabel entlang der Trägerstruktur, dass die Vorrichtung an voneinander abweichende Gesichtsformen von Nutzern angepasst werden kann, da die Kabel bei einer Deformation der Trägerstruktur durch äußere Krafteinwirkung des Nutzers nicht reißen.

Gemäß einem Ausführungsbeispiel ist die Trägerstruktur ausgebildet, um einen Abstand zwischen zwei Elektroden der Elektrodenanordnung um maximal 20 %, 15 % oder 10 % zu verlängern. Bei dem Abstand handelt es sich um einen Abstand zwischen zwei benachbarten Elektroden, zwischen denen auf der Trägerstruktur keine weitere Elektrode angeordnet ist. Die Verlängerung wird z. B. durch eine äußere Krafteinwirkung des Nutzers auf die Trägerstruktur bewirkt. Dafür verantwortlich, dass der Abstand um maximal 20 %, 15 % oder 10 % verlängert werden kann, sind unter anderem das dehnbare oder elastische Trägermaterial und das Spiel bei der Führung der Kabel entlang der Trägerstruktur. Es ist vorteilhaft, wenn die Trägerstruktur ausgebildet ist, um den Abstand zwischen zwei Elektroden z. B. zumindest um 1%, 3 % oder 5 % zu längen. Besonders vorteilhaft ist es, wenn die Trägerstruktur ausgebildet ist, um den Abstand zwischen zwei Elektroden z. B. in einem Bereich von 3 % bis 15 % oder 5 % bis 20 % zu längen. Durch die Mindestanforderung an die Trägerstruktur, dass diese ausgebildet sein sollte den Abstand zumindest um 1%, 3 % oder 5 % zu längen, wird erreicht, dass der Tragekomfort für den Nutzer hoch ist und die Vorrichtung für Nutzer mit unterschiedlicher Gesichtsform geeignet ist. Durch die Maximalgrenze, dass die Trägerstruktur ausgebildet sein sollte den Abstand maximal um 20 %, 15 % oder 10 % zu längen, wird verhindert, dass der Nutzer durch äußere Krafteinwirkung die Elektroden, der auf der Trägerstruktur angeordneten Elektrodenanordnung, an Positionen auf der Gesichtsoberfläche zu positionieren, die von den von der Trägerstruktur vorgegebenen Elektrodenpositionen auf der Gesichtsoberfläche mehr als ein gewisser Toleranzbereich abweichen. Bezüglich des Toleranzbereichs wird auf die obigen Ausführungen verwiesen. Begrenzt wird die mögliche Verlängerung des Abstandes unter anderem durch Spiel bei der Führung der Kabel entlang der Trägerstruktur und durch die Dehnbarkeit oder Elastizität des Trägermaterials.

Gemäß einem Ausführungsbeispiel ist die Trägerstruktur durch das dehnbare oder elastische Trägermaterial und z. B. durch die spezielle Führung der Leiterbahnen oder Kabel in, auf oder entlang der Trägerstruktur für unterschiedliche Kopfumfänge angepasst, wobei sich die unterschiedlichen Kopfumfänge um maximal 5 cm voneinander unterscheiden. Gemäß einem alternativen Ausführungsbeispiel ist die Trägerstruktur durch das elastische Trägermaterial und z. B. durch die spezielle Führung der Leiterbahnen oder Kabel in, auf oder entlang der Trägerstruktur für die Positionierung des zumindest einen Teils der Elektrodenanordnung an der Gesichtsoberfläche des Kopfes des Nutzers mit einem Kopfumfang in einem Bereich von 50 cm bis 55 cm oder von 55 cm bis 60 cm oder von 60 cm bis 65 cm angepasst.

Gemäß einem Ausführungsbeispiel weist die Trägerstruktur auf einer der Elektrodenanordnung abgewandten Seite ein lösbares Versteifungselement auf, das ausgebildet ist, um die Trägerstruktur zumindest lokal zu versteifen. Das Versteifungselement kann z. B. Papiermaterial, Polyurethanschaum oder Polyimidmaterial aufweisen. Durch das Versteifungselement wird ein selbstständiges Positionieren der Vorrichtung an der Gesichtsoberfläche des Nutzers vereinfacht, da das Versteifungselement verhindert, dass die Trägerstruktur unter Einwirkung der Schwerkraft ihre Form verändert. Dadurch, dass verhindert wird, dass sich die Form der Trägerstruktur verändert, ist die Trägerstruktur ausgebildet, um den Nutzer anzuleiten an welchen Positionen auf dessen Gesichtsoberfläche die Elektroden der Elektrodenanordnung fixiert werden sollen, da die Form der Trägerstruktur die Elektrodenpositionen der Elektroden auf der Gesichtsoberfläche definiert. Ein weiterer Vorteil des Versteifungselements liegt darin, dass verhindert wird, dass die Trägerstruktur sich so verbiegt, dass verschiedene Teile der Trägerstruktur miteinander verkleben und dadurch die Funktionstüchtigkeit der Vorrichtung eingeschränkt wird. Das Versteifungselement ist lösbar, so dass es nach dem Anbringen der Struktur entfernt werden kann und die Eigenschaft der Biegeschlaffheit und Elastizität der Trägerstruktur für eine flächige Anpassung der Trägerstruktur an das Gesicht vollständig gegeben ist.

Gemäß einem Ausführungsbeispiel ist das Versteifungselement ausgebildet, um eine durch eine äußere Krafteinwirkung des Nutzers hervorgerufene Deformierung der Trägerstruktur aufrechtzuerhalten, wenn die äußere Krafteinwirkung des Nutzers beendet ist. Dadurch wird der Tragekomfort erhöht. Der Tragekomfort wird insbesondere erhöht, wenn das Versteifungselement z. B. auf der Trägerstruktur in einem Bereich angeordnet ist, der einem Bereich um ein Auge des Nutzers oder einem Wangenbereich des Nutzers entspricht. Im Kinnbereich sollte z. B. kein Versteifungselement angeordnet sein, da insbesondere für die Mundbewegung des Nutzers der Tragekomfort erhöht wird, wenn die Trägerstruktur in diesem Bereich reversibel elastisch oder leicht dehnbar ausgebildet ist.

Gemäß einem Ausführungsbeispiel weist die Trägerstruktur an einer Position, an der eine Elektrode der Elektrodenanordnung angeordnet ist, zumindest einen Fortsatz auf. Bei dem Fortsatz handelt es sich z. B. um eine Ausbuchtung der Trägerstruktur, um einen abzweigenden Teil der Trägerstruktur, der maximal 2 cm, 1,5 cm oder 1 cm lang ist, oder um eine Verlängerung der Trägerstruktur, die maximal 2 cm, 1,5 cm oder 1 cm lang ist. Die Trägerstruktur erstreckt sich bzw. verzweigt sich z. B. innerhalb einer Ebene und ein Fortsatz erstreckt sich lateral aus dieser Ebene heraus. Der Fortsatz erstreckt sich nicht orthogonal zu der Ebene. Beispielsweise definiert die Oberfläche der Trägerstruktur, an der die Elektrode angeordnet ist, eine Ebene und der Fortsatz erstreckt sich innerhalb dieser Ebene. Der Fortsatz erstreckt sich beispielsweise orthogonal zu einem Normalenvektor der Trägerstruktur an einer Position, an der eine Elektrode der Elektrodenanordnung angeordnet ist. Optional ist auf dem Fortsatz Klebematerial angeordnet, damit die Haftung der Elektroden auf der Gesichtsoberfläche des Nutzers verbessert wird. Ohne Klebematerial erleichtert der Fortsatz eine Handhabung der Vorrichtung, insbesondere ein selbstständiges Aufbringen und Entfernen der Elektrode auf der Gesichtsoberfläche des Nutzers. Zum einen kann durch den Fortsatz die Elektrode leichter gefasst werden, ohne die Elektrode bzw. die empfindliche Sensoreinheit der Elektrode zu berühren und zum anderen stabilisiert der Fortsatz die Elektrode auf der Gesichtsoberfläche, wenn diese durch den Nutzer positioniert wird.

Gemäß einem Ausführungsbeispiel weist die Vielzahl von Elektroden aktive Elektroden auf, die einen Schaltkreis aufweisen, der ausgebildet ist, um eine Impedanzwandlung des gemessenen elektrischen Signals durchzuführen, und/oder das gemessene elektrische Signal zu verstärken. Dadurch, dass die Elektrodenanordnung aktive Elektroden aufweist, ist das Signal von höherer Qualität bzgl. des Signal-Rausch-Verhältnisses und weniger anfällig für Signalstörungen aufgrund von Bewegungen der Elektrode.

Alternativ oder auch zusätzlich zu den aktiven Elektroden, kann die Vielzahl von Elektroden passive Elektroden aufweisen. Weist die Elektrodenanordnung passive Elektroden auf, so wird meist z. B. ein Impedanzwandler und/oder Signalverstärker benötigt, die/der am Körper des Nutzers in der Nähe der Elektrodenanordnung der Vorrichtung angeordnet sein sollte. Optional kann die Vorrichtung einen Impedanzwandler und/oder Signalverstärker aufweisen, die/der mit einem Signalausgang der Vorrichtung gekoppelt sind/ist. Die Nutzung von passiven Elektroden hat den Vorteil, dass in einem Vergleich zu der Nutzung von aktiven Elektroden ein Gewicht der Trägerstruktur mit der daran angeordneten Elektrodenanordnung reduziert werden kann. Dadurch erhöht sich für den Nutzer der Tragekomfort auf der Gesichtsoberfläche.

Ein weiteres Ausführungsbeispiel ist auf eine Vorrichtung gerichtet, die eine Trägerstruktur und eine Elektrodenanordnung mit einer Vielzahl von Elektroden zur Messung von elektrischen Signalen an einer Gesichtsoberfläche eines Kopfes eines Nutzers und hinter einem Ohr des Nutzers aufweist. Bei den Elektroden der Vielzahl von Elektroden handelt es sich um Nasselektroden (mit Gel oder Festgel). Die Trägerstruktur definiert zumindest teilweise eine Relativposition der Elektroden zueinander und die Trägerstruktur ist ausgebildet, um zwischen den einzelnen Elektroden der Elektrodenanordnung flächig in Kontakt mit der Gesichtsoberfläche des Nutzers zu sein. Somit wird durch die spezielle Ausgestaltung der Trägerstruktur ermöglicht, dass diese flächig auf der Gesichtsoberfläche aufliegen kann und Gesichts- und Kopfstrukturen folgt bzw. sich diesen anpasst. Ferner ist die Trägerstruktur ausgebildet, um zumindest einen Teil der Elektrodenanordnung an der Gesichtsoberfläche und hinter dem Ohr zu positionieren. Die Elektroden sind entweder mit Leiterbahnen verbunden, die einen geschlungenen Verlauf an oder in der Trägerstruktur aufweisen, oder mit Kabeln verbunden, die an einer der Elektrodenanordnung abgewandten Oberfläche der Trägerstruktur mit Spiel entlanggeführt sind. Durch den geschlungenen Verlauf der Leiterbahnen oder z. B. durch das Spiel bei der Führung der Kabel ist die Trägerstruktur ausgebildet, um sich zu dehnen ohne dass die Leiterbahnen oder die Kabel beschädigt werden, da sich die Leiterbahnen oder Kabel korrespondierend zu der Dehnung der Trägerstruktur längen können. Das Spiel mit dem die Kabel an der Trägerstruktur entlanggeführt sind begrenzt z. B. eine Kraftübertragung einer Dehnung der Trägerstruktur auf die Kabel. Die Vorrichtung basiert auf den gleichen Überlegungen wie die oben beschriebene Vorrichtung. Die Vorrichtung kann mit allen Merkmalen und Funktionen ergänzt werden, die auch im Hinblick auf die oben beschriebene Vorrichtung beschrieben werden.

Ein weiteres Ausführungsbeispiel ist auf eine Vorrichtung gerichtet, die eine Trägerstruktur und eine Elektrodenanordnung mit einer Vielzahl von Elektroden zur Messung von elektrischen Signalen an einer Gesichtsoberfläche eines Kopfes eines Nutzers und hinter einem Ohr des Nutzers aufweist. Die Elektrodenanordnung ist z. B. auf einer ersten Seite bzw. ersten Oberfläche der Trägerstruktur angeordnet. Bei den Elektroden der Vielzahl von Elektroden handelt es sich um Nasselektroden (mit Gel oder Festgel). Die Trägerstruktur definiert zumindest teilweise eine Relativposition der Elektroden zueinander. Die Trägerstruktur weist ein Trägersubstrat aus Polyurethanfolienmaterial und ein auf dem Trägersubstrat lösbar angeordnetes Versteifungselement auf. Die erste Seite bzw. erste Oberfläche der Trägerstruktur, auf der die Elektrodenanordnung angeordnet ist, liegt z. B. gegenüber des auf dem Trägersubstrat lösbar angeordneten Versteifungselements. In anderen Worten sind die Elektrodenanordnung, das Trägersubstrat und das auf dem Trägersubstrat lösbar angeordnetes Versteifungselement z. B. in dieser Reihenfolge angeordnet. Die Trägerstruktur mit dem auf dem Trägersubstrat lösbar angeordneten Versteifungselement ist innerhalb eines Toleranzbereichs formstabil gebildet und die Trägerstruktur ohne das auf dem Trägersubstrat lösbar angeordnete Versteifungselement ist formlabil bzw. biegeschlaff gebildet. Die Trägerstruktur ist ausgebildet, um zumindest einen Teil der Elektrodenanordnung an der Gesichtsoberfläche und hinter dem Ohr zu positionieren. Die Vorrichtung basiert auf den gleichen Überlegungen wie die oben beschriebenen Vorrichtungen. Die Vorrichtung kann mit allen Merkmalen und Funktionen ergänzt werden, die auch im Hinblick auf die oben beschriebenen Vorrichtungen beschrieben werden.

Gemäß einem Ausführungsbeispiel ist das Trägersubstrat mehrfach ineinander reversibel verdrehbar und/oder entlang zweier zueinander senkrecht stehender lateraler Richtungen gleichzeitig in einer Dickenrichtung der Trägerstruktur reversibel verformbar, um die Trägerstruktur ohne das auf dem Trägersubstrat lösbar angeordneten Versteifungselement formlabil zu bilden. In anderen Worten, ist die Trägerstruktur ohne das auf dem Trägersubstrat lösbar angeordnete Versteifungselement derart formlabil, dass das Trägersubstrat, im Rahmen eines bestimmungsgemäßen Gebrauchs, mehrfach ineinander verdrehbar ist und/oder das Trägersubstrat gleichzeitig entlang zwei zueinander senkrecht stehende laterale Richtungen in einer Dickenrichtung der Trägerstruktur, im Rahmen eines bestimmungsgemäßen Gebrauchs, verformbar ist. Die Verformungen und Verdrehungen des Trägersubstrats können ohne Beschädigung (Risse und Brüche) erfolgen. Es sei angemerkt, dass an einem Punkt, an dem die Trägerstruktur verformt oder verdreht wird, ein Normalenvektor, der orthogonal auf einer der Elektrodenanordnung abgewandten Oberfläche der Trägerstruktur steht, die Dickenrichtung definiert. Die zwei zueinander senkrecht stehenden lateralen Richtungen (d.h. eine erste laterale Richtung und eine zweite laterale Richtung) spannen eine Ebene senkrecht zu dem Normalenvektor auf. Somit ist das Trägersubstrat beispielsweise ausgebildet, um, von einem Verformungspunkt aus gesehen, gleichzeitig eine in der ersten lateralen Richtung liegende Oberfläche des Trägersubstrats und eine in der zweiten lateralen Richtung liegende Oberfläche des Trägersubstrats, nach oben oder unten, d.h. in Dickenrichtung, zu verformen bzw. zu verbiegen. Diese Art der Verformung kann ohne äußere Krafteinwirkung, der Schwerkraft folgend, erfolgen. Sind in oder auf dem Trägersubstrat Leiterbahnen, z. B. mit geschwungenem Verlauf, angeordnet, so kann die oben beschriebene Art der Verformung unter geringer Krafteinwirkung (< 1 N) erfolgen. Bei der oben beschriebenen mehrfachen Verdrehung des Trägersubstrats handelt es sich um eine Torsion um eine Achse entlang der ersten lateralen Richtung, der zweiten lateralen Richtung oder auch einer anderen Richtung innerhalb der von den beiden senkrecht zueinanderstehenden lateralen Richtungen aufgespannten Ebene. Unter Formlabilität bzw. Biegeschlaffheit ist zu verstehen, dass sämtliche oben beschriebenen Verformungen bzw. Verdrehungen erfolgen können ohne das Trägersubstrat zu beschädigen.

Gemäß einem Ausführungsbeispiel ist das Trägersubstrat gleichzeitig entlang der zwei zueinander senkrecht stehenden lateralen Richtungen mit einem Biegeradius in einem Bereich von 0.1 mm bis 1 mm in Dickenrichtung verformbar. Das Trägersubstrat ist so formlabil, dass sich das Trägersubstrat selbst an kleinste Unebenheiten, wie z. B. Hautfalten, anpassen kann. Durch die Formlabilität wird erreicht, dass die Trägerstruktur flächig auf der Gesichtsoberfläche des Nutzers aufliegen kann und selbst bei Unebenheiten ein Abstehen der Trägerstruktur reduziert oder sogar komplett vermieden werden kann. Dadurch wird eine bessere Haftung der Vorrichtung an der Gesichtsoberfläche des Nutzers erzielt, da eine sehr große Klebefläche der Trägerstruktur in Kontakt mit der Gesichtsoberfläche des Nutzers gebracht werden kann. Ferner wird der Tragekomfort erhöht und Langzeitmessungen ermöglicht.

Die oben beschriebene Formlabilität wird beispielsweise durch eine maximale Dicke von 0.5 mm des Trägersubstrats erzielt. Die Dicke entspricht einer Ausdehnung des Trägersubstrats in der Dickenrichtung.

Die Trägerstruktur ist somit ohne äußere Krafteinwirkung eines Nutzers innerhalb eines Toleranzbereichs formstabil, wenn auf dem Trägersubstrat der Trägerstruktur das lösbare Versteifungselement angeordnet ist. Ferner ist die Trägerstruktur unter äußerer Krafteinwirkung des Nutzers deformierbar, um einen Abstand zwischen zwei benachbart angeordneten Elektroden zu verändern und sich Gesichts- und Kopfstrukturen flächendeckend anzupassen, wenn das Versteifungselement von dem Trägersubstrat gelöst ist

Ein weiteres Ausführungsbeispiel ist auf eine Vorrichtung gerichtet, die eine Trägerstruktur und eine Elektrodenanordnung mit einer Vielzahl von Elektroden zur Messung von elektrischen Signalen an einer Gesichtsoberfläche eines Kopfes eines Nutzers und hinter einem Ohr des Nutzers aufweist. Bei den Elektroden der Vielzahl von Elektroden handelt es sich um Nasselektroden (mit Gel oder Festgel). Die Trägerstruktur definiert zumindest teilweise eine Relativposition der Elektroden zueinander und weist als Trägermaterial Polyurethanschaum auf. Die Trägerstruktur ist ohne äußere Krafteinwirkung eines Nutzers innerhalb eines Toleranzbereichs formstabil und unter äußerer Krafteinwirkung des Nutzers deformierbar, um sich Gesichts- und Kopfstrukturen flächendeckend anzupassen. Die Trägerstruktur ist ausgebildet, um unter der äußeren Krafteinwirkung des Nutzers mehrfach ineinander reversibel verdreht zu werden und/oder entlang zweier zueinander senkrecht stehender lateraler Richtungen gleichzeitig in einer Dickenrichtung der Trägerstruktur reversibel verformt zu werden. Die Trägerstruktur ist ausgebildet, um zumindest einen Teil der Elektrodenanordnung an der Gesichtsoberfläche und hinter dem Ohr zu positionieren. Die Vorrichtung basiert auf den gleichen Überlegungen wie die oben beschriebenen Vorrichtungen. Die Vorrichtung kann mit allen Merkmalen und Funktionen ergänzt werden, die auch im Hinblick auf die oben beschriebenen Vorrichtungen beschrieben werden.

In anderen Worten, ist die Trägerstruktur unter der äußeren Krafteinwirkung des Nutzers, im Rahmen eines bestimmungsgemäßen Gebrauchs, mehrfach ineinander verdrehbar und/oder entlang zweier zueinander senkrecht stehenden lateralen Richtungen in einer Dickenrichtung der Trägerstruktur, im Rahmen eines bestimmungsgemäßen Gebrauchs, verformbar. Die Verformungen und Verdrehungen der Trägerstruktur können ohne Beschädigung (Risse und Brüche) erfolgen. Es sei angemerkt, dass an einem Punkt, an dem die Trägerstruktur verformt oder verdreht wird, ein Normalenvektor, der orthogonal auf einer der Elektrodenanordnung abgewandten Oberfläche der Trägerstruktur steht, die Dickenrichtung definiert. Die zwei zueinander senkrecht stehenden lateralen Richtungen (d.h. eine erste laterale Richtung und eine zweite laterale Richtung) spannen eine Ebene senkrecht zu dem Normalenvektor auf. Somit ist die Trägerstruktur beispielsweise ausgebildet, um von einem Nutzer unter der äußeren Krafteinwirkung derart verformt zu werden, dass, von einem Verformungspunkt aus gesehen, gleichzeitig eine in der ersten lateralen Richtung liegende Oberfläche der Trägerstruktur und eine in der zweiten lateralen Richtung liegende Oberfläche der Trägerstruktur, nach oben oder unten, d.h. in Dickenrichtung, verformt bzw. verbogen werden können. Bei der oben beschriebenen mehrfachen Verdrehung der Trägerstruktur handelt es sich um eine Torsion um eine Achse entlang der ersten lateralen Richtung, der zweiten lateralen Richtung oder auch einer anderen Richtung innerhalb der von den beiden senkrecht zueinanderstehenden lateralen Richtungen aufgespannten Ebene, bzw. um eine Torsion um eine Achse die in einer Ebene senkrecht zu der Dickenrichtung angeordnet ist. Sämtliche oben beschriebenen Verformungen bzw. Verdrehungen können erfolgen ohne die Trägerstruktur zu beschädigen.

Gemäß einem Ausführungsbeispiel ist die Trägerstruktur gleichzeitig entlang der zwei zueinander senkrecht stehenden lateralen Richtungen mit einem Biegeradius in einem Bereich von 0.1 mm bis 1 mm in Dickenrichtung verformbar. Durch die Formlabilität wird erreicht, dass die Trägerstruktur flächig auf der Gesichtsoberfläche des Nutzers aufliegen kann und selbst bei Unebenheiten ein abstehen der Trägerstruktur reduziert oder sogar komplett vermieden werden kann. Dadurch wird eine bessere Haftung der Vorrichtung an der Gesichtsoberfläche des Nutzers erzielt, da eine sehr große Klebefläche der Trägerstruktur in Kontakt mit der Gesichtsoberfläche des Nutzers gebracht werden kann. Ferner wird der Tragekomfort erhöht und Langzeitmessungen ermöglicht.

Die Trägerstruktur weist z. B. in der Dickenrichtung eine Dicke in einem Bereich von 0.5 mm bis 2 mm auf. Eine Dicke in diesem Bereich ermöglicht eine Formstabilität ohne äußere Krafteinwirkung und eine Formlabilität unter äußerer Krafteinwirkung.

Ein weiteres Ausführungsbeispiel betrifft ein System mit einer Vorrichtung nach einem der oben beschriebenen Ausführungsbeispielen und mit einer Auswerteanordnung. Die Auswerteanordnung ist ausgebildet, um die von der Vielzahl von Elektroden gemessenen elektrischen Signale auszuwerten.

Gemäß einem Ausführungsbeispiel ist die Auswerteanordnung ausgebildet, um basierend auf den elektrischen Signalen eine Information über eine Schlafarchitektur des Nutzers bereitzustellen; oder eine Information über ein epileptisches Verhalten des Nutzers bereitzustellen; oder eine neurologische Erkrankung des Nutzers anzuzeigen; oder eine kognitionspsychologische Information bereitzustellen. Somit ermöglicht das erfindungsgemäße System ein häusliches oder ambulantes Schlafmonitoring oder ein häusliches oder ambulantes Monitoring bei Patienten mit Epilepsie oder Verdacht darauf oder bei Patienten mit anderen neurologischen Erkrankungen oder Verdacht darauf (z. B. Depression, Alzheimer) u.a. zur Ableitung von Frühindikatoren. Alternativ ist das System für den Einsatz in kognitionspsychologischen Testreihen geeignet. Das System ist z. B. ausgelegt, um eine Aufmerksamkeit, eine Vigilanz, eine Höranstrengung, eine kognitive Belastung, eine kognitive Verarbeitungsfähigkeit und/oder eine sensorische Funktionalität des Nutzers zu untersuchen bzw. zu bestimmen. Diese kognitionspsychologischen Testreihen können durch die spezielle Ausgestaltung des Systems in der Häuslichkeit, in Pflegeheimen, aber auch bei niedergelassenen Ärzten oder in Kliniken durchgeführt werden. Ein besonderer Vorteil des Systems stellt auch hier die einfache Installation der Elektroden dar, da dadurch Zeit eingespart werden kann. Das erfindungsgemäße System eignet sich ferner für den Einsatz zur Erfassung eben genannter kognitiver Zustände in Arbeitssituationen, insbesondere für sicherheitskritische Anwendungen (z.B. Lokführer, Piloten, Fluglotsen, LKW-Fahrer, Minenfahrzeuge). Eine weitere mögliche Anwendung des Systems besteht in der neuropsychologischen Forschung und in angrenzenden Forschungsbereichen zu kognitiven Zuständen, aber auch in Forschungsbereichen zu neurologischen Krankheitssymptomen im wachen, aber auch im schlafenden Zustand.

Gemäß einem Ausführungsbeispiel weist das System ein Hörgerät auf. Die Auswerteanordnung ist ausgebildet, um basierend auf den elektrischen Signalen das Hörgerät zu steuern.

Gemäß einem Ausführungsbeispiel ist die Auswerteanordnung ausgebildet, um die von der Vorrichtung gemessenen Signale mit einer Einhüllenden eines von dem Hörgerät aufgenommenen Audiosignals zu korrelieren, um einen Strahlformer des Hörgeräts zu steuern. Die Erfinder erkannten, dass die von der Vorrichtung aufgezeichneten elektrischen Signale anzeigen können, auf welchem Audiosignal die Aufmerksamkeit des Nutzers gerichtet ist. Das System kann somit ausgebildet sein, um den Strahlformer in die Richtung der Aufmerksamkeit des Nutzers zu richten, um das entsprechende Audiosignal hervorzuheben. Die Auswerteanordnung ist z. B. ausgebildet, um basierend auf der Korrelation eine Aufmerksamkeit des Nutzers auf ein Zeitsignal, d. h. das Audiosignal, zu erkennen. Die Vorrichtung ist z. B. ausgebildet, um elektrische Signale zu messen bzw. zu erfassen, die auf Muskelbewegungen des Auges und/oder auf dem Augenkörper, der als Dipol fungieren kann, basieren. Die Auswerteeinrichtung ist z. B. ausgebildet, um basierend auf den elektrischen Signalen ein EOG zu erstellen und so Informationen zur Blickrichtung des Nutzers oder zu einer Blinzelrate des Nutzers abzuleiten. Die Blickrichtung zeigt ebenfalls Elemente der Aufmerksamkeit an, so dass das System somit ausgebildet sein kann, um den Strahlformer in die Richtung der Aufmerksamkeit des Nutzers zu richten. Dadurch ist das Hörgerät ausgebildet, um gezielt die Audiosignale aus der Richtung der Aufmerksamkeit des Nutzers für den Nutzer zu verstärken. Somit wird ein System geschaffen, dass die bei dem Nutzer ankommende Hörqualität verbessert.

Gemäß einem Ausführungsbeispiel ist die Auswerteanordnung ausgebildet, um die von der Vorrichtung gemessenen Signale auf vorbestimmte Signaleigenschaften auszuwerten, um das Hörgerät in Antwort auf Signale des Nutzers zu steuern, wie z. B. das Hörgerät anzuschalten und auszuschalten oder auch die Lautstärke oder bestimmte Parametersätze oder auch die Signalverstärkung aus einer spezifischen Richtung zu regulieren. Optional weist die Auswerteanordnung eine Datenbank auf, in der die vorbestimmten Signaleigenschaften abgespeichert sind. Mit den vorbestimmten Signaleigenschaften können Steuerbefehle für das Hörgerät verknüpft sein. Die Vorrichtung kann beispielsweise ausgelegt sein, um elektrische Signale zu messen bzw. zu erfassen, die auf Muskelbewegungen des Auges und/oder auf dem Augenkörper, der als Dipol fungieren kann, basieren. Die Auswerteanordnung kann ausgebildet sein, um basierend auf diesen Signalen Signaleigenschaften zu detektieren, die z. B. einer vorbestimmten Augenbewegung, wie z. B. einem Augenzwinkern oder auch einer Augenbrauenbewegung, zugeordnet sind. Dies basiert auf der Erkenntnis, dass die mittels der Vorrichtung gemessenen Signale an der Gesichtsoberfläche bzw. an dem Kopf des Nutzers charakteristische Gesichtsbewegungen anzeigen können. Somit wird mit dem System ermöglicht, das Hörgerät automatisch mit Gesichtsbewegungen zu steuern, ohne Knöpfe an dem Hörgerät betätigen zu müssen. Dies vereinfacht die Handhabung des Hörgeräts.

Ein weiteres Ausführungsbeispiel betrifft ein Verfahren zur Herstellung der Vorrichtung nach einem der oben beschriebenen Ausführungsbeispielen. Das Verfahren umfasst unter anderem den Schritt Bereitstellen einer Trägerstruktur, wobei die Trägerstruktur an eine Gesichtsoberfläche eines Kopfes eines Nutzers der Vorrichtung angepasst wird und wobei die Trägerstruktur ohne äußere Krafteinwirkung eines Nutzers innerhalb eines Toleranzbereichs formstabil ist und unter äußerer Krafteinwirkung des Nutzers deformierbar ist, um einen Abstand zwischen zwei benachbart angeordneten Elektroden zu verändern und/oder sich Gesichts- und Kopfstrukturen flächendeckend anzupassen. Die Formstabilität kann z. B. dadurch erreicht werden, dass für die Trägerstruktur ein Material, wie z. B. Polyurethanschaummaterial, und eine bestimmte Dicke, z. B. in einem Bereich von 0.5 mm bis 2 mm, verwendet wird. Alternativ kann die Trägerstruktur aus einem Trägersubstrat und einem lösbaren Versteifungselement zusammengesetzt werden. Das Trägersubstrat kann z. B. ein Trägermaterial aufweisen, wobei sich Polyurethanmaterial, Polymermaterial, und/oder Silikonmaterial eignet. Das lösbare Versteifungselement ist z. B. ausgebildet, um die Form der Trägerstruktur zu stabilisieren. Gemäß einem Ausführungsbeispiel kann zumindest ein lösbares Versteifungselement lokal auf dem Trägersubstrat angeordnet werden. Alternativ kann auch das komplette Trägersubstrat mit dem Versteifungselement bedeckt werden. Ein weiterer Schritt des Verfahrens betrifft ein Anbringen einer Elektrodenanordnung an die Trägerstruktur. Die Elektrodenanordnung weist eine Vielzahl von Elektroden zur Messung von elektrischen Signalen an einer Gesichtsoberfläche eines Kopfes eines Nutzers der Vorrichtung und/oder zur Messung von elektrischen Signalen hinter einem Ohr des Nutzers der Vorrichtung auf. Die Trägerstruktur definiert zumindest teilweise eine Relativposition der Elektroden zueinander, wobei die Trägerstruktur ausgebildet ist, um zumindest einen Teil der Elektrodenanordnung an der Gesichtsoberfläche und/oder hinter dem Ohr des Nutzers zu positionieren.

Ein weiteres Ausführungsbeispiel betrifft ein Verfahren zur Verwendung der Vorrichtung nach einem der oben beschriebenen Ausführungsbeispielen. Das Verfahren umfasst unter anderem die Schritte Anlegen der Vorrichtung an einer Gesichtsoberfläche eines Kopfes eines Nutzers durch den Nutzer selbst oder einer anderen Person und Überwachen des Nutzers anhand der von der Vielzahl von Elektroden gemessenen elektrischen Signale. Besonders vorteilhaft an der vorliegenden Erfindung ist, dass diese von dem Nutzer selbst angelegt werden kann.

Gemäß einem Ausführungsbeispiel weist das Verfahren ein Überwachen eines Schlafverhaltens des Nutzers basierend auf den gemessenen elektrischen Signalen; oder ein Überwachen eines kognitionspsychologischen Verhaltens basierend auf den gemessenen elektrischen Signalen auf.

Gemäß einem Ausführungsbeispiel weist das Anlegen der Vorrichtung an der Gesichtsoberfläche des Kopfes des Nutzers unter anderem die Schritte Ertasten eines oberen Wangenknochens und Anbringen einer ersten Elektrode an dem oberen Wangenknochen; Anbringen einer zweiten Elektrode vor dem Tragus eines Ohres des Nutzers; Anbringen einer dritten Elektrode an einer Stirn senkrecht oberhalb eines Nasion in einem Bereich von 1-3 cm über einer Augenbrauenlinie; und Anbringen einer vierten Elektrode hinter dem Ohr, auf. Es ist besonders vorteilhaft, wenn in einem ersten Schritt die erste Elektrode und in einem zweiten Schritt die zweite Elektrode auf der Gesichtsoberfläche angebracht werden. Dies basiert auf der Erkenntnis, dass der Wangenknochen und der Punkt vor dem Tragus des Ohres sehr markante Punkt auf einer Gesichtsoberfläche darstellen und daher für einen Nutzer sehr einfach zu finden sind. Dadurch können die erste und zweite Elektrode sehr exakt auf der Gesichtsoberfläche angebracht werden. Für das Anbringen der restlichen Elektroden der Vielzahl von Elektroden der Elektrodenanordnung der Vorrichtung wird der Nutzer durch eine Formgebung der Trägerstruktur der Vorrichtung angeleitet, da die Form der Trägerstruktur z. B. ausgebildet ist, um die Position der restlichen Elektroden relativ zu der ersten Elektrode und der zweiten Elektrode auf der Gesichtsoberfläche zu definieren.

### Figurenkurzbeschreibung

Alle hierin erläuterten Beispiele, die nicht alle Merkmale, oder Äquivalente derselben, eines der unabhängigen Ansprüche aufweisen, dienen dazu das Verständnis der Erfindung zu erleichtern. Ausführungsbeispiele gemäß der vorliegenden Erfindung werden nachfolgend Bezug nehmend auf die beiliegenden Figuren näher erläutert. Hinsichtlich der dargestellten schematischen Figuren wird darauf hingewiesen, dass die dargestellten Funktionsblöcke sowohl als Elemente oder Merkmale der erfindungsgemäßen Vorrichtung als auch als entsprechende Verfahrensschritte des erfindungsgemäßen Verfahrens zu verstehen sind, und auch entsprechende Verfahrensschritte des erfindungsgemäßen Verfahrens davon abgeleitet werden können. Es zeigen:
- Fig. 1a: eine schematische Darstellung einer Vorrichtung, gemäß einem Ausführungsbeispiel der vorliegenden Erfindung;
- Fig. 1b: eine schematische Darstellung der Vorrichtung an einem Kopf eines Nutzers, gemäß einem Ausführungsbeispiel der vorliegenden Erfindung;
- Fig. 2: eine n=1 Rotation;
- Fig. 3a: eine schematische Darstellung einer Vorrichtung mit einem Versteifungselement, gemäß einem Ausführungsbeispiel der vorliegenden Erfindung;
- Fig. 3b: eine Formstabilität eines Verbindungsstegs einer Trägerstruktur mit oder ohne Versteifungselement einer Vorrichtung, gemäß einem Ausführungsbeispiel der vorliegenden Erfindung;
- Fig. 4a: eine schematische Darstellung einer Vorrichtung mit einem Verbindungssteg zu einem Kinnbereich eines Nutzers der Vorrichtung, gemäß einem Ausführungsbeispiel der vorliegenden Erfindung;
- Fig. 4b: eine schematische Darstellung einer Vorrichtung mit einer Trägerstruktur, die Ausbuchtungen an bestimmten Elektrodenpositionen aufweist, gemäß einem Ausführungsbeispiel der vorliegenden Erfindung;
- Fig. 5a: eine schematische Darstellung einer Vorrichtung an einem Kopf eines Nutzers in einer Seitansicht, gemäß einem Ausführungsbeispiel der vorliegenden Erfindung;
- Fig. 5b: eine schematische Darstellung einer Vorrichtung an einem Kopf eines Nutzers in einer Ansicht von einem Hinterkopf eines Nutzers aus, gemäß einem Ausführungsbeispiel der vorliegenden Erfindung;
- Fig. 6a: eine schematische Darstellung von Linearkombinationen von Elektrodenkanälen für ein EEG, gemäß einem Ausführungsbeispiel der vorliegenden Erfindung;
- Fig. 6b: eine schematische Darstellung von Linearkombinationen von Elektrodenkanälen für ein EOG und EMG, gemäß einem Ausführungsbeispiel der vorliegenden Erfindung;
- Fig. 7: eine schematische Darstellung einer Vorrichtung mit Kabelverbindungen, gemäß einem Ausführungsbeispiel der vorliegenden Erfindung;
- Fig. 8a: eine schematische Darstellung einer in eine Trägerstruktur der Vorrichtung integrierten Elektrode, gemäß einem Ausführungsbeispiel der vorliegenden Erfindung;
- Fig. 8b: eine schematische Darstellung einer in eine Trägerstruktur der Vorrichtung integrierten Elektrode mit Gellinse, gemäß einem Ausführungsbeispiel der vorliegenden Erfindung;
- Fig. 9a: eine schematische Darstellung einer Vorrichtung mit einem Signalverstärker in einem Kopfband, gemäß einem Ausführungsbeispiel der vorliegenden Erfindung;
- Fig. 9b: eine schematische Darstellung eines Signalverstärkers in einem Halsband, gemäß einem Ausführungsbeispiel der vorliegenden Erfindung;
- Fig. 9c: eine schematische Darstellung einer Vorrichtung mit einem Signalverstärker hinter oder unterhalb eines Ohrs eines Nutzers, gemäß einem Ausführungsbeispiel der vorliegenden Erfindung;
- Fig. 9d: eine schematische Darstellung einer Vorrichtung mit einem Signalverstärker auf einer Schulter oder an einer Brust eines Nutzers, gemäß einem Ausführungsbeispiel der vorliegenden Erfindung;
- Fig. 9e: eine schematische Darstellung einer Position eines Signalverstärkers relativ zu einer Muskelanordnung in einem Halsbereich eines Nutzers, gemäß einem Ausführungsbeispiel der vorliegenden Erfindung;
- Fig. 10: ein Blockdiagramm eines Verfahrens zur Verwendung einer erfindungsgemäßen Vorrichtung;
- Fig. 11: ein Blockdiagramm eines Systems mit einem Verstärker und einer erfindungsgemäßen Vorrichtung;
- Fig. 12: eine schematische Darstellung eines Systems mit einer Auswerteanordnung und einer erfindungsgemäßen Vorrichtung;
- Fig. 13: ein Blockdiagramm eines Verfahrens zur Herstellung einer erfindungsgemäßen Vorrichtung;
- Fig. 14: eine herkömmliche Elektrodenplatzierung bei einer Polysomnographie;
- Fig. 15: eine schematische Darstellung einer Vorrichtung mit angezeigtem Signalausgang auf der Trägerstruktur gemäß einem Ausführungsbeispiel der vorliegenden Erfindung;
- Fig. 16: eine schematische Darstellung einer Vorrichtung mit einer Elektrodenanordnung, die hinter einem Ohr eines Nutzers angeordnet wird.

### Detaillierte Beschreibung der Ausführungsbeispiele gemäß den Figuren

Bevor nachfolgend Ausführungsbeispiele der vorliegenden Erfindung im Detail anhand der Zeichnungen näher erläutert werden, wird darauf hingewiesen, dass identische, funktionsgleiche oder gleichwirkende Elemente, Objekte und/oder Strukturen in den unterschiedlichen Figuren mit den gleichen oder ähnlichen Bezugszeichen versehen sind, so dass die in unterschiedlichen Ausführungsbeispielen dargestellte Beschreibung dieser Elemente untereinander austauschbar ist bzw. aufeinander angewendet werden kann.

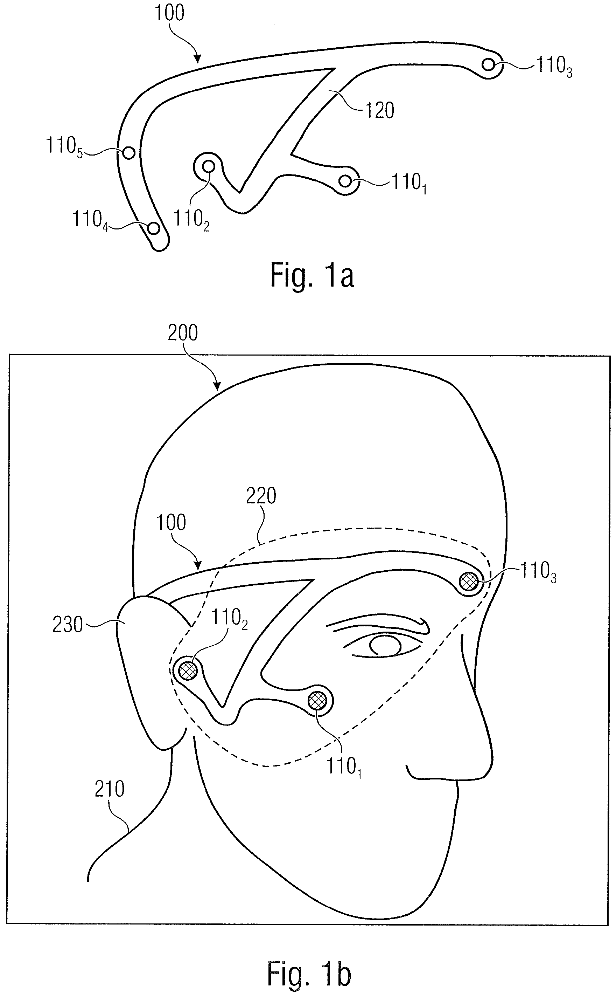

Fig. 1a zeigt eine schematische Darstellung einer Vorrichtung 100 gemäß einem Ausführungsbeispiel der vorliegenden Erfindung und Fig. 1b zeigt die Vorrichtung 100 an einem Kopf 200 eines Nutzers 210.

Die Vorrichtung 100 weist eine Elektrodenanordnung 110 mit einer Vielzahl von Elektroden 110₁ bis 110₅ zur Messung von elektrischen Signalen an einer Gesichtsoberfläche 220 des Kopfes 200 des Nutzers 210 und hinter einem Ohr 230 des Nutzers 210 auf. Gemäß Fig. 1a und Fig. 1b sind eine erste Elektrode 110₁, eine zweite Elektrode 110₂ und eine dritte Elektrode 110₃ ausgebildet, um elektrische Signale an einer Gesichtsoberfläche 220 des Kopfes 200 des Nutzers 210 zu messen. Eine vierte Elektrode 110₄ und eine fünfte Elektrode 110₅ sind ausgebildet, um elektrische Signale hinter dem Ohr 230 des Nutzers 210 zu messen. Die Vorrichtung ist aber nicht auf die in den Figuren 1a und 1b dargestellte Anzahl und Anordnung der Elektroden 110₁ bis 110₅ beschränkt.

Ferner weist die Vorrichtung 100 eine Trägerstruktur 120 auf. Die Vielzahl von Elektroden 110₁ bis 110₅ sind z. B. an einer korrespondierenden Vielzahl von Elektrodenpositionen an der Trägerstruktur 120 angeordnet. Die Vielzahl von Elektroden 110₁ bis 110₅ der Elektrodenanordnung 110 sind z. B. über die Trägerstruktur 120 miteinander verbunden, wobei die Trägerstruktur 120 eine Relativposition der Elektroden 110₁ bis 110₅ zueinander zumindest teilweise definiert. Die Trägerstruktur 120 weist sich verzweigende Verbindungsstege auf, die die Elektroden 110₁ bis 110₅ miteinander verbinden und die Relativposition der Elektroden 110₁ bis 110₅ zueinander zumindest teilweise definieren, da die Positionen der Vielzahl von Elektroden 110₁ bis 110₅ auf Positionen auf den Verbindungsstegen beschränkt sind. Die Form und Länge eines Verbindungsstegs zwischen zwei benachbarten Elektroden definiert z. B. die Relativposition der beiden benachbarten Elektroden zueinander. Die Form und Länge eines Verbindungsstegs zwischen zwei benachbarten Elektroden definiert z. B. Positionen der beiden Elektroden an dem Kopf 200 des Nutzers. Dabei folgt die Form z. B. einer menschlichen Gesichtsform bzw. menschlichen Gesichtsstrukturen. Die Formgebung der Trägerstruktur definiert z. B. für jede Elektrode der Vielzahl von Elektroden eine Position an dem Kopf 200 des Nutzers, d.h. auf der Gesichtsoberfläche 220 und hinter dem Ohr 230.

Die Trägerstruktur 120 ist ausgebildet, um zumindest einen ersten Teil der Elektrodenanordnung, z. B. die Elektroden 110₁ bis 110₃, an der Gesichtsoberfläche 220 zu positionieren und einen zweiten Teil der Elektrodenanordnung, z. B. die Elektroden 110₄ und 110₅, hinter dem Ohr 230 zu positionieren. Die Trägerstruktur 120 ist z. B. durch ihre spezielle Formgebung für eine Positionierung der Vielzahl von Elektroden 110₁ bis 110₅ ausgebildet, da z. B. die Form und Länge der sich verzweigenden Verbindungsstege einer Gesichts- bzw. Kopfstruktur folgen und somit die sich verzweigenden Verbindungssteg die Positionen der Elektroden 110₁ bis 110₅ auf der Gesichtsoberfläche 220 des Kopfes 200 des Nutzers 210 und hinter dem Ohr 230 des Nutzers 210 definieren.

Die Trägerstruktur 120 ist ohne äußere Krafteinwirkung des Nutzers 210 innerhalb eines Toleranzbereichs formstabil und unter äußerer Krafteinwirkung des Nutzers 210 deformierbar. Innerhalb eines Toleranzbereichs formstabil bedeutet z. B. dass sich die Trägerstruktur z. B. lediglich unter dem Einfluss der Schwerkraft geringfügig verbiegen kann. Dadurch wird vermieden, dass die Form bei einem Anlegen an den Kopf 200 durch den Nutzer in sich zusammenfällt. Die Trägerstruktur ist z. B. ausgebildet, um ohne äußere Krafteinwirkung des Nutzers 210 eine Verbiegung von maximal 6 cm über einen Längenabschnitt der Trägerstruktur 120 von 10 cm zuzulassen. Hierfür kann die Trägerstruktur z. B. eine Stützstruktur aufweisen, die ausgebildet ist, um einer Verformung der Trägerstruktur entgegenzuwirken. Diese Stützstruktur kann als ablösbares Versteifungselement gestaltet sein, so dass sie nach Aufbringen der Trägerstruktur an der Kopfoberfläche entfernt werden kann. Alternativ, kann die Formstabilität auch über die Dicke der Trägerstruktur erzielt werden. Je dicker die Trägerstruktur, desto formstabiler ist diese. Bei einer Dicke von 1 mm wird beispielsweise eine ausreichende Formstabilität erzielt. Bei geringeren Dicken kann die Formstabilität über die Stützstruktur erreicht werden. Zusätzlich zu der Formstabilität weist die Trägerstruktur 120 eine gewisse Dehnbarkeit oder Elastizität auf, wodurch die Trägerstruktur unter äußerer Krafteinwirkung des Nutzers 210 deformiert werden kann. Der Nutzer kann die Trägerstruktur beispielsweise dehnen oder verbiegen, um einen Abstand zwischen zwei benachbart angeordneten Elektroden zu verändern und/oder sich Gesichts- und Kopfstrukturen flächendeckend anzupassen. Durch die flächendeckende Anpassung hat die Trägerstruktur eine große Auflagefläche auf der Kopfoberfläche, wodurch die Trägerstruktur robuster an der Kopfoberfläche des Nutzers fixiert werden kann. Die Trägerstruktur ist daher ausgebildet, um einige Stunden oder über eine gesamte Nacht oder über einen kompletten Tag oder mehrere Tage stabil auf der Haut zu sitzen. Die Trägerstruktur ist z. B. ausgebildet, um den Abstand zwischen zwei benachbart angeordneten Elektroden unter der äußeren Krafteinwirkung des Nutzers 210 in einem Bereich von 5 % bis 10 % oder 5 % bis 20 % zu vergrößern. In der Regel wird dies nur zur Erreichung der Stirnelektrode, z. B. der dritten Elektrode 110₂, besonders ausgenutzt. Die Flexibilität des Materials ist aber vorteilhaft, um kleine Anpassungen beim Verlauf über und hinter dem Ohr 230 ausführen zu können und in der Bewegung in dem Gesicht einen hohen Tragekomfort zu erlauben.

Für die Materialwahl der Gesamtstruktur ist es z. B. wichtig, dass
(a) die an der Haut klebende Trägerstruktur 120 unter Einwirkung des Nutzers 210 leicht verformbar (biegeschlaff und/oder in Teilen dehnbar) ist, um sich Gesichtsstrukturen (z. B. Wölbungen) für eine flächige und damit robuste Klebung und Proportionsunterschieden des Gesichts zur Erreichung der gewünschten Elektrodenpositionen gut anzupassen; und dass
(b) die Trägerstruktur 120 für den Klebeprozess ohne Einwirkung des Nutzers formstabil bleibt, damit die Arme des Grids (z. B. des Netzes der sich verzweigenden Verbindungsstegen der Trägerstruktur) nicht beim Ankleben ungewollt an Gesichtsstellen, Fingern oder miteinander verkleben, wenn diese durch eine sehr hohe Biegeschlaffheit herunterhängen.

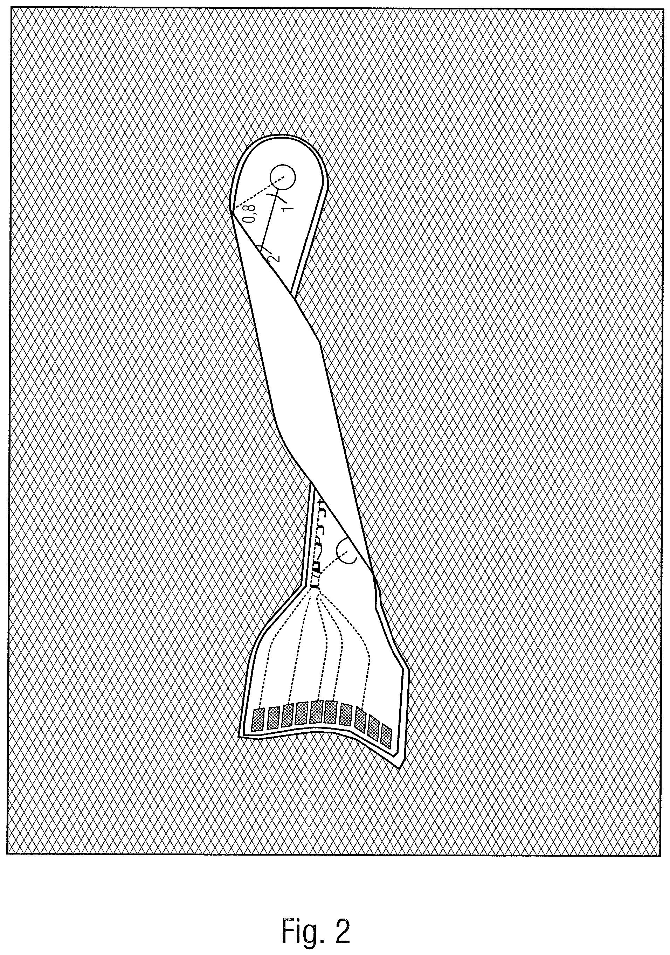

Die Trägerstruktur 120, die letztlich auf der Haut klebt, zeichnet sich daher durch Dehnbarkeit und/oder Biegeschlaffheit, d.h. Forminstabilität oder Formlabilität, aus:
Die Trägerstruktur ist z. B. derart deformierbar, dass ein Verbindungssteg der Trägerstruktur 120 mehrfach ineinander verdreht werden kann, ohne dass der Verbindungssteg reißt. In Fig. 2 ist eine solche Verdrehung mit Papier mit n=1 Drehungen bzw. Rotationen gezeigt. Eine derartige Verformbarkeit kann beispielsweise erzielt werden, wenn die Trägerstruktur Polyurethanmaterial, Polymermaterial und/oder Silikonmaterial als Trägermaterial aufweist. Einen weiteren Einfluss auf die Verformbarkeit der Trägerstruktur können ferner elektrische Verbindungen, der Vielzahl von Elektroden mit einem Signalausgang der Vorrichtung, haben. Zum Erhalt der Dehnbarkeit der Gesamtstruktur, sind Leiterbahnen bzw. Kabelverbindungen z. B. so gestaltet, dass Längennachschub, z. B. Zusatzlängen, in Form von Wellen oder Schlaufen vorhanden ist. Weist die Trägerstruktur als Trägermaterial z. B. ein Polyurethanmaterial auf, so kann dieses z. B. in Form von Polyurethan-Folienmaterial vorliegen, in das Leiterbahnen mit einem geschlungenen Verlauf eingebracht sind, oder z. B. in Form von Polyurethanschaum vorliegen, auf dem Kabelverbindungen angeordnet sind. Im Fall des Polyurethan-Folienmaterials ist die Trägerstruktur z. B. ausgebildet, um eine Dehnung in einem Bereich von 5 % bis 10% zu ermöglichen bzw. zuzulassen und im Falle von Polyurethanschaum ist die Trägerstruktur z. B. ausgebildet, um eine Dehnung in einem Bereich von 5 % bis zu 20 % zu ermöglichen bzw. zuzulassen.

Ein dünnes (z. B. mit einer Dicke zwischen 0.1 mm und 0.5 mm) Polyurethan-Folienmaterial kann als Materialstreifen mehrfach ineinander verdreht werden, ohne dass das Material reißt. Das hier als dünne Folie vorgeschlagene Trägermaterial soll mehr als n = 1 Drehungen aushalten, ohne einzureißen oder andersartig zu versagen. Die Biegeschlaffheit führt dazu, dass es sehr leicht (geringer Druck der Finger, d. h. unter äußerer Krafteinwirkung des Nutzers 210) in beliebe Richtung verformbar ist und so zu hohem Tragekomfort führt, da sich die Kanten der Trägerstruktur so leicht an die Kopfoberfläche anschmiegen. Außerdem weist es eine Dehnfähigkeit von bis zu 5 % oder von bis zu 10%, z. B. je nach Wahl einer Leiterbahnengeometrie oder je nach Kabelführung entlang der Trägerstruktur, auf. Die hohe Biegeschlaffheit kann ein lösbares Versteifungselement auf der Haut abgewandten Seite der Trägerstruktur erfordern, um für den Prozess des Aufbringens die notwendige Steifigkeit zu erhalten, wobei das Versteifungselement z. B. Teil der Trägerstruktur ist. Nach oder während des Aufbringens kann dieses zusätzliche Versteifungselement entfernt werden, um die geringe Dicke und die volle Dehnbarkeit für den Tragekomfort ausnutzen zu können.

Polyurethanschaum mit einer Dicke bis zu 1mm weist eine nicht so hohe Biegeschlaffheit wie das dünne Polyurethan-Folienmaterial auf, dafür ist es aber stark dehnbar (bis zu 20% der Länge) und ist ebenfalls sehr leicht verformbar (geringer Druck der Finger) und erfüllt so auch die hier gestellten Anforderungen.

Generell ist ein Material im Sinne dieser Anmeldung biegeschlaff, wenn dieses in sämtliche Richtungen verformbar bzw. verbiegbar ist sowie verdreht werden kann ohne beschädigt zu werden, z.B. ohne zu reißen. Unter Biegeschlaffheit kann z. B. verstanden werden, wenn sich eine Folie bzw. Schicht aus dem Material bereits lediglich unter Einfluss der Gravitationskraft, d.h. ohne weitere äußere Krafteinwirkung oder nur mit geringer äußerer Krafteinwirkung (z. B. F≤1 N), verbiegt. Die Biegeschlaffheit erlaubt die flächige Anpassung an Unebenheiten von Gesicht- und Kopfstrukturen. Wird die Struktur (z. B. die Trägerstruktur mit Polyurethanfolie mit einer maximalen Dicke von 0.1 mm, 0.3 mm oder 0.5 mm oder die Trägerstruktur mit Polyurethanschaum mit einer Dicke in einem Bereich von 0.5 mm bis 2 mm) auf eine radiale Erhebung (als Zylinder hier angenommen) mit einem Durchmesser von z.B. 0.5 cm und einer Höhe von 1 cm gelegt, kann das Trägermaterial gleichzeitig durch das Eigengewicht der Schwerkraft folgend oder durch geringe Kraftaufbringung (< 1N) jeweils auf der Kreisfläche und auf vier Stellen der Zylindermantelfläche flächig zum Liegen kommen, wovon jeweils zwei der Bereiche auf der Mantelfläche sich gegenüberliegen und die Verbindungslinien der jeweils gegenüberliegenden Flächen zueinander senkrecht stehen, ohne dass dabei die Trägerstruktur beschädigt wird (reißen oder brechen). Dabei folgt die Struktur der 90° Grad Ecke von Kreisfläche auf Zylindermantelfläche mit einem eng anliegenden Radius von maximal 1mm. Ist eine Schicht oder Struktur biegeschlaff, so folgt diese beispielsweise sämtlichen Unebenheiten. Biegeschlaffe Strukturen und Schichten sind z. B. gekennzeichnet durch einen niedrigen Elastizitätsmodul, geringe Dehnsteifigkeit und daher große Verformungen bereits infolge geringer Kraft- und Momentbeanspruchung. Unter Biegeschlaffheit kann z. B. verstanden werden, wenn eine Struktur gleichzeitig entlang zwei zueinander senkrecht stehende laterale Richtungen in einer Dickenrichtung der Struktur verformbar ist ohne beschädigt zu werden und/oder wenn die Struktur mehrfach ineinander verdrehbar ist ohne beschädigt zu werden, d.h. wenn beispielsweise ein Torsionsmoment auf die Struktur wirkt, um diese mehrfach um ein Achse senkrecht zu der Dickenrichtung zu verdrehen.

Die Gesamtstruktur soll ohne Einwirkung des Nutzers 210 formstabil bleiben, damit die Vorrichtung 100 leicht von dem Nutzer 210 selbst anlegbar ist. Weist die Trägerstruktur beispielsweise das dünne Polyurethan-Folienmaterial auf, so weist die Trägerstruktur optional ferner weitere Materialien oder Elemente auf, die ausgebildet sind, um die Trägerstruktur zu stabilisieren und somit die Formstabilität der Trägerstruktur zu gewährleisten. Diese Verstärkungsmaterialien oder Verstärkungselemente sind z. B. lösbar an dem dünnen Polyurethan-Folienmaterial angeordnet, so dass die Trägerstruktur, wenn diese Polyurethan-Folienmaterial und ein Verstärkungsmaterial oder ein Verstärkungselement aufweist formstabil ist und wenn das Verstärkungsmaterial oder das Verstärkungselement von dem Polyurethan-Folienmaterial gelöst ist die Trägerstruktur biegeschlaff ist. Bei Polyurethanschaum ist hingegen keine Stützstruktur notwendig, da das Material selbst schon eine Formstabilität bei gleichzeitiger Dehnbarkeit unter Nutzereinwirkung mit sich bringt. Optional kann die Trägerstruktur aber auch bei einem Trägermaterial mit Polyurethanschaum weiter stabilisiert werden.

Die Formstabilität der Trägerstruktur im Falle keiner äußeren Krafteinwirkung des Nutzers 210 kann auf verschiedene Arten erzielt werden:
Gemäß einem Ausführungsbeispiel weist die Trägerstruktur 120 ein Klebematerial zur Fixierung der Vielzahl von Elektroden 110₁ bis 110₅ und der Trägerstruktur 120 an der Gesichtsoberfläche und hinter dem Ohr 230 auf. Das Klebematerial ist z. B. von einer lösbaren Schutzschicht bedeckt. Die Schutzschicht weist z. B. ein Folien- oder Papiermaterial auf. Die Schutzschicht weist z. B. eine Mehrzahl von Schutzschichtabschnitten auf, die unabhängig voneinander von der Trägerstruktur 120 lösbar sind. Das Folien- oder Papiermaterial zur Abdeckung der Klebeschicht bzw. des Klebematerials kann zusätzliche Stabilität bringen, da das Folien- oder Papiermaterial beim Aufbringen der Trägerstruktur 120 an dem Kopf 200 in einzelnen Passagen, d.h. in einzelnen Schutzschichtabschnitten, abgezogen werden kann, und so Stabilität während des Prozesses aufrecht gehalten werden kann.

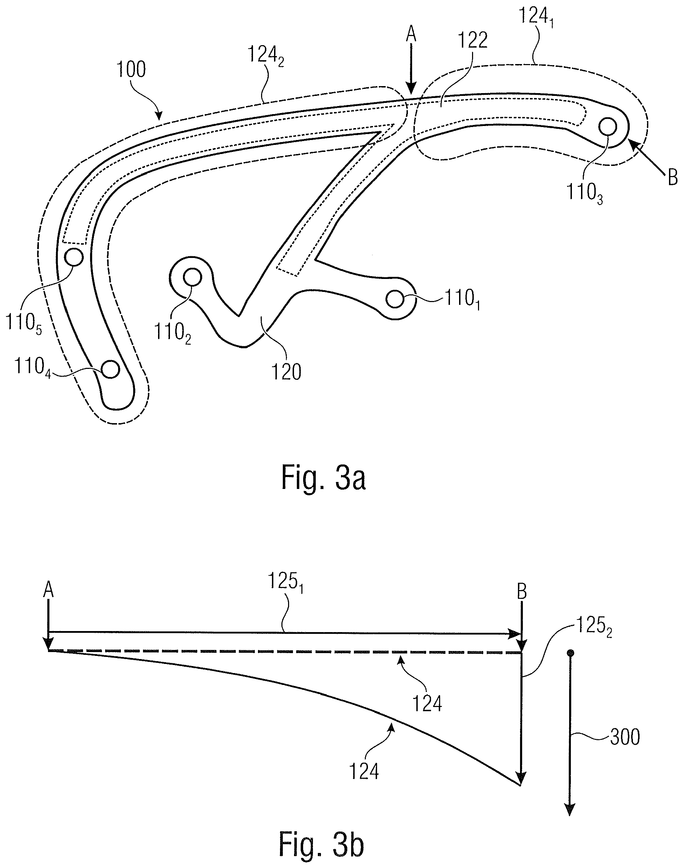

Zusätzlich oder alternativ kann auf der Haut abgewandten Seite der Trägerstruktur 120 eine Stützstruktur z. B. in Form von Folie (z. B. aus Polyimidmaterial, Polyestermaterial, Polyethylenmaterial oder FR4-Material) oder Papier oder Polyurethanschaum aufgebracht sein. Die Stützstruktur kann auch als Versteifungselement 122 angesehen werden. Wie in Fig. 3a dargestellt, kann die Trägerstruktur 120 auf einer der Elektrodenanordnung 110 abgewandten Seite ein lösbares Versteifungselement 122 aufweisen, das ausgebildet ist, um die Trägerstruktur 120 zumindest lokal zu versteifen. Das Versteifungselement 122 ist z. B. nur während eines Aufbringungsprozesses der Trägerstruktur 120 an der Kopfoberfläche, d.h. auf der Gesichtsoberfläche 220 und hinter dem Ohr 230, vorhanden. Nach dem oder während des Aufkleben bzw. Aufbringen der Trägerstruktur 120, was wie oben beschrieben ggf. auch stückweise erfolgen kann, kann das Versteifungselement 122 von der Trägerstruktur 120 abgezogen werden. Dies ist besonders für dünne Trägermaterialien mit sehr hoher Biegeschlaffheit relevant, da das Versteifungselement 122 der Biegeschlaffheit des Trägermaterials entgegenwirkt und damit ein selbstständiges Anbringen der Vorrichtung 100 an einer Kopfoberfläche des Nutzers 210 wesentlich erleichtert. Dadurch, dass das Versteifungselement 122 nach dem Anbringen der Trägerstruktur 120 an einer Kopfoberfläche des Nutzers 210 wieder von der Trägerstruktur gelöst werden kann, wird der Tragekomfort für den Nutzer 210 erhöht, da das Trägermaterial ohne dem Versteifungselement 122 deformierbar ist und somit ausgelegt ist, um sich mit Bewegungen in dem Gesicht, wie z. B Muskelbewegungen oder auch Kieferbewegungen, mitzuverformen. Die Klebeverbindung zwischen dem Versteifungselement 122 und der Trägerstruktur 120 ist z. B. ausgebildet, um bei einem entfernen des Versteifungselements keine Klebereste zu hinterlassen.

In Fig. 3a ist eine Vorrichtung 100 dargestellt, die alle Merkmale und Funktionalitäten aufweisen kann, die auch in Zusammenhang mit der Vorrichtung aus Fig. 1a und 1b beschrieben worden sind. Fig. 3a zeigt ein Versteifungselement 122, das auf einem sich verzweigenden Verbindungssteg der Trägerstruktur 120 angeordnet ist. Der Verbindungssteg ist z. B. ausgebildet, um die erste Elektrode 110₁ und die zweite Elektrode 110₂ mit der dritten Elektrode 110₃ einerseits und mit der vierten Elektrode 110₄ und der fünften Elektrode 110₅ andererseits zu verbinden. Der Verbindungssteg verzweigt sich, wobei ein erster Teil 124₁ des Verbindungssteges zu der dritten Elektrode 110₃ führt und ein zweiter Teil 124₂ des Verbindungssteges zu der vierten Elektrode 110₄ und der fünften Elektrode 110₅ führt. Durch das Versteifungselement auf dem Verbindungssteg wird die Form der Verbindung zwischen den Elektroden aufrechterhalten. Dies ist insbesondere für die Positionierung der Trägerstruktur 120 an der Kopfoberfläche wichtig, da die Form des Verbindungssteges die Relativposition der Elektroden zueinander zumindest teilweise definiert und ausgebildet ist, um die Elektroden so auf der Kopfoberfläche zu positionieren, dass die Elektroden vorbestimmte Positionen auf der Kopfoberfläche einnehmen. Alternativ zu dem einen Versteifungselement 122 kann die Vorrichtung 100 auch mehrere Versteifungselemente aufweisen oder auch ein Versteifungselement 122, das die komplette Oberfläche der Trägerstruktur 120 (d. h. die Oberfläche auf der der Elektrodenanordnung 110 abgewandten Seite) abdeckt.

Eine weitere Möglichkeit besteht darin, die Trägerstruktur mit einer größeren Dicke auszuführen. Weist die Trägerstruktur 120 als Trägermaterial z. B. Polyurethanschaummaterial auf, kann dieses z. B. mit einer Dicke in einem Bereich von 0.5 mm bis zu 2 mm ausgeführt werden, um die Formstabilität der Trägerstruktur ohne zusätzliches Versteifungselement 122 zu erzielen. Aber auch in diesem Fall kann die Trägerstruktur 120 optional zusätzlich ein Versteifungselement aufweisen.

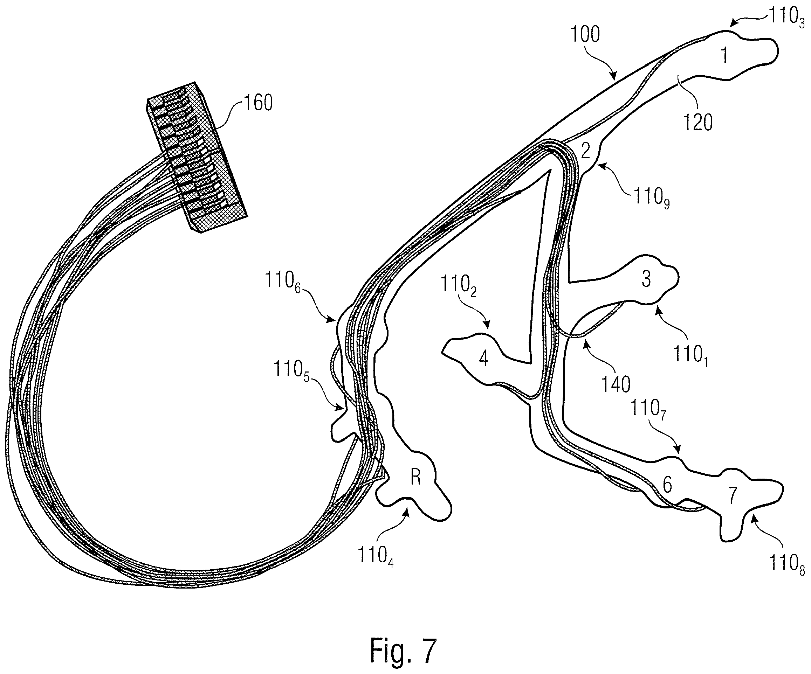

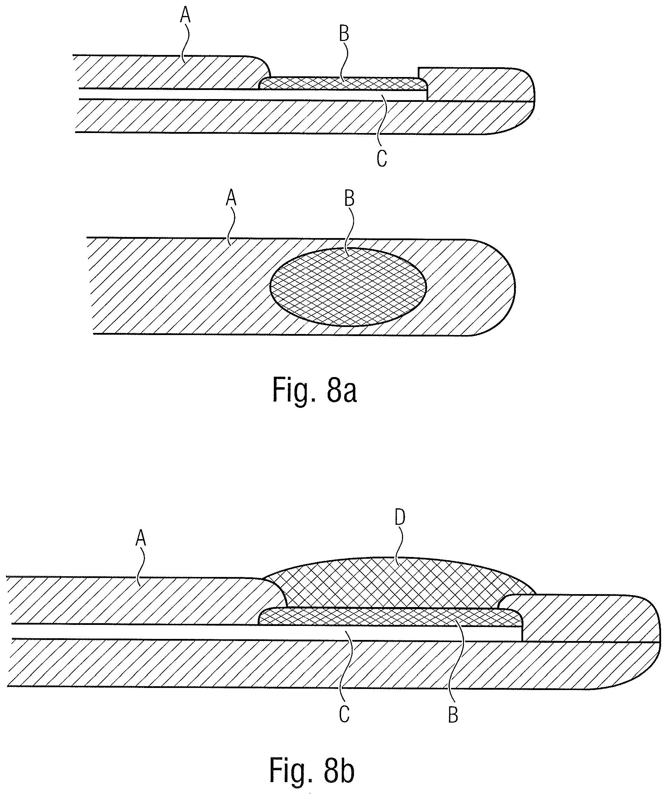

Als Materialien für die Trägerstruktur 120 eigenen sich besonders Polyurethane, als dünne Trägerfolie mit dann entsprechend aufgebrachter zusätzlicher, temporärer Stützstruktur, d. h. mit dem Versteifungselement 122, oder als Polyurethanschaum für Dicken in einem Bereich 0.5 mm bis 2 mm. Vorzugsweise werden auf der Polyurethanfolie die Leiterbahnen direkt aufgebracht bzw. die Leiterbahnen (abgesehen von den Elektrodenflächen) in eine Polyurethan-Sandwich-Konstruktion eingebracht (siehe die Figuren 8a und 8b mit zugehöriger Beschreibung), die Polyurethanschaumausführung kann als Trägerstruktur für herkömmliche Festgelelektroden mit Kabeln dienen (siehe Fig. 7 mit zugehöriger Beschreibung).

Die Formstabilität der Gridstruktur, d. h. der Trägerstruktur 120, mit entsprechender Stützstruktur, d. h. mit dem Versteifungselement 122, wird in Fig. 3b exemplarisch daran gezeigt, wie sich ein Arm 124, d. h. ein Verbindungssteg, der Länge 10 cm (siehe 125₁) nach unten, der Schwerkraft 300 folgend, neigt, wenn der Arm 124 horizontal (senkrecht zu der Schwerkraft 300) ausgerichtet wird. Der Arm 124 neigt sich auf dieser Länge nicht weiter als etwa 6 cm (siehe 125₂). Wendet man dies z. B. auf den Verbindungssteg 124₁ der Fig. 3a an, so neigt sich der Verbindungssteg 124₁, der eine Länge von ca. 7 cm zwischen den Punkten A und B aufweist (siehe 125₁), nicht weiter als ca. 4 cm (siehe 125₂). Allgemein ist ein Verbindungssteg der Trägerstruktur 125 ausgebildet, um ohne äußere Krafteinwirkung des Nutzers 210 innerhalb eines Toleranzbereichs formstabil zu sein. Ein horizontal ausgerichteter Verbindungssteg der Trägerstruktur 120 ist z. B. ausgebildet, um bei einer Länge von bis zu maximal 12 cm unter Einfluss der Schwerkraft 300 sich maximal um 60 % seiner Länge, der Schwerkraft folgend, zu neigen. Die Limitierung der Neigung bzw. Biegung kann unter anderem mittels eines der oben diskutierten Merkmalen, wie Klebeschutzfolie, Versteifungselement 122 oder spezieller Dicke der Trägerstruktur, erzielt werden. Während der Anbringung wird das Grid, d. h. die Trägerstruktur, aufrecht gehalten, so dass der Arm 124 sich dann noch weniger neigt, da er sich in einem anderen Winkel zur Schwerkraft 300 befindet. Fig. 3b zeigt somit exemplarisch eine Steifigkeit der Trägerstruktur 120 mit Stützstruktur bzw. eine Wirkung der Stützstruktur auf die Formstabilität der Trägerstruktur 120.

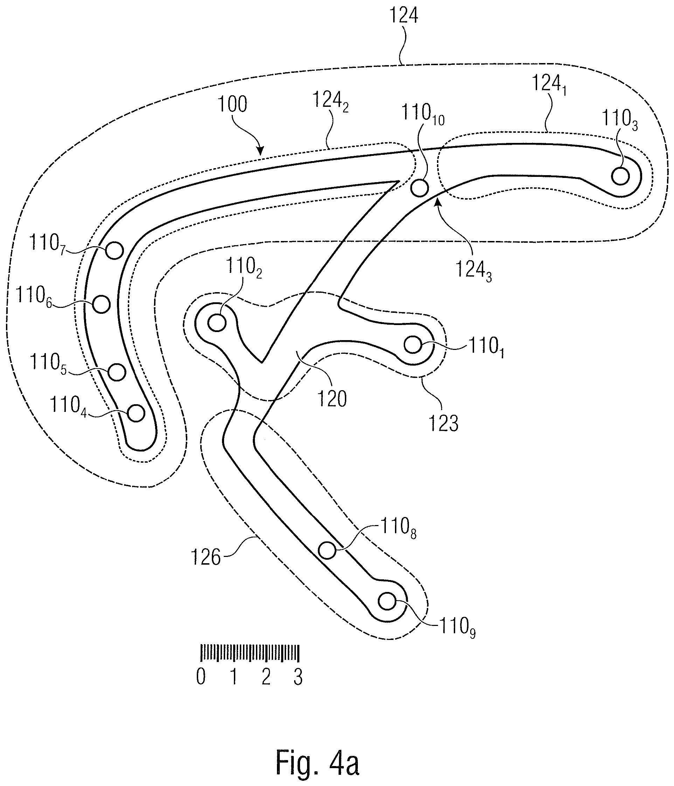

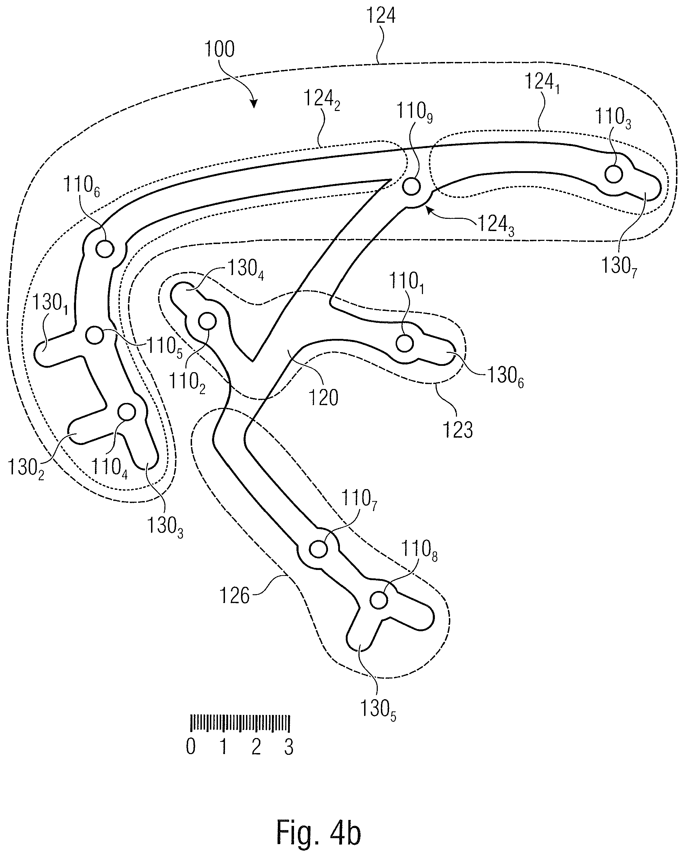

Die Figuren 4a und 4b zeigen eine weitere Ausführungsform der erfindungsgemäßen Vorrichtung 100. Optional kann die Vorrichtung Merkmale und Funktionalitäten, wie sie in Zusammenhang mit Ausführungsbeispielen der Figuren 1a bis 3b beschrieben worden sind, aufweisen. Ebenso ist es möglich, dass Ausführungsbeispiele aus den Figuren 1b bis 3b Merkmale oder Funktionalitäten aufweisen, wie sie im Folgenden beschrieben werden. Die Vorrichtung in Fig. 4a unterscheidet sich von der Vorrichtung in Fig. 1a lediglich darin, dass mehr Elektroden auf der Trägerstruktur 120 angeordnet sind und die Trägerstruktur 120 ferner einen Verbindungssteg aufweist, der die erste Elektrode 110₁ und die zweite Elektrode 110₂ mit Elektroden verbindet, deren Positionen auf einem Kinn des Nutzers zugeordnet werden können. Die Vorrichtung in Fig. 4b unterscheidet sich von der Vorrichtung in Fig. 4a lediglich in der Anzahl und Durchmesser der Elektroden und in Ausbuchtungen der Trägerstruktur an bestimmten Elektrodenpositionen.

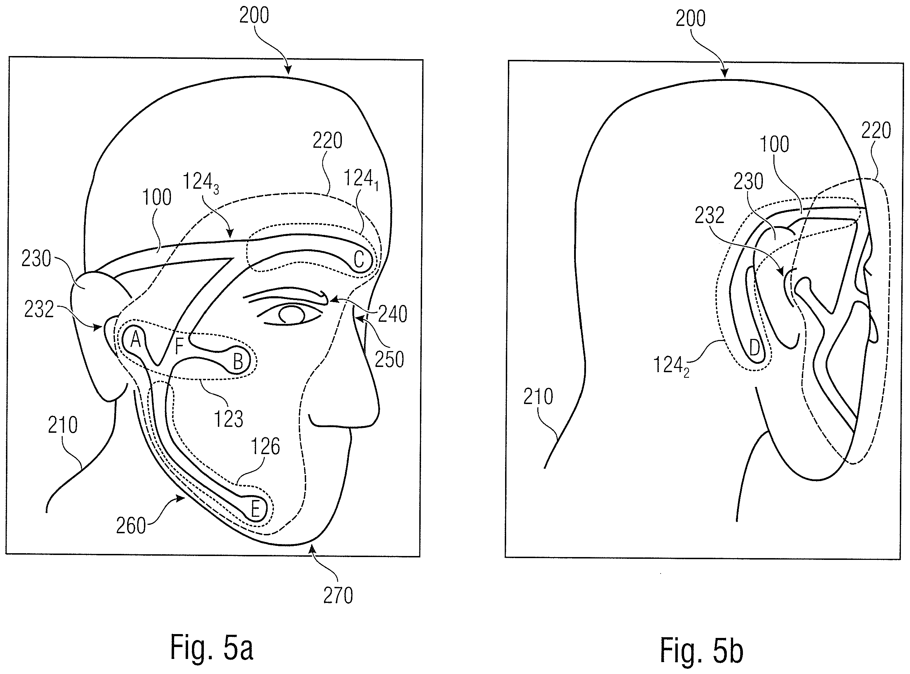

Wie in Fig. 4a dargestellt, stellt die Formgebung der Trägerstruktur 120, auf der die Vielzahl von Elektroden 110₁ bis 110₁₀ (bzw. 110₁ bis 110₉ in Fig. 4b) angeordnet ist, ein besonderes Merkmal der vorliegenden Erfindung dar. Die Formgebung orientiert sich dabei an Strukturen an einem Kopf 200 des Nutzers 210. In den Figuren 5a und 5b ist beispielhaft die Vorrichtung 100 aus Fig. 4a und Fig. 4b an dem Kopf 200 des Nutzers 210 angeordnet. Fig. 5a zeigt eine Platzierung der Vorrichtung 100 in einer Seitansicht des Kopfes 200 des Nutzers 210 und Fig. 5b zeigt eine Platzierung der Vorrichtung 100 in einer Ansicht von einem Hinterkopf des Nutzers 210 aus.

Die Trägerstruktur weist z. B. einen ersten Verbindungssteg 123 auf, der die erste Elektrode 110₁ und die zweite Elektrode 110₂ miteinander verbindet, wobei die erste Elektrode 110₁ an einem ersten Ende des ersten Verbindungsstegs 123 angeordnet ist und die zweite Elektrode 110₂ an einem zweiten Ende des ersten Verbindungsstegs 123 angeordnet ist. Der erste Verbindungssteg 123 weist z. B. einen gewunden bzw. geschlungenen Verlauf, wie z. B. einen wellenförmigen Verlauf, auf. Durch den gewundenen Verlauf des Verbindungsstegs zwischen der ersten Elektrode 110₁ und der zweiten Elektrode 110₂ wird eine sehr formstabile Verbindung zwischen den beiden Elektroden realisiert. Dies ist insbesondere vorteilhaft, da die erste Elektrode und/oder die zweite Elektrode eine Referenz für die Positionen der restlichen Elektroden der Vielzahl von Elektroden 110₁ bis 110₁₀ (bzw. 110₁ bis 110₉ in Fig. 4b) darstellen können und somit sichergestellt wird, dass der Nutzer selbstständig diese beiden Elektroden sehr exakt auf der Gesichtsoberfläche 220 anbringen kann. Ferner erhöht der spezielle Verlauf des ersten Verbindungsstegs 123 den Tragekomfort. Dies begründet sich darauf, dass eine Position der ersten Elektrode 110₁ auf der Gesichtsoberfläche 220 z. B. einer Position auf einem Wangenknochen, d.h. auf einem ersten markanten Punkt der Gesichtsoberfläche, entspricht und eine Position der zweiten Elektrode 110₂ auf der Gesichtsoberfläche 220 z. B. einer Position vor einem Tragus 232 des Ohres 230, d.h. auf einem zweiten markanten Punkt der Gesichtsoberfläche, entspricht. Durch den geschwungenen Verlauf des ersten Verbindungsstegs 123 und durch die Deformierbarkeit bzw. Elastizität des Trägermaterials der Trägerstruktur 120 ist diese ausgebildet, um Gesichtsbewegungen in einem Bereich zwischen der ersten Elektrode 110₁ und der zweiten Elektrode 110₂ zu folgen. Hierdurch wird der Tragekomfort erhöht.

Gemäß einem Ausführungsbeispiel weist die Trägerstruktur 120 z. B. einen sich verzweigenden zweiten Verbindungssteg 124 auf, der die erste Elektrode 110₁ und die zweite Elektrode 110₂ mit einer dritten Elektrode 110₃ einerseits und mit einer vierten Elektrode 110₄ andererseits verbindet (die vierte Elektrode kann auch eine der Elektroden 110₅ bis 110₇ aus Fig. 4a sein oder die Elektrode 110₅ oder 110₆ aus Fig. 4b sein), wobei sich der zweite Verbindungssteg 124 an einer Verzweigungsstelle 124₃ in einen ersten Teil 124₁ und in einen zweiten Teil 124₂ verzweigt.

Der erste Teil 124₁ des zweiten Verbindungsstegs 124 ist ausgebildet, um die erste Elektrode 110₁ und die zweite Elektrode 110₂ mit der dritten Elektrode 110₃ zu verbinden Der zweite Verbindungssteg 124 führt z. B. ca. von einer Mitte des ersten Verbindungsstegs 123 über den ersten Teil 124₁ zu der dritten Elektrode 110₃, wobei die dritte Elektrode 110₃ an einem Ende des ersten Teils 124₁ angeordnet ist. Der zweite Verbindungssteg 124 verläuft von einer Mitte des ersten Verbindungsstegs 123 über den ersten Teil 124₁ in einem Bogen bzw. in einer gekrümmten Form. Der zweite Verbindungssteg folgt zusammen mit dem ersten Teil 124₁ z. B. einer Form eines Auges bzw. einer Augenbraue 240. Ist die Trägerstruktur 120 an einer Kopfoberfläche des Nutzers angeordnet, so verläuft der zweite Verbindungssteg 124 oberhalb entlang der Augenbraue 240 zu der dritten Elektrode 110₃. Die Position der dritten Elektrode 110₃ entspricht auf der Gesichtsoberfläche 220 z. B. einer Position senkrecht oberhalb eines Nasions 250 des Nutzers 210, d.h. einem dritten markanten Punkt auf der Gesichtsoberfläche 220. In anderen Worten, weist die Trägerstruktur einen Verbindungssteg auf, nämlich den zweiten Verbindungssteg 124, der eine erste Elektrodenposition, die der ersten Elektrode 110₁ der Vielzahl von Elektroden zugeordnet ist, und/oder eine zweite Elektrodenposition, die der zweiten Elektrode 110₂ der Vielzahl von Elektroden zugeordnet ist, einerseits mit einer dritten Elektrodenposition, die der dritten Elektrode 110₃ der Vielzahl von Elektroden zugeordnet ist, andererseits zumindest teilweise verbindet und wobei die dritte Elektrodenposition innerhalb eines durch die Trägerstruktur 120 bestimmten Toleranzbereichs von 1 bis 2 cm beidseitig neben einer Position auf einer Stirn der Gesichtsoberfläche in einem Bereich von 1 cm bis 3 cm oberhalb der Augenbrauenlinie senkrecht oberhalb eines Nasions 250 des Nutzers 210 übereinstimmt.

Der zweite Teil 124₂ des zweiten Verbindungsstegs 124 ist ausgebildet, um die erste Elektrode 110₁ und die zweite Elektrode 110₂ mit der vierten Elektrode 110₄ zu verbinden. Optional können auf dem zweiten Teil 124₂ des zweiten Verbindungsstege 124 weitere Elektroden (z. B. die Elektroden 110₅ bis 110₇ aus Fig. 4a oder die Elektroden 110₅ und 110₆ aus Fig. 4b) der Vielzahl von Elektroden angeordnet sein, weshalb der zweite Teil 124₂ des zweiten Verbindungsstege 124 ferner ausgebildet sein kann, um die erste Elektrode 110₁ und die zweite Elektrode 110₂ mit den weiteren Elektroden zu verbinden. Der zweite Teil 124₂ des zweiten Verbindungsstegs 124 verläuft z. B. von der Verzweigungsstelle 124₃ in entgegengesetzte Richtung als der erste Teil 124₁ des zweiten Verbindungsstegs 124. An einem Ende des zweiten Teils 124₂ ist der zweite Verbindungssteg 124 z. B. gekrümmt bzw. er verläuft in einem Bogen. Der Bogen des zweiten Verbindungsstegs verläuft z. B. um die Position der zweiten Elektrode 110₂. Ist die Trägerstruktur 120 an der Kopfoberfläche des Nutzers 210 angeordnet, so verläuft der Bogen des zweiten Verbindungsstegs 124 z. B. entlang der Form des Ohrs 230. Die vierte Elektrode 110₄ sowie mögliche weitere Elektroden der Vielzahl von Elektroden sind vorzugsweise auf dem Bogen des zweiten Verbindungsstegs 124 angeordnet. Die Position der vierten Elektrode 110₄ entspricht auf der Kopfoberfläche z. B. einer Position hinter dem Ohr 230 des Nutzers 210. Optional entspricht die Position der vierten Elektroden der Position des Mastoids (siehe Punkt D in Fig. 5b), d.h. einem vierten markanten Punkt auf der Kopfoberfläche. In anderen Worten, weist die Trägerstruktur einen Verbindungssteg auf, nämlich den zweiten Verbindungssteg 124, der die erste Elektrodenposition und/oder die zweite Elektrodenposition einerseits mit einer vierten Elektrodenposition, die der vierten Elektrode 110₄ der Vielzahl von Elektroden zugeordnet ist, andererseits zumindest teilweise verbindet und wobei die vierte Elektrodenposition innerhalb eines durch die Trägerstruktur 120 bestimmten Toleranzbereichs mit einer Position hinter dem Ohr 230 an einem Mastoid des Nutzers 210 übereinstimmt. Der zweite Teil 124₂ des zweiten Verbindungsstegs 124 führt z. B. oberhalb des Ohrs 230 des Nutzers 210 zu dem Bogen, der hinter dem Ohr 230 der Form des Ohrs 230 folgt.

Gemäß einem Ausführungsbeispiel kann eine Elektrode (z. B. 110₁₀ in Fig. 4a oder 110₉ in Fig. 4b) der Vielzahl von Elektroden in einem Bereich der Verzweigungsstelle 124₃ des zweiten Verbindungsstege 124 angeordnet sein. Hierdurch bilden die erste Elektrode 110₁, die dritte Elektrode 110₃ und die Elektrode 110₁₀/110₉ in dem Bereich der Verzweigungsstelle 124₃ des zweiten Verbindungsstege 124 z. B. ein Dreieck. Das Dreieck weist mit einem Toleranzbereich von ±5° einen rechten Winkel bei der Elektrode 110₁₀/110₉ in dem Bereich der Verzweigungsstelle 124₃ des zweiten Verbindungsstegs 124 auf. Durch die spezielle Anordnung dieser drei Elektroden kann eine Augenbewegung mit der Vorrichtung 100 sehr exakt erfasst werden.

Optional weist die Trägerstruktur 120 ferner einen dritten Verbindungssteg 126 auf, der die erste Elektrode 110₁ und die zweite Elektrode 110₂ mit einer fünften Elektrode (z. B. 110₉ in Fig. 4a oder 110₃ in Fig. 4b) verbindet. Der dritte Verbindungssteg 126 verläuft z. B. von ca. der Mitte des ersten Verbindungsstegs 123 in entgegengesetzte Richtung als der zweite Verbindungssteg 124, wobei die fünfte Elektrode 110₉/110₈ an einem Ende des dritten Verbindungsstegs 126 angeordnet ist. Der dritte Verbindungssteg 126 weist z. B. einen gekrümmten Verlauf auf. Ist die Trägerstruktur 120 an einer Gesichtsoberfläche 220 des Nutzers 210 angeordnet, so verläuft der dritte Verbindungssteg 126 entlang eines Kiefers 260 zu der fünften Elektrode 110₉/110₈. Die Position der fünften Elektrode 110₉/110₈ entspricht auf der Gesichtsoberfläche 220 z. B. einer Position auf oder an einem Kinn 270 des Nutzers 210, d.h. einem fünften markanten Punkt auf der Gesichtsoberfläche 220. Optional können weitere Elektroden der Vielzahl von Elektroden auf dem dritten Verbindungssteg 126 angeordnet sein. In anderen Worten, weist die Trägerstruktur 120 einen Verbindungssteg auf, nämlich den dritten Verbindungssteg 126, der die erste Elektrodenposition und/oder die zweite Elektrodenposition einerseits mit einer fünften Elektrodenposition, die der fünften Elektrode 110₉/110₈ der Vielzahl von Elektroden zugeordnet ist, andererseits zumindest teilweise verbindet und wobei die fünfte Elektrodenposition innerhalb eines durch die Trägerstruktur 120 bestimmten Toleranzbereichs mit einer Position auf dem Kinn 270 der Gesichtsoberfläche 220 des Nutzers 210 übereinstimmt.

Die Verbindungsstege der Trägerstruktur 120 weisen eine Stegbreite auf, die höchstens einem dreifachen, einem zweifachen oder einem 1,5fachen Elektrodendurchmesser einer Elektrode der Vielzahl von Elektroden entspricht. Übergangsbereiche, d.h. die Verbindungsstege, können als "schlanke Taille" ausgeführt werden, um eine optimale Flexibilität zur Anpassung an verschiedene Kopfformen und Kopfgrößen zu erreichen.

Die erste Elektrode 110₁ und/oder die zweite Elektrode 110₂ definieren/definiert beispielsweise eine Referenzposition und die restlichen Elektroden der Vielzahl von Elektroden sind auf der Trägerstruktur 120 relativ zu der Referenzposition angeordnet. Gemäß einem Ausführungsbeispiel kann eine erste Elektrodenposition, die der Position der ersten Elektrode 110₁ auf der Trägerstruktur 120 entspricht, als Referenzposition dienen, oder eine zweite Elektrodenposition die der Position der zweiten Elektrode 110₂ auf der Trägerstruktur 120 entspricht, als Referenzposition dienen. Alternativ ist es auch möglich, dass die restlichen Elektroden der Vielzahl von Elektroden relativ zu der ersten Elektrodenposition und der zweiten Elektrodenposition auf der Trägerstruktur 120 angeordnet sind. In diesem Fall könnte z. B. eine Position mittig zwischen den beiden Elektrodenpositionen als Referenzposition dienen. Wie bereits oben erwähnt dienen die Positionen der ersten und zweiten Elektrode besonders gut als Referenzpositionen, da diese von dem Nutzer sehr exakt auf dessen Kopfoberfläche positioniert werden können. Basierend auf diesen beiden Positionen kann die Trägerstruktur z. B. mittels des zweiten Verbindungsstegs und mittels des optionalen dritten Verbindungsstegs die Positionen der restlichen Elektroden der Vielzahl von Elektroden auf der Kopfoberfläche, d. h. auf der Gesichtsoberfläche 220 und hinter dem Ohr 230, sehr exakt definieren.

Gemäß einem Ausführungsbeispiel weist die Trägerstruktur 120 ein Klebematerial zur Fixierung der Vielzahl von Elektroden und der Trägerstruktur 120 an der Kopfoberfläche, d.h. auf der Gesichtsoberfläche 220 und hinter dem Ohr 230, auf. Das Klebematerial ist z. B. auf der Seite der Trägerstruktur 120, auf der auch die Elektrodenanordnung angeordnet ist, aufgebracht. Das Klebematerial ist somit auf der der Haut des Nutzers zugewandten Seite der Trägerstruktur angeordnet. Optional können die Elektroden der Vielzahl von Elektroden zusätzlich oder alternativ zu dem Klebematerial ein klebendes Elektrolytgel aufweisen, das ausgebildet ist, um die Vielzahl von Elektroden auf der Kopfoberfläche zu fixieren und die Impedanz zu senken. Das Klebematerial und/oder das klebende Elektrolytgel sind zumindest bereichsweise mit einer Schutzfolie bedeckt.

Die Schutzfolie kann beispielsweise in eine Mehrzahl von Schutzfolienabschnitte unterteilt sein, die unabhängig voneinander von der Trägerstruktur 120 und/oder der Vielzahl von Elektroden lösbar sind. Ein erster Schutzfolienabschnitt der Mehrzahl von Schutzfolienabschnitte bedeckt z. B. eine erste Teilmenge der Vielzahl von Elektroden und der Trägerstruktur 120 und ein zweiter Schutzfolienabschnitt der Mehrzahl von Schutzfolienabschnitte bedeckt z. B. eine disjunkte zweite Teilmenge der Vielzahl von Elektroden und der Trägerstruktur 120. Der erste Schutzfolienabschnitt bedeckt z. B. den ersten Verbindungssteg 123, der die erste Elektrode 110₁ und die zweite Elektrode 110₂ aufweist. Der zweite Schutzfolienabschnitt bedeckt z. B. den zweiten Verbindungssteg 124, der unter anderem die dritte Elektrode 110₃ und die vierte Elektrode 110₄ und optional weitere Elektroden der Vielzahl von Elektroden aufweist. Optional kann der zweite Verbindungssteg 124 auch von zwei unterschiedlichen Schutzfolienabschnitten bedeckt sein. Ein Schutzfolienabschnitt (z. B. der zweite Schutzfolienabschnitt) bedeckt dann z. B. den Teil des zweiten Verbindungsstegs 124, der mit dem ersten Verbindungssteg 123 in Verbindung steht und ferner den ersten Teil 124₁ des zweiten Verbindungsstegs 124 aufweist und ein weiterer Schutzfolienabschnitt (z. B. ein dritter Schutzfolienabschnitt) bedeckt dann z. B. nur den zweiten Teil 124₂ des zweiten Verbindungsstegs. Ein optionaler weiterer Schutzfolienabschnitt bedeckt z. B. den dritten Verbindungssteg 126, der zumindest die fünfte Elektrode 110₉/110₈ der Vielzahl von Elektroden aufweist. Diese Aufteilung der Schutzfolienabschnitte ist besonders vorteilhaft, da immer von dem Bereich, der als nächstes auf der Kopfoberfläche des Nutzers 210 angebracht werden soll, der Schutzfolienabschnitt entfernt werden kann und dadurch die Vorrichtung bei der Anbringung einfach zu handhaben ist, da immer nur für einen kleinen Bereich das Klebematerial offen zugänglich ist. Ferner wird durch die besondere Wahl des ersten Schutzfolienabschnitts ermöglicht, dass der Nutzer sich beim Anbringen zunächst vollständig auf die Positionierung der ersten Elektrode und der zweiten Elektrode konzentrieren kann, wodurch diese sehr genau positioniert werden können. Die Positionen der restlichen Elektroden der Vielzahl von Elektroden auf der Kopfoberfläche ergibt sich dann aus der Formgebung der Trägerstruktur 120, insbesondere aus der Form des zweiten Verbindungsstegs 124 und des optionalen dritten Verbindungssteg 126. Somit führt die Mehrzahl an Schutzfolienabschnitten zu einer hohen Genauigkeit bei der Positionierung der Vorrichtung 100 und ermöglicht ferner eine selbstständige Positionierung und Fixierung dieser an der Kopfoberfläche des Nutzers 210 durch den Nutzer 210.

Vorteilhaft sind bei der Positionierung und Fixierung der Vorrichtung 100 an der Kopfoberfläche des Nutzers 210 ferner Fortsätze 130₁ bis 130₇ bzw. Nasen, wie sie z. B. in Fig. 4b dargestellt sind. Die Trägerstruktur 120 weist z. B. an einer Position, an der eine Elektrode der Elektrodenanordnung 110 angeordnet ist, zumindest einen Fortsatz 130₁ bis 130₇ auf. Wie z. B. für die vierte Elektrode 110₄ dargestellt ist, kann die Trägerstruktur 120 auch mehrere Fortsätze, wie z. B. zwei Fortsätze, an einer Position, an der eine Elektrode der Elektrodenanordnung 110 angeordnet ist, aufweisen. Auf den Fortsätzen ist z. B. ein Klebematerial angeordnet, wodurch die Fixierung der Elektroden, an deren Position die Trägerstruktur zumindest einen Fortsatz 130₁ bis 130₇ aufweist, auf der Gesichtsoberfläche verbessert werden kann.

Die Positionen der Elektroden der Elektrodenanordnung 110 wurden so ausgewählt, dass einerseits nur von Haaren freiliegende Hautpartien zur Anbringung z. B. eines klebenden Trägersubstrats verwendet werden und andererseits aus Linearkombinationen der Elektrodensignale sich Signalanteile der klassisch zu verwendenden PSG-Elektroden ergeben. Das Vorgehen zur Bildung von Linearkombinationen ist in [Da Silva Souto, C. F., Pätzold, W., Wolf, I., Paul, M., Matthiesen, I., Bleichner, M. G., & Debener, S. (2021). "Flexprinted ear-EEG sensors for adequate sleep staging at home."; Frontiers in Digital Health, 3, 66] auf Basis der flexiblen Elektrodenlösung cEEGrid demonstriert.

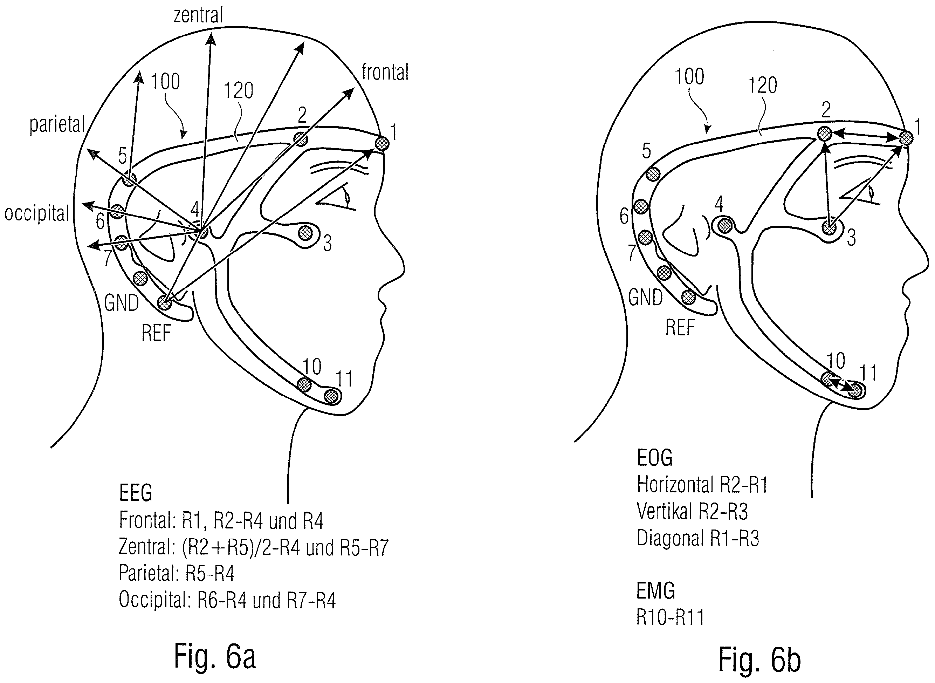

Wie in den Figuren 6a und 6b dargestellt, ist die Form der Trägerstruktur 120 z. B. ausgebildet, um die Vielzahl von Elektroden an einer korrespondierenden Vielzahl von Positionen an der Gesichtsoberfläche zu positionieren; wobei die Vielzahl von Positionen für eine Erfassung der elektrischen Signale an der Gesichtsoberfläche für zumindest eine Linearkombination der elektrischen Signale zum Abbilden von Signalen aus einer vorbestimmten Kopfregion des Nutzers eingerichtet ist. Mittels der Linearkombinationen kann z. B. für eine Kopfregion des Nutzers ein EEG, ein EOG und ein EMG abgeleitet werden. Signale eines EKG bilden sich ebenfalls in den Elektrodenkanälen ab und lassen sich über Signalverarbeitungsverfahren, z.B. die Unabhängigkeitsanalyse (d. h. eine Independent Component Analysis (ICA)), extrahieren. Die Linearkombinationen der Elektrodenkanäle können z. B. genutzt werden, um spezifischen Signalanteile hervorzuheben. Die Elektrodenkanäle werden mit Rᵢ für i=1 bis 11 bezeichnet und sind auf die Elektrode REF referenziert

Fig. 6a zeigt exemplarisch spezifische Hirnregionen für die durch Bildung spezifischer Linearkombinationen Signale abgebildet werden können. Das Elektroenzephalogramm (EEG) ist z. B. für einen frontalen Bereich, für einen zentralen Bereich, für einen parietalen Bereich und für einen occipitalen Bereich eingerichtet. Für den Frontalen Bereich werden z. B. die Signale der Elektrodenkanäle 1 und 4 direkt ausgelesen und die Signale der Elektrodenkanäle 2 und 4 miteinander über eine Linearkombination verknüpft (R2-R4). Für den Zentralen Bereich werden z. B. Linearkombinationen aus den Signalen der Elektrodenkanäle 1, 4, 5 und 7 (z. B. eine erste Linearkombination mit (R2+R5)/2-R4 und eine zweite Linearkombination mit R5-R7) gebildet. Für den Parietalen Bereich kann z. B. eine Linearkombinationen aus den Signalen der Elektrodenkanäle 4 und 5 gebildet werden (R5-R4) und für den Occipitalen Bereich werden z. B. Linearkombinationen aus den Signalen der Elektrodenkanäle 4, 6 und 7 (z. B. eine erste Linearkombination mit R6-R4 und eine zweite Linearkombination mit R7-R7) gebildet. So lassen sich relevante Schlafparameter im EEG abbilden, trotz eingeschränkter Elektrodenplatzierung.

Fig. 6b zeigt, dass darüber hinaus sich ein Elektrookulogramm EOG (vertikal (R2-R3), horizontal (R2-R1) und diagonal (R1-R3)) ableiten lässt und sich ein Elektromyogram EMG insbesondere im Bereich des Kinns ableiten lässt (R10-R11). Die Elektroden der Elektrodenanordnung, die um das Auge des Nutzers angeordnet sind, sind z. B. innerhalb eines Toleranzbereichs von ±5° in einem rechtwinkligen Dreieck angeordnet. Die Augenbewegung wird somit über die Elektroden 1 und 3 diagonal erfasst, über die Elektroden 1 und 2 horizontal und über die Elektroden 3 und 2 vertikal. Die Elektroden 10 und 11 dienen zur Erfassung eines EMG.

Weitere Anwendungen für diese Elektrodenpositionen sind denkbar, der Arm zu den Kinnelektroden kann im Design aber auch weggelassen werden. Bei einer denkbaren Reduktion auf ausschließlich den Arm D (Bezeichnung aus Abbildung 5a und Abbildung 5b) hinter dem Ohr und C an der Stirn kann die Vorplatzierung und mögliche Drehung über die Elektrode 2 (Abbildung 6a und 6b) erfolgen.

In den Signalen sind z. B. auch Informationen eines Elektrokardiogramms enthalten. Sie lassen sich über Signalverarbeitungsverfahren, z.B. die Independent Component Analysis (ICA), extrahieren.

Gemäß einem Ausführungsbeispiel kann der Nutzer eine erste erfindungsgemäße Trägerstruktur mit Elektrodenanordnung auf einer Gesichtshälfte tragen und eine zweite erfindungsgemäße Trägerstruktur mit Elektrodenanordnung auf einer gegenüberliegenden Gesichtshälfte tragen. Dadurch können die Signale der beiden Elektrodenanordnungen kombiniert werde, was weitere Linearkombinationen erlaubt, die mittig durch den Kopf verlaufen.

Bei den hierin beschriebenen Vorrichtungen 100 weist die Vielzahl von Elektroden Nasselektroden auf Durch eine ungeübte Person selbstanlegbare Nasselektroden erweitern das Einsatzgebiet der Nasselektroden für die Erhebung elektrophysiologischer Signale, insbesondere EEG, aber auch EOG und EMG, erheblich in einem breiten Anwendungsgebiet. Personen können damit in der Häuslichkeit regelmäßig ohne Anwesenheit einer geschulten Person hochwertige Datenerhebungen vornehmen. Durch Tragekomfort und diskrete Platzierung ist eine Langzeitmessung zur Biosignalüberwachung auch im Alltag denkbar. Mit der Vorrichtung 100 wird eine Konfiguration von Nasselektroden derart in eine Grid-Struktur gebracht, d. h. auf der Trägerstruktur angeordnet, dass diese von einer ungeübten Person selbst angelegt werden können und mit geringer Positionsvarianz verlässlich bestimmte Elektrodenpositionen im Gesicht und hinter dem Ohr einnehmen.

Dem Designentwurf des Elektrodengrids, d. h. der Trägerstruktur 120 mit der Elektrodenanordnung 110, lagen unter anderem z. B. folgende Anforderungen zugrunde:
Es soll gut selbst anlegbar sein, d.h. ungeschulte Personen können sich das Grid auf Basis eines Manuals oder ähnlichem problemlos direkt selbst applizieren. Es soll für verschiedene Kopfformen passen, d.h. die Konfiguration ist für verschiedene Kopfgrößen und Kopfformen Jugendlicher und Erwachsener geeignet. Die Elektroden kommen in einer bestimmten Positionskonfiguration auf dem Gesicht und hinter dem Ohr an spezifischen Positionen zu liegen. Der Tragekomfort soll ein angenehmes Tragen über mehrere Stunden bis Tage hinweg möglich machen. Die Signalqualität soll mit Impedanzwerten im Bereich von wenigen KOhm bis etwa 20 kOhm für EEG, EMG, EKG und EOG-Messungen gut geeignet sein. Es sollen mehr als 3 Elektrodenpositionen in der Gridformation abgebildet sein, in Fig. 4a werden z. B. 8 (zuzüglich Referenz REF und Ground GRD für die Differenzmessung der Potentialunterschiede) abgebildet, mehr sind aber an den Positionen der Armausführungen oder kleineren Seitenarmen möglich. Das Grid gibt es z. B. in Varianten, in ähnlicher Formausführung, aber aus verschiedenen Materialien bzw. Ausführungen der Elektroden.

Eine erste Variante der Vorrichtung 100, wie sie z. B. in Fig. 7 dargestellt ist, weist ein Festgel-Grid mit Festgelelektroden auf. Hier können z. B. handelsübliche EKG-Elektroden 110₁ bis 110₉ für den Neonatalbereich in eine Trägerstruktur eingefügt werden. Alternativ zu den Festgelelektroden können auch Trockenelektroden verwendet werden. Eine Trägerstruktur 120, die z. B. als Trägermaterial Polyurethanschaum aufweist, bildet mit den Festgelelektroden ein Festgel-Grid. Die Trägerstruktur weist z. B. ein dehnbares Trägermaterial, wie z. B. der Polyurethanschaum, auf. Die Elektroden der Vielzahl von Elektroden sind z. B. an Kabel angeschlossen und die Kabelführung verläuft auf der Oberseite der Pflasterfläche. In anderen Worten, werden die Kabel an einer der Elektrodenanordnung 110 abgewandten Oberfläche der Trägerstruktur 120 mit Spiel 140 entlanggeführt. Das Spiel 140 bei der Kabelführung begrenzt eine Kraftübertragung einer Dehnung der Trägerstruktur 120 auf die Kabel. Das Spiel entspricht z. B. einem Längenzuschuss bei der Kabellänge, z. B. in Form von einem geschwungenen Verlauf der Kabel. Die Trägerstruktur ist z. B. ausgebildet, um einen Abstand zwischen zwei Elektroden der Elektrodenanordnung um maximal 20 % zu verlängern. Die in Fig. 7 dargestellte Ausführungsform weist eine Trägerstruktur 120 und eine Elektrodenanordnung 110, wie sie ebenfalls auch in Fig. 4b dargestellt ist, auf. Die Nasen an bestimmten Elektrodenpositionen dienen dem besseren Klebehalt, sie könnten aber auch entfernt werden. Alternativ kann aber auch die in Fig. 4a dargestellten Ausführungsform als Festgel-Grid (Trägerstruktur mit handelsüblichen Festgelelektroden verbunden mit Kabeln) ausgebildet sein. Optional weist die Vorrichtung 100 einen Signalausgang 160 auf, der ausgebildet ist, um ein auf den von der Vielzahl von Elektroden 110₁ bis 110₉ gemessenen elektrischen Signalen basierendes Ausgangssignal bereitzustellen. Der Signalausgang 160 ist über Kabel mit den Elektroden der Elektrodenanordnung verbunden. Die Kabel gehen z. B. über die Trägerstruktur 120 hinaus und münden in einen Stecker, der als Signalausgang 160 fungiert.

Eine zweite Variante der Vorrichtung 100 weist ein Leiterbahnen-Grid auf. In dieser Ausführung sind z. B. Leiterbahnen, die ein Metallmaterial (z.B. Silber bzw. Ag/AgCl) oder ein Graphenmaterial aufweisen, auf ein Trägermaterial der Trägerstruktur 120 aufgebracht, z.B. über ein Ätzverfahren, ein Laserverfahren oder auch über ein Druckverfahren. Es ist auch möglich eine Metallkombination zu verwenden, die Leiterbahnen zunächst in einem Kupferätzverfahren zu erstellen und nachfolgend über chemische Bindungen die Elektrodenflächen zu versilbern. Vorteilhaft sind gedruckte (bzw. geätzte) Elektrodensysteme auf Folienträger, da diese einfach auf die Haut geklebt werden können. Zudem sind Klebelösungen, d.h. gedruckte Leiterbahnen auf einem Trägermaterial, das auf die Haut geklebt werden kann, robuster gegen Artefakte durch Bewegung der Elektroden auf der Haut. Das Trägermaterial ist z. B. ein semi-flexibles, d. h. dehnbares oder elastisches, dünnes folienartiges Material (z. B. ein Polymermaterial, wie Polyestermaterial oder Polyamidematerial, oder ein Polyurethanmaterial oder ein Silikonmaterial). Vorgezogen wird Polyurethanmaterial, da dies neben der Hautverträglichkeit als biokompatibler Stoff eine Dehnbarkeit und Elastizität erlaubt und eine Flexibilität in mehr als einer Dimension. Damit schmiegt sich das Grid, d. h. die Trägerstruktur 120 an Gesichts- und Kopfunebenheiten an. Zur Isolation werden die Leiterbahnen z. B. als Sandwich-Konstrukt mit einer zweiten Schicht des Trägermaterials der Trägerstruktur 120 belegt, wobei die Elektrodenflächen (in den Figuren 4a und 4b als Kreise dargestellt) freigestellt bleiben. Die Leiterbahnen weisen z. B. einen geschlungenen Verlauf an oder in der Trägerstruktur 120 auf (z.B. wellenförmig, mäanderförmig oder gezackt), um sich bei Dehnung der Trägerstruktur 120 korrespondierend zu längen. Die Trägerstruktur ist somit ausgebildet, um einen Abstand zwischen zwei benachbarten Elektroden der Elektrodenanordnung um maximal 10 % zu verlängern.

Eine Prinzipskizze des Aufbaus ist in Fig. 8a dargestellt, dabei ist A Polyurethan, C Kupfer und B die Versilberung. Die Trägerstruktur 120 der Vorrichtung 100 weist somit z. B. eine erste Schicht aus Trägermaterial, eine zweite Schicht aus Trägermaterial und eine dazwischen angeordnete Schicht, die ein leitendes Material, wie z. B. Kupfermaterial, aufweist, auf. An den Positionen auf der Trägerstruktur 120, an denen die Elektroden der Elektrodenanordnung angeordnet sind weist die zweite Schicht aus Trägermaterial eine Lücke auf. An diesen Stellen wird das leitende Material der dazwischen angeordneten Schicht z. B. versilbert oder mit dem Graphenmaterial bedeckt. Anschließend wird auf die versilberte Stelle auf der Trägerstruktur 120 z. B. ein Gel, wie z. B. ein klebendes Elektrolytgel oder ein Festgel, aufgebracht bzw. vorappliziert. Fig. 8b zeigt den Aufbau nach Vorapplizierung des Gels (D), allerdings ohne eine Abdeckung durch eine optionale Schutzfolie. Wichtig ist, dass das Gel nicht in Kontakt mit der Kupferleiterbahn kommt, da es sonst zu chemischen Reaktionen kommt. Das Gelmaterial, das auf der versilberten Elektrodenoberfläche angeordnet ist, kann z. B. als Gellinse bezeichnet werden. Die freien Elektrodenflächen (in den Figuren 4a und 4b als Kreise dargestellt) sind mit einer Elektrolyt-Gellinse belegt. Diese wird z. B. durch Abziehen einer Schutzfolie direkt vor dem Anlegen des Grids von der Person selbst freigelegt. Die Schutzfolienbereiche sind z. B. unterteilt, so dass es möglich ist, die Arme des Grids nach und nach aufzukleben. Optional kann auf ein Applizieren des Gels auf die Elektrodenstelle verzichtet werden (die Vielzahl von Elektroden weist somit z. B. Trockenelektroden auf), wenn ein hautverträgliches Material wie z. B. Graphen zum Bedecken des leitenden Materials, das zwischen den beiden Schichten aus Trägermaterial angeordnet ist, verwendet wird.

Ggf. kann bei dieser zweiten Variante und optional auch bei der ersten Variante ein Versteifungselement 122, das z. B. Polyurethanschaum aufweist, als zusätzliche Stabilisierung angebracht werden. Alternativ kann eine sub-millimeter dicke Klebefläche aber auch ausreichend sein. Ebenfalls zur Stabilisierung für die Applizierung des Grids kann auf der oberen (nicht Haut) Seite, d.h. auf der der Elektrodenanordnung 110 abgewandten Seite der Trägerstruktur 120, ein Papier- oder Folienmaterial angebracht sein, um die Steifigkeit zu erhöhen. Dieses Material wird nach Platzierung des Grids ähnlich wie bei einem Duschpflaster abgezogen, so dass ein insgesamt höherer Tragekomfort entsteht.

Gemäß einem Ausführungsbeispiel weist die Trägerstruktur 120 durchsichtige Trägermaterialien, d.h. transparente und/oder farblose Trägermaterialien, oder unterschiedlich eingefärbte Trägermaterialien auf, um eine geringe (oder ggfs. besonders hohe) Sichtbarkeit der Trägerstruktur 120 bei verschiedenen Hauttypen zu erzielen. Das schmale Griddesign ist geeignet, ggf. mit einem optisch ansprechenden Aufdruck, im Alltag getragen zu werden oder aber durch transparente Ausgestaltung unauffällig zu bleiben. Die Vorrichtung kann somit diskret ausfallen und einen angenehmen Tragekomfort bieten, so dass sogar eine Anwendung im Alltag ermöglicht wird. Hierdurch werden Langzeitaufnahmen ermöglicht, da eine erhöhte Akzeptanz für eine Nutzung der Vorrichtung im Alltag erreicht wird. Besonders vorteilhaft sind durchsichtige Trägermaterialien, da diese eine schnelle Erkennung von Hautirritationen, z. B. Reaktionen auf ein Klebemittel zum Anbringen der Trägerstruktur auf der Haut, ermöglichen. Zudem erleichtern durchsichtige Trägermaterialien das selbstständige Anlegen der Trägerstruktur, da markante Gesichtspunkte nicht verdeckt werden und ein Nutzer so effizient die Elektroden der Elektrodenanordnung korrekt an sich selbst positionieren kann. Zudem kann nach Anbringen der Elektrodenanordnung einfach festgestellt werden, ob die Elektroden an den korrekten Positionen des Nutzers positioniert wurden.

Gemäß einem Ausführungsbeispiel sind die hierin beschriebenen Elektrodenanordnungen 110 der Vorrichtung 100 mit passiven oder aktiven Elektroden denkbar. Passive Elektroden leiten die elektrische Aktivität über eine Metallfläche ab, in dem Beispiel der "Variante Festgel-Grid" z. B. über ein Metallplättchen aus Silber mit einem Silberchloridüberzug (Ag/AgCl-Elektroden), und bei dem Beispiel der "Variante Leiterbahnen-Grid" ist es eine Silberfläche, die in einem chemischen Verfahren auf eine Kupferleiterbahn aufgebracht wird. Als anderen Typ von Elektroden existieren aktive Elektroden, die über spezifische Schaltkreise eine Verbesserung der Signalqualität schon vor Eingang in den Differenzverstärker erreichen. Dieser zusätzliche Schaltkreis kann dabei verschiedene Aufgaben übernehmen. Eine Möglichkeit ist die Impedanzwandlung. Ähnlich wie z. B. bei einem Mikrofonkabel, lässt sich damit z. B. das Signal gegenüber Störungen durch Bewegungen der Kabel unempfindlicher machen. Auch lassen sich mit Hilfe dieser zusätzlichen Schaltung bereits kleine Verstärker direkt an der Elektrode einsetzen. Dadurch kann der SNR bereits direkt an der Elektrode verbessert werden, was zu einer besseren Signalqualität führt. Außerdem kann ein Signalverstärker, den die Vorrichtung optional aufweist, weiter entfernt am Körper sitzen, da aufgrund des besseren Signal-Rausch-Verhältnisses Bewegungen der Kabel weniger kritisch für die Signalqualität sind.

Bei sehr großen oder sehr kleinen Kopfformen (Grundschulkindern, Teenager, Jugendliche) lässt sich die Konfiguration skalieren, beispielsweise auf 70% der Normalgröße für Grundschulkindern. Orientiert an den internationalen Hutgrößen bzgl. des Kopfumfanges können die Größen S, M, L von der vorgeschlagenen Trägerstruktur abgedeckt werden, d.h. bei S Kopfumfänge von 55-56 cm, bei M von 57-58 cm und L von 59-60 cm. Die Trägerstruktur ist durch das elastische Trägermaterial für unterschiedliche Kopfumfänge angepasst, wobei sich die unterschiedlichen Kopfumfänge um maximal 5 cm voneinander unterscheiden. Darüber hinaus sollte die Konfiguration mit einem Faktor von 1,1 oder 1,2 vergrößert werden, so dass auch Kopfgrößen XL von 61-62 abgedeckt werden können. Eine weitere entsprechende Vergrößerung darüberhinaus ist denkbar. Ebenso kann die Konfiguration durch eine Skalierung um Faktor 0.9 oder 0.8 für die Größen XS von 53-54 cm angepasst werden. Aufgrund der Weichheit des Trägermaterials der Trägerstruktur 120 ist es unproblematisch, wenn die Trägerstruktur 120 bei einem etwas zu kleinen Kopf eine kleine Welle zwischen den Elektrodenpositionen schlägt. Ähnlich können Zwischenpassagen auch etwas gedehnt werden, um die Positionen auf Stirn und hinter dem Ohr gut zu erreichen. Eine Anpassung an verschiedene Kopfformen ist z. B. in der Art möglich, dass der Arm hinter dem Ohr etwas verdreht/verkippt wird. Das flexible Material ermöglich dies. Vorzugsweise sind die Leiterbahnen in Bereichen, die verdreht oder gedehnt werden, in geschlungener Form, z. B. in Mäanderform, gedruckt, so dass die Leitungsbahnen die Dehnung bzw. Spannung aufnehmen können. Die Trägerstruktur ist durch das elastische Trägermaterial für die Positionierung des zumindest einen Teils der Elektrodenanordnung an der Gesichtsoberfläche des Kopfes des Nutzers mit einem Kopfumfang in einem Bereich von 50 cm bis 55 cm oder von 55 cm bis 60 cm oder von 60 cm bis 65 cm angepasst.

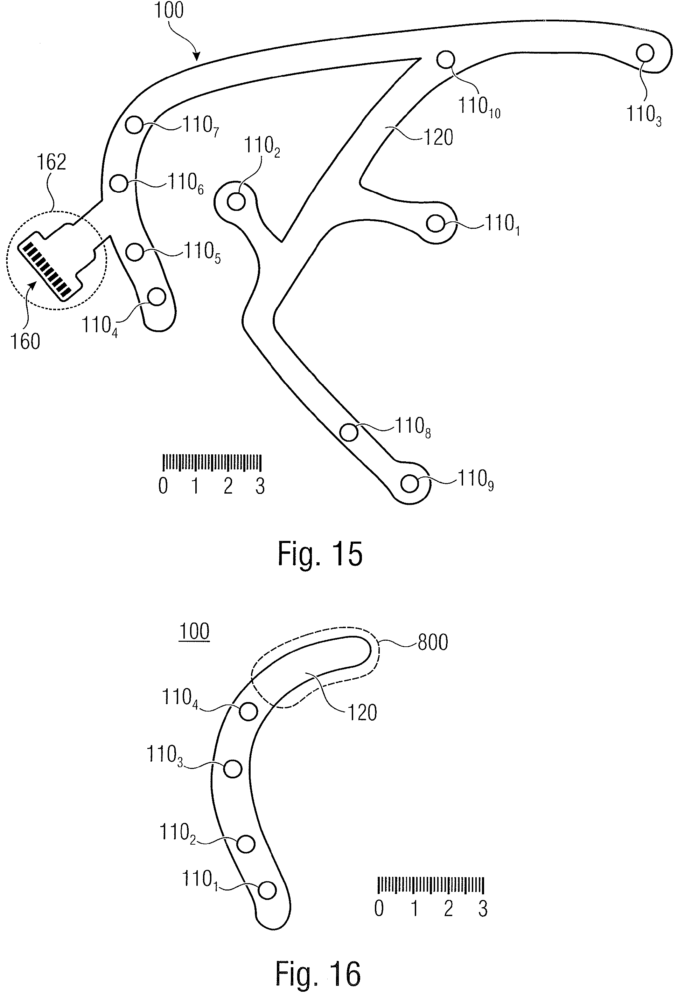

Gemäß einem Ausführungsbeispiel weist die Vorrichtung 100 einen Signalverstärker 150 auf, der ausgebildet ist, um von einem Signalausgang (z. B. 160 in Fig. 7 oder Fig. 15) der Vorrichtung 100 die gemessenen elektrischen Signale zu erhalten. Der Signalausgang 160 kann optional auf der Trägerstruktur 120 angeordnet sein, siehe z. B. Fig. 15. Fig. 15 stellt z. B. ein Leiterbahnen-Grid dar, bei dem die Elektroden 110₁ bis 110₁₀ über Leiterbahnen mit dem Signalausgang 160 verbunden sind. Die Leiterbahnen sind z. B. in die Trägerstruktur 120 integriert. Die Vorrichtung 100 weist ferner z. B. ein Befestigungsmittel auf, das eine Position des Signalverstärkers 150 an einem Hinterkopf, einem Arm, einer Schulter, einer Brust oder einem Hals des Nutzers definiert. Die Vorrichtung 100 weist zur Signalerfassung z. B. einen EEG-Differenzverstärker auf. Zur Vermeidung von Kabelartefakten sollte der Verstärker in der Nähe der Elektroden platziert werden. Es ist auch möglich zunächst in einen Adapter zu münden, um dann ein gebündeltes, abgeschirmtes Kabel zu dem Verstärker laufen zu lassen. Dann kann der Verstärker auch beispielsweise mit einem Brustgurt oder einer Armbinde getragen werden. Für mobile EEG-Anwendungen wird er im Bereich der Forschung häufig am Hinterkopf mit Hilfe eines Kopfbandes getragen, diese Lösung ist hier auch grundsätzlich denkbar. Der EEG-Verstärker zeichnet die Signale entweder lokal auf oder sendet sie kabelgebunden oder kabellos, z.B. über eine Bluetooth-Verbindung, zu einer Empfangsstation.

Für den Anschluss der Vorrichtung 100 aus Fig. 15 an einen Adapter oder direkt an den Signalverstärker 150, bedarf es in der Regel einer formstabilen/starren Verstärkung, da sonst die Verbindung durch ein Einschieben nicht hergestellt werden kann. Dafür kann ein starres Material als Stütze auf das Einsteckstück 162 der Trägerstruktur 120 geklebt werden. Die Trägerstruktur 120 weist z. B. eine Stützstruktur auf, die z. B. an einer Position 162 des Signalausgangs 160 auf der Trägerstruktur 120 angeordnet ist. Die Stützstruktur weist z. B. Polyimidmaterial, Polyestermaterial, Polyethylenmaterial und/oder FR4-Material auf.

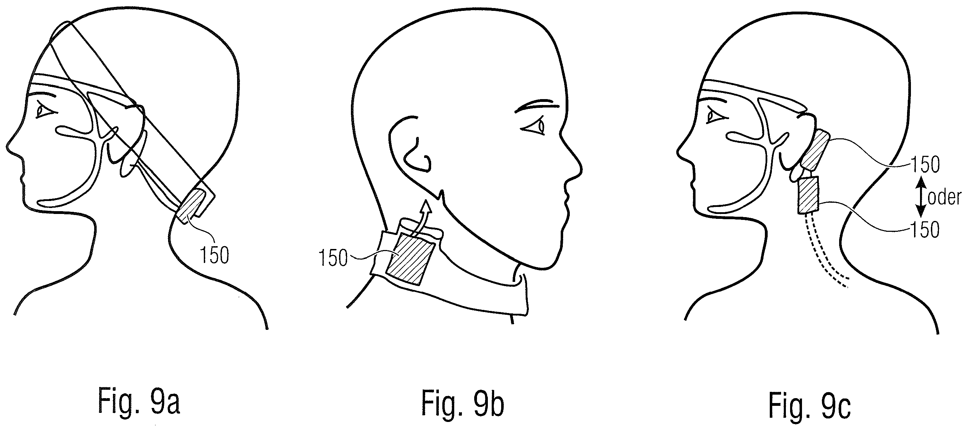

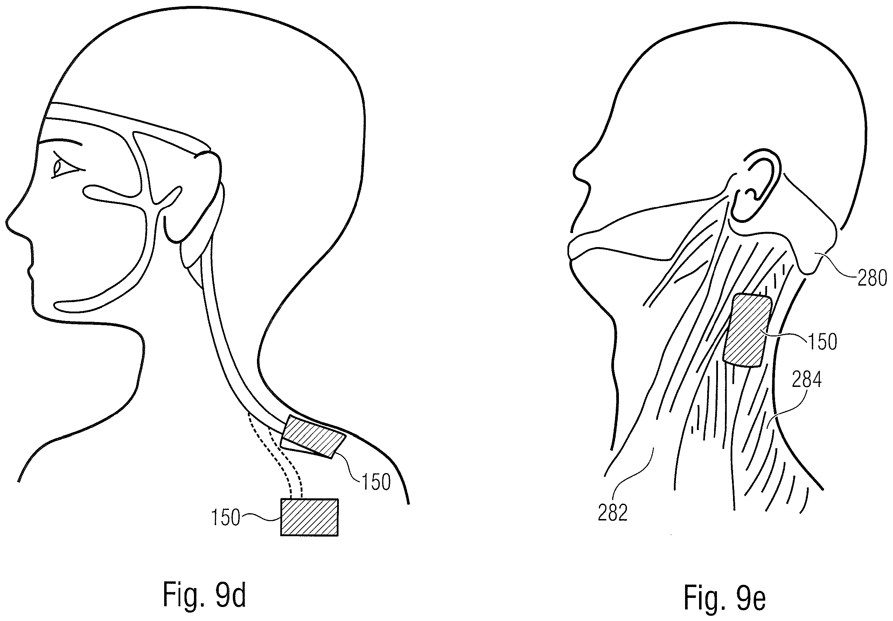

Auf der Schulter oder auf Brusthöhe könnte der Signalverstärker 150 durch Befestigung an Kleidung oder einem Haltegurt getragen werden. Die Figuren 9a bis 9e zeigen verschiedene Formen, wie der Verstärker an einem Körper des Nutzers vorteilhaft getragen werden kann. Fig. 9a zeigt eine mögliche Positionierung des Anschlusses an den Verstärker oder Adapter an dem Hinterkopf des Nutzers mit einem Stirnband. Eine andere Möglichkeit ist die Platzierung am Hals des Nutzers. Der Adapter oder direkt der Verstärker könnte z. B. direkt auf die Haut geklebt werden mit Hilfe eines hautverträglichen Klebepatches. Eine andere Möglichkeit ist die Nutzung eines Schals bzw. Loop, der um den Hals getragen wird, wie in Fig. 9b dargestellt. Der Adapter oder Verstärker kann dabei in einer Tasche an diesem Schal oder Loop aufgenommen werden. Der Schal oder Loop kann zudem zur Abpolsterung dienen. Als Alternative zum Schal kann auch ein Halteelement um den Halsansatz auf dem Oberkörper zu liegen kommen. In Fig. 9c sind zwei Alternativen dargestellt, einmal kann der Signalverstärker 150 direkt hinter dem Ohr und alternativ unterhalb des Ohrs angeordnet bzw. befestigt werden. Gemäß Fig. 9d kann der Signalverstärker 150 auf der Schulter des Nutzers abgelegt oder an Bekleidung oder einem Haltegurt befestigt werden oder z. B. an der Brust des Nutzers bzw. seiner Kleidung oder einem Haltegurt befestigt werden. Insbesondere für die Anwendung im Schlaf wird aber die in Fig. 9e dargestellte Position für den Verstärker oder Adapter vorgezogen. Das Befestigungsmittel ist z. B. ein Halsband oder Schal, das/der ausgebildet ist, um den Signalverstärker 150 an dem Hals des Nutzers unterhalb des knöchernden Vorsprungs processus mastoideus 280 und zwischen den beiden Muskeln sternocleidomastoideus 282 und trapezius 284 anzubringen.

Im Folgenden wird auf die Verwendung der erfindungsgemäßen Vorrichtung 100 näher eingegangen, insbesondere wird darauf eingegangen, wie der Nutzer sich die Vorrichtung 100 selbst anlegen kann. Die Vorrichtung 100 entspricht dabei z. B. einem der zuvor beschriebenen Ausführungsbeispielen.

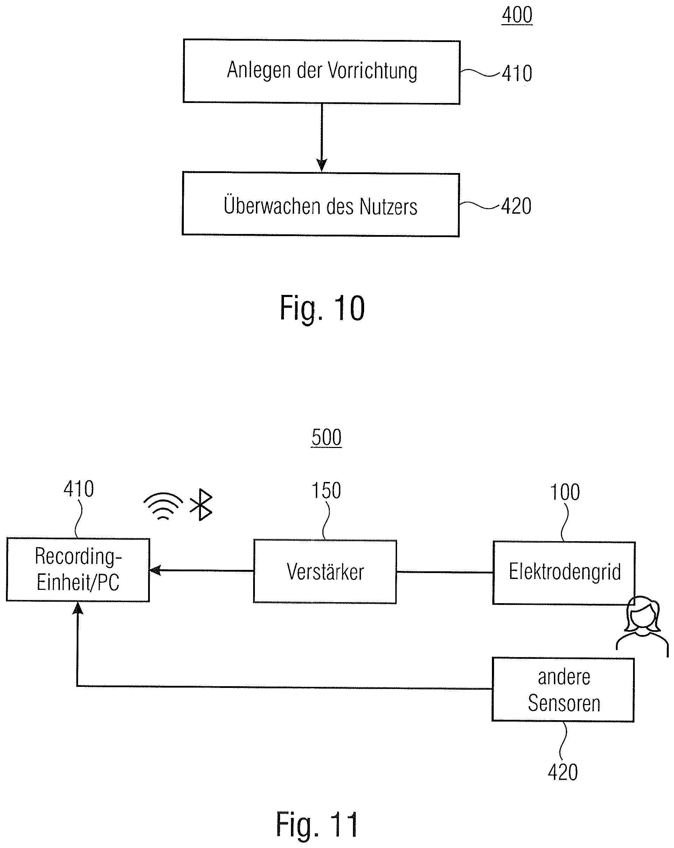

Wie in Fig. 10 dargestellt, weist das Verfahren 400 zur Verwendung der erfindungsgemäßen Vorrichtung zumindest die Schritte Anlegen 410 der Vorrichtung 100 an einer Gesichtsoberfläche 220 eines Kopfes 200 eines Nutzers 210 und hinter einem Ohr 230 des Nutzers durch den Nutzer 210 selbst oder einer anderen Person und ein Überwachen 420 des Nutzers 210 anhand der von der Vielzahl von Elektroden gemessenen elektrischen Signale auf. Für das Anlegen 410 wird ein selbstständiges Anlegen 410 durch den Nutzer 210 bevorzugt.

Optional kann das Verfahren 400 weitere der im Folgenden beschriebenen Schritte aufweisen:
Das Verfahren kann ein Reinigen der Kopfoberfläche, d. h. der Haut, aufweisen, wobei sich das Reinigen optional auf die Bereiche, auf denen die Vorrichtung angelegt 410 wird, beschränkt. Eine Reinigung der Haut mit einer Alkohollösung zur Entfernung von Schmutz, Fett und Talg ist für eine bessere Signalqualität von Vorteil. Alternativ oder zusätzlich kann die Reinigung, z. B. die Vorbehandlung der Kopfoberfläche, mit einem abrasiven Gel, wie bei Ringelektroden oft üblich, erfolgen. Ein technisches Problem ist die geringe Signalstärke der Potentialdifferenz im Bereich von 0.1 bis 100 microVolt. Aus diesem Grund muss der Kontakt zwischen Elektrode und Haut mit bestmöglicher Leitfähigkeit hergestellt werden. Der Impedanzwert der Haut liegt im Bereich von wenigen kOhm bis MOhm. Um diesen Wert gering zu halten, werden vor der Messung die Stellen, an welchen die Elektroden positioniert werden, z. B. mit Alkohol entfettet und ggfs. mit einem Peeling-ähnlichen Gel behandelt, um Fett, Talg oder abgestorbene Hautzellen zu entfernen.

Bei einem Ausführungsbeispiel der Vorrichtung 100 weist die Trägerstruktur 120 Klebematerial auf, dass z. B. von einer Schutzfolie bedeckt ist. Die untere Seite der Trägerstruktur, d.h. die Seite der Trägerstruktur, auf der die Elektrodenanordnung angeordnet ist, ist z. B. mit der Schutzfolie belegt, darunter befindet sich eine Klebefläche aus dem Klebematerial auf den Gridarmen, d.h. auf den Verbindungsstegen der Trägerstruktur. An den Elektrodenpositionen ist z. B. eine Gellinse für die signaltechnisch vorteilhafte Verbindung zwischen Haut und Elektrode angeordnet. Bevor die Schutzfolie zur Befestigung der Vorrichtung auf der Haut abgezogen wird, erfolgt z. B. eine Vorplatzierung. Das Anlegen 410 der Vorrichtung kann somit z. B. die Schritte
vorplatzieren der Vorrichtung auf der Kopfoberfläche, d.h. auf der Gesichtsoberfläche 220 und hinter dem Ohr 230,
Abziehen der Schutzfolie von der Vorrichtung, und
Ankleben der Vorrichtung auf der Kopfoberfläche.

Das Abziehen und Ankleben kann dabei z. B. mehrfach wiederholt werden, falls die Vorrichtung eine Mehrzahl von Schutzfolienabschnitten aufweist. Die Reihenfolge welcher Schutzfolienabschnitt wann abgezogen wird, kann z. B. wie weiter oben in Zusammenhang mit der Vorrichtung bereits beschrieben erfolgen.

Über die beiden Elektroden A und B (Bezeichnungen aus Abbildung 5a und Abbildung 5b) erfolgt z. B. die Vorplatzierung, indem der obere Wangenknochen ertastet wird, dort die Elektrode B vorplatziert wird und auf etwa horizontaler Linie die Elektrode A vorplatziert wird. Dann kann geprüft werden, ob der obere Gridarm oberhalb des Auges etwa zur Stirnmitte (C) platzierbar ist. Ebenso kann geprüft werden, ob der linke Arm des Grids (D) gut hinter das Ohr passt. Zur einfachen Handhabung wurde im Design daher der Verlauf des Arms über dem Ohr gewählt, da hier eine natürliche Abstützung auf dem oberen Ansatz des Ohrs am Kopf bei der Platzierung möglich wird. Durch geringfügige Drehung um Punkt F, lassen sich die Positionen von linkem und oberem Arm (d. h. die Positionen der beiden Teile 124₁ und 124₂ in die sich der zweite Verbindungssteg 124 aufteilt) optimieren. Mit einer freien Hand und einem hautfreundlichen Stift können die Positionen von (A) und (B) auf dem Gesicht auch zusätzlich sichtbar markiert werden, so dass bei Abrutschen der Hand leicht korrigiert werden kann.

Nach der Vorplatzierung kann beginnend an dem Arm mit der Elektrode A und B die Schutzfolien abgezogen werden, um dann in der bei der Vorplatzierung benannten Reihenfolge (C, D) die Arme des Grids, d.h. die Verbindungsstege der Trägerstruktur, aufgeklebt werden. Die Schutzfolie ist in entsprechende Abschnitte unterteilt, so dass die Arme des Grids einzeln nach und nach befestigt werden können.

Das Anlegen 410 der Vorrichtung 100 an der Gesichtsoberfläche 220 des Kopfes 200 des Nutzers 210 kann somit z. B. die Schritte Ertasten eines oberen Wangenknochens und Anbringen einer ersten Elektrode an dem oberen Wangenknochen; Anbringen einer zweiten Elektrode vor dem Tragus eines Ohres des Nutzers; Anbringen einer dritten Elektrode an einer Stirn senkrecht oberhalb eines Nasion in einem Bereich von 1-3 cm über einer Augenbrauenlinie; und anbringen einer vierten Elektrode hinter dem Ohr, aufweisen.

Zur Beschreibung der Platzierung:
1. Wangenknochen und haarfreien Bereich (wichtig für Einsatz bei Bartträgern) vor dem Ohr (ca 1 cm vor dem Tragus "Knorpelgnupsi") ertasten. Die beiden kurzen Äste des Grids auf diese Punkte platzieren, in etwa horizontaler Ausrichtung.
2. Grid grob an Stirn und hinterm dem Ohr ausrichten, ggf. über Punkt F rotieren
3. Grid stückweise beginnend an Wangenknochen und vor dem Tragus aufkleben.
4. An der Stirn trifft man den Punkt vom Nasion senkrecht nach oben in Richtung Stirn auf etwa 1-2 cm über der Augenbrauenlinie. Eine Abweichung horizontal im Bereich von 1-2 cm ist akzeptabel.
5. Hinter dem Ohr erreicht die letzte Elektrode in der Regel den Mastoid als ertastbaren Knochenfortsatz. Diese Elektrode wird typischerweise als Referenz für die Messung der Potentialunterschiede gewählt.

In einer optionalen Ausführung des Grids existiert ein dritter Arm (E), der entlang des Kinns geklebt wird, um über die Elektroden für ein EMG im Kinnbereich aufzunehmen. Eine solche Aufnahme ist für die Erfassung von Schlafphasen von Vorteil zur Unterscheidung der Rapid Eye Movement (REM) Phase gegenüber den anderen, insbesondere der Wachphase. Das vollständige Grid-Design mit den Elektroden am Kinn zur Erfassung eines EMG zielt auf die Anwendung für das Schlafmonitoring in der Häuslichkeit.

Die gute Handhabung des Grids und die beschriebene Möglichkeit der Applizierung ermöglicht die sichere Platzierung beim ersten Versuch. Bei einer Re-Platzierung würde man Klebekraft verlieren, die Gefahr wächst, dass die Signalstabilität nicht über längere Zeiträume erhalten bleibt.

Der Bügelbereich über dem Ohr ist z. B. als Klebefläche ausgespart, damit das Grid nicht mit den Haaren verklebt. Neben der Hilfestellung bei der Gridplatzierung hat dieser Bügel darüber hinaus die Funktion als Verbindung zwischen dem Gesichtspart und dem Arm hinter dem Ohr. Eine andere Ausführungsvariante unterhalb des Ohrs ist möglich, dies verläuft über den Kieferknochen, was bei höherer Kieferaktivität die Stabilität der Elektrodenplatzierung gefährdet und darüber hinaus einen etwas geringeren Tragekomfort bietet.

Für die Erhebung elektrophysiologischer Signale, insbesondere Elektroenzephalogramm (EEG), Elektrookulogramm (EOG), Elektrokardiogramm (EKG) und Elektromyogramm (EMG) werden die Elektroden über ein Gel (Nasselektroden) in Verbindung mit der Haut des Menschen gebracht, um von Gehirn und Muskelzellen generierte elektrische Aktivität zu messen. Es werden Potentialschwankungen einer Elektrode in Differenz zu einer Referenzelektrode gemessen, die Verstärkung des Signals erfolgt z. B. über einen Differenzverstärker. Bei der Nutzung von Nasselektroden wird ein Elektrolytgel zur Reduktion der Übergangsimpedanz zwischen Haut und Elektrode eingesetzt.

Mittels des Schritts Überwachen 420 des Nutzers, kann z. B. basierend auf den gemessenen elektrischen Signalen ein Schlafverhalten des Nutzers oder ein kognitionspsychologisches Verhalten des Nutzers überwacht werden.

Technische Anwendungsgebiete, wie sie im Folgenden beschrieben werden, sind denkbar:
- Häusliches oder ambulantes Schlafmonitoring
- Häusliches oder ambulantes Monitoring bei Patienten mit Epilepsie oder Verdacht darauf (nicht unter die beanspruchten Verfahren fallend)
- Häusliches oder ambulantes Monitoring bei Patienten mit anderen neurologischen Erkrankungen oder Verdacht darauf (z.B. Depression, Alzheimer) u.a. zur Ableitung von Frühindikatoren (nicht unter die beanspruchten Verfahren fallend)
- den Einsatz in kognitionspsychologischen Testreihen, beispielsweise zur Aufmerksamkeit, Vigilanz, Höranstrengung, kognitiver Belastung, Bestimmung von sensorischer Funktionalität sowie kognitiver Verarbeitungsfähigkeit des Patienten in der Häuslichkeit, in Pflegeheimen, aber auch bei niedergelassenen Ärzten oder in Kliniken, da auch hier die einfache Installation der Elektroden Zeit spart
- Einsatz zur Erfassung eben genannter kognitiver Zustände in Arbeitssituationen, insbesondere für sicherheitskritische Anwendungen (z. B. Lokführer, Piloten, Fluglotsen, LKW-Fahrer, Minenfahrzeuge)
- in der neuropsychologischen Forschung und angrenzenden Forschungsbereichen zu kognitiven Zuständen, aber auch zu neurologischen Krankheitssymptomen im wachen, aber auch im schlafenden Zustand (nicht unter die beanspruchten Verfahren fallend)
- Erfassung der akustischen Aufmerksamkeit für nachfolgende Analysen oder zur Steuerung eines Hörgeräts, beispielsweise zur Verbesserung des Sprachsignals eines attendierten Sprechers
- Erfassung der Höranstrengung zur nachfolgenden Analyse oder ebenfalls zur Steuerung der Einstellungen eines Hörgeräts
- Monitoring von der Wirksamkeit von Positive-Air-Pressure Systemen zur Behandlung von Apnoe, wobei solche Verfahren nicht unter die beanspruchten Verfahrensansprüche fallen.

Positive-Air-Pressure (PAP) Systeme sind eine der Therapien bei Apnoe. Zunächst wird in der Regel in einem Schlaflabor eine Apnoe-Erkrankung diagnostiziert, dann erfolgt ebenfalls in einem Schlaflabor die Einstellung des PAP-Gerätes. In vielen Fällen in der Praxis soll diese Einstellung aber nicht ausreichend gut funktionieren, hier wird in der Regel lediglich über eine Polygraphie (ohne EEG) der Schlaf in Abständen kontrolliert. Die hier vorgeschlagene Lösung könnte eine Verbesserung erbringen, da die Erfassung weiterhin in der Häuslichkeit möglich ist, aber hochwertigere Informationen über die Schlafarchitektur mittels EEG erlaubt.

Fig. 11 zeigt ein Blockschema für den Aufbau der Datenerfassung mit der Vorrichtung 100. Personen können ein System 500 zur Datenerfassung nach Hause nehmen und dort selbst applizieren. Das System weist z. B. einen EEG-Verstärker 150, eine Recording-Einheit 410 und ggf. weitere Sensoren 420 und eben die hierin beschriebene erfindungsgemäße Vorrichtung 100 auf.

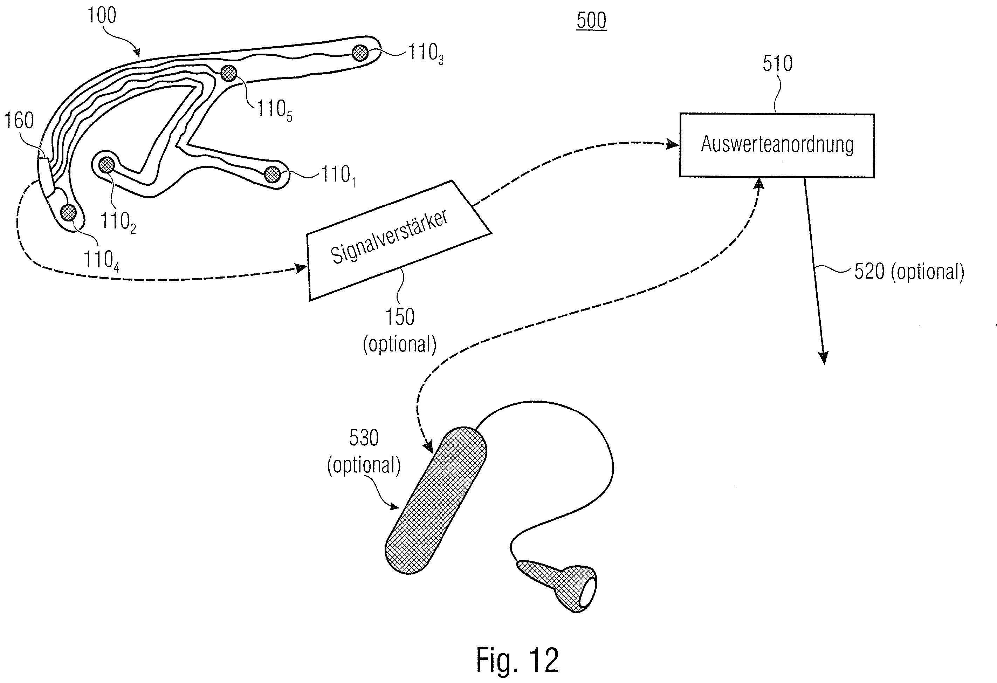

Fig. 12 betrifft ebenfalls ein Ausführungsbeispiel eines Systems 500, das die erfindungsgemäße Vorrichtung 100 aufweist. Zusätzlich weist das System 500 eine Auswerteanordnung 510 auf, die ausgebildet ist, um die von der Vielzahl von Elektroden gemessenen elektrischen Signale auszuwerten. Optional kann das System 500 Merkmale und Funktionalitäten, wie sie in Zusammenhang mit Fig. 11 beschrieben worden sind, aufweisen.

Die Auswerteanordnung 510 ist z. B. ausgebildet, um die von der Vielzahl von Elektroden gemessenen elektrischen Signale von einem Signalausgang 160 z. B. über einen Signalverstärker 150 zu erhalten. Dies kann z. B. kabellos über WLAN oder Bluetooth oder auch über eine Kabelverbindung erfolgen.

Die Auswerteanordnung 510 ist z. B. ausgebildet, um basierend auf den elektrischen Signalen eine Information 520 über eine Schlafarchitektur des Nutzers bereitzustellen; oder eine Information 520 über ein epileptisches Verhalten des Nutzers bereitzustellen; oder eine neurologische Erkrankung des Nutzers anzuzeigen 520; oder eine kognitionspsychologische Information 520 bereitzustellen.

Gemäß einem alternativen Ausführungsbeispiel, weist das System 500 ferner ein Hörgerät 530 auf. In diesem Fall ist die Auswerteanordnung z. B. ausgebildet, um basierend auf den elektrischen Signalen das Hörgerät 530 zu steuern.

Die Auswerteanordnung 510 kann z. B. ausgebildet sein, um:
- eine akustische Aufmerksamkeit für nachfolgende Analysen und/oder zur Steuerung des Hörgeräts 530, beispielsweise zur Verbesserung des Sprachsignals eines attendierten Sprechers, zu erfassen; und/oder
- eine Höranstrengung zur nachfolgenden Analyse und/oder ebenfalls zur Steuerung der Einstellungen des Hörgeräts 530 zu erfassen.

Der Mehrwert der hier vorgeschlagenen Lösung ist, dass auf Basis der selbstanlegbaren Vorrichtung 100 in vorgegebener Konfiguration der Elektroden 110₁ bis 110₅ (Die Konfiguration der Elektroden kann gemäß einem hierin beschriebenen Ausführungsbeispiel vorliegen, siehe z. B. die Figuren 1a, 4a, 4b und 6a/b) neben EKG, EMG für die vorgeschlagene Anwendung vorteilhaft EEG und EOG Informationen im Alltag erfasst werden können und so mit dem Hörgerät 530 und optional mit einem EEG/EOG-Signalverstärker verbunden werden können. Besonders vorteilhaft ist die Vorrichtung 100 in dem System 500, wenn die Elektrodenanordnung Nasselektroden aufweist. Es gibt Lösungen mit Trockenelektroden im Ohrkanal oder in der Ohrmuschel, diese können aber unangenehmen Druck erzeugen oder schlechtere Signalqualität nur bieten. Die hier vorgeschlagene Lösung vereint Tragekomfort und gute Signalqualität. Kann man EEG im Alltag in der Form erfassen, sind die benannten Anwendungen in Verbindung mit einem Hörgerät denkbar, also
- Erfassung der akustischen Aufmerksamkeit auf einen bestimmten Sprecher in einer Mehrsprechersituation (oder auf ein Geräusch in lauter Umgebung) auf Basis des EEG über eine Korrelation von EEG und der Einhüllenden des Zielaudiosignals (wiss. Arbeiten dazu werden seit einigen Jahren publiziert). Ist das Zielsignal derart erfasst, kann nachfolgend der Beamformer, d.h. der Strahlformer, des Hörgeräts so eingestellt werden, dass dieses Zielsignal besonders hervorgehoben wird und für den Hörenden damit besser hörbar ist.
- Im zuvor benannten Anwendungsfall ist es eine besondere Herausforderung, dem Hörenden zu erlauben aus der unterstützten Situation auch wieder "herauszukommen", das heißt, dem Hörenden ermöglichen auch wieder auf andere Sprecherquellen zu fokussieren, obwohl diese nun stärker im Hintergrund sind. Eine Lösung auf Basis des vorgeschlagenen Elektrodengrids ist es, über das EOG Informationen zu Augenbewegung (Richtung), Zwinkern und Augenschließen zu bekommen und diese Information sozusagen als Fernbedienung zu verwenden. So kann der Hörende in einer sehr viel robusteren Art und Weise und gleichzeitig einfach und intuitiv steuern, welches Signal besonders hervorgehoben werden soll (Blick in die Richtung, zweimal zwinkern zur Bestätigung beispielsweise). Die Auswerteanordnung 510 kann somit ausgebildet sein, um die von der Vorrichtung 100 gemessenen Signale auf vorbestimmte Signaleigenschaften auszuwerten, um das Hörgerät 530 in Antwort auf Signale des Nutzers zu steuern.
- Erfassung der Höranstrengung auf Basis von EEG-Daten - steigt die Höranstrengung können die Hörgerätealgorithmen automatisch angepasst werden, beispielsweise auf ein anderes vorprogrammiertes Profil der Hörunterstützungsalgorithmen. Hier kann aber auch noch die Analyse des Audiokontextes eine zusätzliche Informationsquelle bieten, um den Hörunterstützungsalgorithmus auszuwählen.

Ein weiteres Ausführungsbeispiel betrifft ein Verfahren 600 zur Herstellung der erfindungsgemäßen Vorrichtung 100, siehe Fig. 13. Das Verfahren 600 weist unter anderem die Schritte Bereitstellen 610 einer Trägerstruktur 120 und Anbringen 620 einer Elektrodenanordnung 110 an die Trägerstruktur 120 und optional das Aufbringen 630 von Elektrolytgel oder Gellinsen auf Elektroden der Elektrodenanordnung auf. Die Elektrodenanordnung weist z. B. eine Vielzahl von Elektroden auf. Bei dem Aufbringen 630 können z. B. nur auf einem Teil der Vielzahl von Elektroden jeweils eine Gellinse bzw. Elektrolytgel aufgebracht 630 werden oder es kann auf jeder Elektrode der Vielzahl von Elektroden jeweils eine Gellinse bzw. Elektrolytgel aufgebracht 630 werden.

Die Elektrodenanordnung weist eine Vielzahl von Elektroden zur Messung von elektrischen Signalen an einer Gesichtsoberfläche eines Kopfes und hinter einem Ohr eines Nutzers der Vorrichtung auf.

Die Trägerstruktur wird an eine Gesichtsoberfläche des Kopfes des Nutzers der Vorrichtung angepasst. Die Trägerstruktur ist ohne äußere Krafteinwirkung des Nutzers innerhalb eines Toleranzbereichs formstabil und unter äußerer Krafteinwirkung des Nutzers deformierbar, um einen Abstand zwischen zwei benachbart angeordneten Elektroden zu verändern und/oder sich Gesichts- und Kopfstrukturen flächendeckend anzupassen. Ferner definiert die Trägerstruktur eine Relativposition der Elektroden der Vielzahl von Elektroden zueinander zumindest teilweise. Die Trägerstruktur ist ausgebildet, um zumindest einen Teil der Elektrodenanordnung an der Gesichtsoberfläche und hinter dem Ohr zu positionieren. Die Vielzahl von Elektroden der Elektrodenanordnung kann z. B. in eine erste Teilmenge von Elektroden und eine disjunkte zweite Teilmenge von Elektroden aufgeteilt werden, wobei die Trägerstruktur z. B. ausgebildet ist, um die erste Teilmenge von Elektroden an der Gesichtsoberfläche zu positionieren und die zweite Teilmenge von Elektroden hinter dem Ohr zu positionieren.

Das Anbringen 620 der Elektrodenanordnung kann z. B. wie in Zusammenhang mit der Vorrichtung 100 bereits beschrieben erfolgen. Insbesondere sei auf die Ausführungsbeispiele in den Figuren 7, 8a und 8b verwiesen. So können z. B. wie in Fig. 7 dargestellt, fertig hergestellte Nasselektroden auf der Trägerstruktur angeklebt werden oder, wie in den Figuren 8a und 8b dargestellt, die Elektrodenanordnung in die Trägerstruktur integriert werden. Eine Integration der Elektroden in die Trägerstruktur ist insbesondere vorteilhaft, wenn diese mit Leiterbahnen verbunden sind. Eine Sandwichstruktur, wie sie in den Figuren 8a und 8b dargestellt ist, schützt den Nutzer der Vorrichtung, da die Leiterbahnen nicht hautverträglich sind und ist zur elektrischen Isolation der Leiterbahnen notwendig.

Fig. 16 zeigt, eine Trägerstruktur 120, die ausgebildet ist, um die Vielzahl von Elektroden 110₁ bis 110₄ der Elektrodenanordnung 110 nur hinter dem Ohr 230 zu positionieren. Hierfür weist die Trägerstruktur 120 z. B einen Teil 800, wie z. B. einen Bügel, auf, der ausgebildet ist, um auf dem Ohr des Nutzers, bei einem Positionieren und/oder Anbringen der Vorrichtung 100 hinter dem Ohr, aufgestützt zu werden. Dies ermöglicht ein selbstständiges Anbringen der Vorrichtung 100 hinter dem Ohr des Nutzers.

Die Elektroden 110₁ bis 110₄ können z. B. gleiche Merkmale, Positionen und/oder Funktionalitäten wie die Elektroden 110₄ bis 110₇ in Fig. 4a aufweisen. Optional kann an der Elektrode 110₁ und/oder an der Elektrode 110₂ ein Fortsatz 130, wie er z. B. in Zusammenhang mit den Elektroden 110₄ und/oder 110₅ in Fig. 4b dargestellt ist, angeordnet sein. Die Elektroden 110₁ bis 110₄ können z. B. mit Leiterbahnen oder mit Kabeln verbunden sein.

Die Vielzahl von Elektroden 110₁ bis 110₄ der Elektrodenanordnung 110 sind zur Messung von elektrischen Signalen hinter dem Ohr des Nutzers ausgebildet. Bevorzugt weist die Elektrodenanordnung eine Vielzahl von Nasselektroden auf. Die Elektrodenanordnung 110 ist z. B. auf der Trägerstruktur 120 angeordnet oder in dieser integriert.

Die Form der Trägerstruktur 120 folgt z. B. der Form des Ohrs des Nutzers. Die Trägerstruktur 120 definiert zumindest teilweise eine Relativposition der Elektroden der Vielzahl von Elektroden zueinander. Die Form und Länge der Trägerstruktur ist z. B. ausgebildet, um von einem oberen Ohransatz entlang des Ohrs zu einem Mastoid zu führen. Dies ist für ein selbstanlegen der Vorrichtung 100 besonders vorteilhaft, da der Nutzer entweder den oberen Ohransatz oder das Mastoid sehr exakt erfühlen kann und somit die Vorrichtung bzw. die Trägerstruktur 120 hinter dem Ohr positionieren und anbringen kann.

Die Trägerstruktur 120 ist ohne äußere Krafteinwirkung eines Nutzers innerhalb eines Toleranzbereichs formstabil und unter äußerer Krafteinwirkung des Nutzers deformierbar, um einen Abstand zwischen zwei benachbart angeordneten Elektroden zu verändern und/oder sich Gesichts- und Kopfstrukturen flächendeckend anzupassen.

Ferner ist anzumerken, dass die Vorrichtung 100 Merkmale und Funktionalitäten, wie sie in Zusammenhang mit anderen Ausführungsbeispielen der erfindungsgemäßen Vorrichtung 100 hierein beschrieben worden sind, aufweisen kann, siehe z. B. Fig. 1a bis Fig. 9e. Insbesondere kann die Trägerstruktur 120 z. B. Fortsätze 130 an Elektroden der Vielzahl von Elektroden aufweisen. Ferner kann die Trägerstruktur 120 unter anderem Merkmale wie Klebematerial, Schutzfolien und Versteifungselemente aufweisen.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein. Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele als Computerprogrammprodukt mit einem Programmcode implementiert sein, wobei der Programmcode dahingehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere nicht beanspruchte Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein nicht beanspruchtes Ausführungsbeispiel Verfahrens somit ein Computerprogramm, das einen Programmcode zum Durchführen eines der hierin beschriebenen Verfahren aufweist, wenn das Computerprogramm auf einem Computer abläuft.

Ein weiteres nicht beanspruchtes Ausführungsbeispiel der Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist. Der Datenträger, das digitale Speichermedium oder das computerlesbare Medium sind typischerweise gegenständlich und/oder nichtvergänglich bzw. nichtvorübergehend.

Ein weiteres nicht beanspruchtes Ausführungsbeispiel des Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahingehend konfiguriert sein, über eine Datenkommunikationsverbindung, beispielsweise über das Internet, transferiert zu werden.

Ein weiteres nicht beanspruchtes Ausführungsbeispiel umfasst eine Verarbeitungseinrichtung, beispielsweise einen Computer oder ein programmierbares Logikbauelement, die dahingehend konfiguriert oder angepasst ist, eines der hierin beschriebenen Verfahren durchzuführen.

Ein weiteres nicht beanspruchtes Ausführungsbeispiel umfasst einen Computer, auf dem das Computerprogramm zum Durchführen eines der hierin beschriebenen Verfahren installiert ist.

Ein weiteres nicht beanspruchtes Ausführungsbeispiel umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die hierin beschriebenen Vorrichtungen können beispielsweise unter Verwendung eines Hardware-Apparats, oder unter Verwendung eines Computers, oder unter Verwendung einer Kombination eines Hardware-Apparats und eines Computers implementiert werden.

Die hierin beschriebenen Vorrichtungen, oder jedwede Komponenten der hierin beschriebenen Vorrichtungen können zumindest teilweise in Hardware und/oder in Software (Computerprogramm) implementiert sein.

Die hierin beschriebenen Verfahren können beispielsweise unter Verwendung eines Hardware-Apparats, oder unter Verwendung eines Computers, oder unter Verwendung einer Kombination eines Hardware-Apparats und eines Computers implementiert werden.

Die hierin beschriebenen Verfahren, oder jedwede Komponenten der hierin beschriebenen Verfahren können zumindest teilweise durch Hardware und/oder durch Software ausgeführt werden.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Vorrichtung (100) mit:
einer Elektrodenanordnung (110) mit einer Vielzahl von Elektroden zur Messung von elektrischen Signalen an einer Gesichtsoberfläche (220) eines Kopfes (200) eines Nutzers (210) und hinter einem Ohr (230) des Nutzers (210), wobei es sich bei den Elektroden der Vielzahl von Elektroden um Nasselektroden und/oder Trockenelektroden handelt;
einer Trägerstruktur (120);
wobei die Trägerstruktur (120) eine Relativposition der Elektroden zueinander zumindest teilweise definiert; und
wobei die Trägerstruktur (120) ohne äußere Krafteinwirkung eines Nutzers (210) innerhalb eines Toleranzbereichs formstabil ist und unter äußerer Krafteinwirkung des Nutzers (210) deformierbar ist, um einen Abstand zwischen zwei benachbart angeordneten Elektroden zu verändern und sich Gesichts- und Kopfstrukturen flächendeckend anzupassen,
wobei die Trägerstruktur (120) ausgebildet ist, um zumindest einen Teil der Elektrodenanordnung (110) an der Gesichtsoberfläche (220) und hinter dem Ohr (230) zu positionieren;
wobei die Trägerstruktur (120) in Form einer Netzstruktur mit sich verzweigenden Verbindungsstegen ausgebildet ist, die einem Verlauf der Gesichtsoberfläche (220) folgen; und
wobei die verzweigten Verbindungsstege eine Stegbreite aufweisen, die höchstens einem dreifachen Elektrodendurchmesser einer Elektrode der Vielzahl von Elektroden entspricht.

2. Vorrichtung (100) nach Anspruch 1, wobei die Trägerstruktur (120) der Gesichtsoberfläche (220) als einer menschlichen Gesichtsform folgt und die Vielzahl von Elektroden auf der Trägerstruktur (120) angeordnet sind;
wobei eine erste Elektrodenposition, die einer ersten Elektrode (110₁) der Vielzahl von Elektroden zugeordnet ist, mit einer Position eines ersten markanten Punktes (B) der Gesichtsoberfläche (220) übereinstimmt; und eine zweite Elektrodenposition, die einer zweiten Elektrode (110₂) der Vielzahl von Elektroden zugeordnet ist, mit einer Position eines zweiten markanten Punktes (A) der Gesichtsoberfläche (220) übereinstimmt; und sich eine dritte Elektrodenposition, die einer dritten Elektrode (110₃) der Vielzahl von Elektroden zugeordnet ist, ausgehend von der ersten Elektrodenposition und der zweiten Elektrodenposition an der Gesichtsoberfläche (220) innerhalb eines durch die Trägerstruktur (120) bestimmten Toleranzbereichs ergibt.

3. Vorrichtung (100) nach Anspruch 2, wobei der erste markante Punkt (B) der Gesichtsoberfläche (220) einem oberen Wangenknochen entspricht und der zweite markante Punkt (A) einer Position vor dem Tragus eines Ohrs (230) des Nutzers (210) entspricht; und
wobei die Trägerstruktur (120) einen Verbindungssteg (123, 124₁) umfasst, der die erste Elektrodenposition und/oder die zweite Elektrodenposition einerseits mit der dritten Elektrodenposition andererseits zumindest teilweise verbindet und wobei die dritte Elektrodenposition innerhalb eines durch die Trägerstruktur (120) bestimmten Toleranzbereichs von 1 bis 2 cm mit einer Position (C) auf einer Stirn der Gesichtsoberfläche (220) in einem Bereich von 1 cm bis 3 cm oberhalb der Augenbrauenlinie senkrecht oberhalb eines Nasions des Nutzers (210) übereinstimmt; und/oder
wobei die Trägerstruktur (120) einen Verbindungssteg (123, 124₂) umfasst, der die erste Elektrodenposition und/oder die zweite Elektrodenposition einerseits mit einer vierten Elektrodenposition, die einer vierten Elektrode (110₄) der Vielzahl von Elektroden zugeordnet ist, andererseits zumindest teilweise verbindet und wobei die vierte Elektrodenposition innerhalb eines durch die Trägerstruktur (120) bestimmten Toleranzbereichs mit einer Position (D) hinter dem Ohr (230) des Nutzers (210) übereinstimmt; und/oder
wobei die Trägerstruktur (120) einen Verbindungssteg (126) umfasst, der die erste Elektrodenposition und/oder die zweite Elektrodenposition einerseits mit einer fünften Elektrodenposition, die einer fünften Elektrode (110₉; 110₈) der Vielzahl von Elektroden zugeordnet ist, andererseits zumindest teilweise verbindet und wobei die fünfte Elektrodenposition innerhalb eines durch die Trägerstruktur (120) bestimmten Toleranzbereichs mit einer Position (E) auf einem Kinn der Gesichtsoberfläche (220) des Nutzers (210) übereinstimmt.

4. Vorrichtung (100) nach einem der vorangehenden Ansprüche, bei der die Trägerstruktur (120) ein Klebematerial zur Fixierung der Vielzahl von Elektroden und der Trägerstruktur (120) an der Gesichtsoberfläche (220) aufweist,
bei der ferner die Elektroden ein klebendes Elektrolytgel zur Senkung der Impedanz und zur Fixierung der Vielzahl von Elektroden und der Trägerstruktur (120) an der Gesichtsoberfläche (220) aufweisen; wobei das klebende Elektrolytgel zumindest bereichsweise von einer lösbaren Schutzfolie bedeckt ist, und
bei der das Klebematerial von der lösbaren Schutzfolie bedeckt ist,
wobei die Schutzfolie eine Mehrzahl von Schutzfolienabschnitte aufweist, die unabhängig voneinander von der Trägerstruktur (120) und der Vielzahl von Elektroden lösbar sind.

5. Vorrichtung (100) nach einem der Ansprüche 1 bis 4, wobei ein Verbindungssteg (124₂) der sich verzweigenden Verbindungsstege an einem Ende gebogen ist, so dass die Trägerstruktur (120) ein gebogenes Endstück aufweist, das ausgebildet ist, um hinter einem Ohr (230) des Nutzers (210) angebracht zu werden und wobei der Verbindungssteg ausgebildet ist, um von dem Gesicht des Nutzers (210) oberhalb des Ohrs (230) zu dem gebogenen Endstück zu verlaufen und wobei an dem gebogenen Endstück zumindest eine Elektrode der Vielzahl von Elektroden angeordnet ist.

6. Vorrichtung (100) nach einem der Ansprüche 1 bis 5, wobei eine Form der Trägerstruktur (120) ausgebildet ist, um die Vielzahl von Elektroden an einer korrespondierenden Vielzahl von Positionen an der Gesichtsoberfläche (220) zu positionieren; wobei die Vielzahl von Positionen für eine Erfassung der elektrischen Signale an der Gesichtsoberfläche (220) für zumindest eine Linearkombination der elektrischen Signale zum Abbilden von Signalen aus einer vorbestimmten Kopfregion des Nutzers (210) eingerichtet ist.

7. Vorrichtung (100) gemäß einem der vorangehenden Ansprüche, bei der die Trägerstruktur (120) als Trägermaterial Polyurethanmaterial, Polymermaterial, und/oder Silikonmaterial aufweist,
wobei das Trägermaterial elastisch oder dehnbar ist;
wobei die Elektroden mit Leiterbahnen verbunden sind, und
wobei die Leiterbahnen einen geschlungenen Verlauf an oder in der Trägerstruktur (120) aufweisen, um sich bei Dehnung der Trägerstruktur (120) korrespondierend zu längen, und
wobei die Trägerstruktur (120) ausgebildet ist, um einen Abstand zwischen zwei Elektroden der Elektrodenanordnung (110) um maximal 10 % zu verlängern.

8. Vorrichtung (100) gemäß einem der vorangehenden Ansprüche, wobei die Trägerstruktur (120) auf einer der Elektrodenanordnung (110) abgewandten Seite ein lösbares Versteifungselement (122) aufweist, das ausgebildet ist, um die Trägerstruktur (120) zumindest lokal zu versteifen.

9. Vorrichtung (100) gemäß einem der vorangehenden Ansprüche, wobei die Trägerstruktur (120) an einer Position, an der eine Elektrode der Elektrodenanordnung (110) angeordnet ist, zumindest einen Fortsatz (130) mit Klebematerial aufweist.

10. Vorrichtung (100) gemäß einem der vorangehenden Ansprüche, wobei die Vielzahl von Elektroden aktive Elektroden aufweisen, die einen Schaltkreis aufweisen, der ausgebildet ist, um
eine Impedanzwandlung des gemessenen elektrischen Signals durchzuführen, und/oder
das gemessene elektrische Signal zu verstärken.

11. System (500) mit:
einer Vorrichtung (100) nach einem der Ansprüche 1 bis 10; und
einer Auswerteanordnung (510), die ausgebildet ist, um die von der Vielzahl von Elektroden gemessenen elektrischen Signale auszuwerten.

12. System (500) nach Anspruch 11, wobei die Auswerteanordnung (510) ausgebildet ist, um basierend auf den elektrischen Signalen
eine Information (520) über eine Schlafarchitektur des Nutzers (210) bereitzustellen; oder
eine Information (520) über ein epileptisches Verhalten des Nutzers (210) bereitzustellen; oder
eine neurologische Erkrankung des Nutzers (210) anzuzeigen (520); oder eine kognitionspsychologische Information (520) bereitzustellen.

13. System (500) nach Anspruch 11, mit
einem Hörgerät (530);
wobei die Auswerteanordnung (510) ausgebildet ist, um basierend auf den elektrischen Signalen das Hörgerät (530) zu steuern,
wobei die Auswerteanordnung (510) ausgebildet ist, um die von der Vorrichtung (100) gemessenen Signale mit einer Einhüllenden eines von dem Hörgerät (530) aufgenommenen Audiosignals zu korrelieren, um einen Strahlformer des Hörgeräts (530) zu steuern.

14. Verfahren (400) zur Verwendung der Vorrichtung (100) nach einem der Ansprüche 1 bis 10, mit den Schritten:
Anlegen (410) der Vorrichtung (100) an einer Gesichtsoberfläche (220) eines Kopfes (200) eines Nutzers (210) durch den Nutzer selbst oder einer anderen Person;
Überwachen (420) des Nutzers (210) anhand der von der Vielzahl von Elektroden gemessenen elektrischen Signale.

15. Verfahren (400) nach Anspruch 14, wobei das Anlegen der Vorrichtung (100) an der Gesichtsoberfläche (220) des Kopfes (200) des Nutzers (210) die folgenden Schritte aufweist:
ertasten eines oberen Wangenknochens und anbringen einer ersten Elektrode an dem oberen Wangenknochen;
anbringen einer zweiten Elektrode vor dem Tragus eines Ohres (230) des Nutzers (210);
anbringen einer dritten Elektrode an einer Stirn senkrecht oberhalb eines Nasion in einem Bereich von 1-3 cm über einer Augenbrauenlinie;
anbringen einer vierten Elektrode hinter dem Ohr (230).

## Claims

1. Device (100) comprising:
an electrode arrangement (110) with a plurality of electrodes for measuring electric signals on a face surface (220) of a head (200) of a user (210) and behind an ear (230) of the user (210), wherein the electrodes of the plurality of electrodes are wet electrodes and/or dry electrodes ;
a carrier structure (120);
wherein the carrier structure (120) at least partially defines a relative position of the electrodes to one another; and
wherein the carrier structure (120) is shape-stable within a tolerance range without external force exerted by a user (210) and deformable under external force exerted by the user (210), in order to change a distance between two adjacently arranged electrodes and to area-comprehensively adapt to face and head structures,
wherein the carrier structure (120) is configured to position at least a part of the electrode arrangement (110) on the face surface (220) and behind the ear (230);
wherein the carrier structure (120) is configured in the shape of a net structure with branching connecting ribs that follow a course of the face surface (220); and
wherein the branching connecting ribs comprise a rib width corresponding to, at most, three times the electrode diameter of an electrode of the plurality of the electrodes.

2. Device (100) according to claim 1, wherein the carrier structure (120) follows the face surface (220) as a human face shape, and the plurality of electrodes are arranged on the carrier structure (120);
wherein a first electrode position that is assigned to a first electrode (110₁) of the plurality of electrodes corresponds to a position of a first distinctive point (B) of the face surface (220); and a second electrode position that is assigned to a second electrode (110₂) of the plurality of electrodes corresponds to a position of a second distinctive point (A) of the face surface (220); and a third electrode position that is assigned to a third electrode (110₃) of the plurality of electrodes results starting from the first electrode position and the second electrode position on the face surface (220) within a tolerance range determined by the carrier structure (120).

3. Device (100) according to claim 2, wherein the first distinctive point (B) of the face surface (220) corresponds to an upper cheekbone, and the second distinctive point (A) corresponds to a position in front of the tragus of an ear (230) of the user (210); and
wherein the carrier structure (120) includes a connecting rib (123, 124₁) that at least partially connects the first electrode position and/or the second electrode position, on one side, to the third electrode position, on the other side, and wherein the third electrode position corresponds, within a tolerance range of 1 to 2 cm determined by the carrier structure (120), to a position (C) on a forehead of the face surface (220) in an area of 1 cm to 3 cm above the eyebrow line, vertically above a nasion of the user (210); and/or
wherein the carrier structure (120) includes a connecting rib (123, 124₁) that at least partially connects the first electrode position and/or the second electrode position, on one side, to a fourth electrode position that is assigned to a fourth electrode (110₄) of the plurality of electrodes, on the other side, and wherein the fourth electrode position corresponds, within a tolerance range determined by the carrier structure (120), to a position (D) behind the ear (230) of the user (210); and or
wherein the carrier structure (120) includes a connecting rib (126) that at least partially connects the first electrode position and/or the second electrode position, on one side, to a fifth electrode position that is assigned to a fifth electrode (110₉; 110₈) of the plurality of electrodes, on the other side, and wherein the fifth electrode position corresponds, within a tolerance range determined by the carrier structure (120), to a position (E) on the chin of the face surface (220) of the user (210).

4. Device (100) according to one of the preceding claims, wherein the carrier structure (210) comprises an adhesive material for affixing the plurality of electrodes and the carrier structure (120) on the face surface (220),
wherein the electrodes further comprise an adhesive electrolyte gel for lowering the impedance and for affixing the plurality of electrodes and the carrier structure (120) on the face surface (220); wherein the adhesive electrolyte gel is covered, at least in some sections, by a removable protective film, and
wherein the adhesive material is covered by a removable protective film,
wherein the protective film comprises a plurality of protective film portions that are independently removable from the carrier structure (120) and the plurality of electrodes.

5. Device (100) according to any one of claims 1 to 4, wherein a connecting rib (124₂) of the branching connecting rib is curved at one end so that the carrier structure (120) comprises a curved end piece that is configured to be affixed behind an ear (230) of the user (210), and wherein the connecting rib is configured to run from the face of the user (210) above the ear (230) to the curved end piece, and wherein at least one electrode of the plurality of electrodes is arranged at the curved end piece.

6. Device (100) according to any one of claims 1 to 5, wherein a shape of the carrier structure (120) is configured to position the plurality of electrodes at a corresponding plurality of positions on the face surface (220); wherein the plurality of positions is configured for a detection of the electric signals on the face surface (220) for at least one linear combination of the electric signals for mapping signals from a predetermined head region of the user (210).

7. Device (100) according to one of the preceding claims, wherein the carrier structure (120) comprises polyurethane material, polymer material, and/or silicone material as a carrier material,
wherein the carrier material is elastic or stretchable;
wherein the electrodes are connected to conductive traces, and
wherein the conductive traces have a slung course on or in the carrier structure (120) in order to correspondingly extend upon stretching of the carrier structure (120), and
wherein the carrier structure (120) is configured to extend a distance between two electrodes of the electrode arrangement (110) by a maximum of 10 %.

8. Device (100) according to one of the preceding claims, wherein on a side facing away from the electrode arrangement (110), the carrier structure (120) comprises a removable stiffening element (122) that is configured to at least locally stiffen the carrier structure (120).

9. Device (100) according to one of the preceding claims, wherein the carrier structure (120) comprises at least one extension (130) having an adhesive material at a position at which an electrode of the electrode arrangement (110) is arranged.

10. Device (100) according to one of the preceding claims, wherein the plurality of electrodes comprises active electrodes comprising a circuit that is configured to
perform an impedance conversion of the measured electric signal, and/or
amplify the measured electric signal.

11. System (500) comprising:
a device (100) according to any one of claims 1 to 10; and
an evaluation arrangement (510) that is configured to evaluate the electric signals measured by the plurality of electrodes.

12. System (500) according to claim 11, wherein the evaluation arrangement (510) is configured to, based on the electric signals
provide an information (520) on a sleep architecture of the user (210); or
provide an information (520) on an epileptic behavior of the user (210); or
indicate (520) a neurological disorder of the user (210); or
provide a cognitive psychological information (520).

13. System (500) according to claim 11, comprising
a hearing aid (530);
wherein the evaluation arrangement (510) is configured to control the hearing aid (530) based on the electric signals,
wherein the evaluation arrangement (510) is configured to correlate the signals measured by the device (100) with an envelope of an audio signal recorded by the hearing aid (530) in order to control a beam former of the hearing aid (530).

14. Method (400) for using the device (100) according to any one of claims 1 to 10, comprising the steps of:
applying (410) the device (100) on a face surface (220) of a head (200) of a user (210) by the user themselves or by another person;
monitoring (420) the user (210) by means of the electric signals measured by the plurality of electrodes.

15. Method (400) according to claim 14, wherein the application of the device (100) on the face surface (220) of the head (200) of the user (210) comprises the following steps:
feeling an upper cheekbone and affixing a first electrode on the upper cheekbone;
affixing a second electrode in front of the tragus of an ear (230) of the user (210);
affixing a third electrode on a forehead, vertically above a nasion, in an area of 1-3 cm above an eyebrow line;
affixing a fourth electrode behind the ear (230).

## Revendications

1. Dispositif (100) comprenant:
un agencement d'électrodes (110) comprenant une pluralité d'électrodes pour mesurer des signaux électriques sur une surface faciale (220) d'une tête (200) d'un utilisateur (210) et derrière une oreille (230) de l'utilisateur (210), les électrodes de la pluralité d'électrodes étant des électrodes humides et/ou des électrodes sèches ;
une structure porteuse (120) ;
dans lequel la structure porteuse (120) définit au moins en partie une position relative des électrodes entre elles ; et
dans lequel la structure porteuse (120) est stable de forme dans une plage de tolérance sans application d'une force extérieure par un utilisateur (210) et est déformable sous l'application d'une force extérieure par l'utilisateur (210) afin de modifier une distance entre deux électrodes disposées de manière adjacente et de s'adapter aux structures du visage et de la tête sur toute leur étendue,
dans lequel la structure porteuse (120) est formée pour positionner au moins une partie de l'agencement d'électrodes (110) sur la surface faciale (220) et derrière l'oreille (230) ;
dans lequel la structure porteuse (120) est réalisée sous la forme d'une structure en maillage avec des entretoises de connexion ramifiées qui suivent un tracé de la surface faciale (220) ; et
dans lequel les entretoises de connexion ramifiées présentent une largeur d'entretoise au plus égale à trois fois le diamètre d'une électrode de la pluralité d'électrodes.

2. Dispositif (100) selon la revendication 1, dans lequel la structure porteuse (120) suit la surface faciale (220) selon une forme de visage humain et dans lequel la pluralité d'électrodes est disposée sur la structure porteuse (120) ;
dans lequel une première position d'électrode, qui est associée à une première électrode (110₁) de la pluralité d'électrodes, coïncide avec une position d'un premier point remarquable (B) de la surface faciale (220) ; et une deuxième position d'électrode, qui est associée à une deuxième électrode (110₂) de la pluralité d'électrodes, coïncide avec une position d'un deuxième point remarquable (A) de la surface faciale (220) ; et une troisième position d'électrode, qui est associée à une troisième électrode (110₃) de la pluralité d'électrodes, s'obtient à partir de la première position d'électrode et de la deuxième position d'électrode sur la surface faciale (220) dans une plage de tolérance déterminée par la structure porteuse (120).

3. Dispositif (100) selon la revendication 2, dans lequel le premier point remarquable (B) de la surface faciale (220) correspond à une pommette supérieure et le deuxième point remarquable (A) correspond à une position devant le tragus de l'oreille (230) de l'utilisateur (210) ; et
dans lequel la structure de support (120) comprend une entretoise de connexion (123, 124₁) qui relie au moins partiellement la première position d'électrode et/ou la deuxième position d'électrode, d'une part, à la troisième position d'électrode, d'autre part, et dans lequel la troisième position d'électrode coïncide, dans une plage de tolérance de 1 à 2 cm déterminée par la structure de support (120), avec une position (C) sur un front de la surface faciale (220) dans une zone de 1 cm à 3 cm au-dessus de la ligne des sourcils, à la verticale au-dessus du nasion de l'utilisateur (210) ; et/ou
dans lequel la structure porteuse (120) comprend une entretoise de connexion (123, 124₂) qui relie au moins partiellement la première position d'électrode et/ou la deuxième position d'électrode, d'une part, à une quatrième position d'électrode qui est associée à une quatrième électrode (110₄) de la pluralité d'électrodes, d'autre part, et dans lequel la quatrième position d'électrode coïncide, dans une plage de tolérance déterminée par la structure porteuse (120), avec une position (D) derrière l'oreille (230) de l'utilisateur (210) ; et/ou
dans lequel la structure porteuse (120) comprend une entretoise de connexion (126) qui relie au moins partiellement la première position d'électrode et/ou la deuxième position d'électrode, d'une part, à une cinquième position d'électrode qui est associée à une cinquième électrode (110₉ ; 110₈) de la pluralité d'électrodes, d'autre part, et dans lequel la cinquième position d'électrode coïncide, dans une plage de tolérance déterminée par la structure porteuse (120), avec une position (E) sur un menton de la surface faciale (220) de l'utilisateur (210).

4. Dispositif (100) selon l'une des revendications précédentes, dans lequel la structure porteuse (120) comporte un matériau adhésif destiné à fixer la pluralité d'électrodes et la structure porteuse (120) sur la surface faciale (220),
dans lequel les électrodes comportent en outre un gel électrolytique adhésif destiné à réduire l'impédance et à fixer la pluralité d'électrodes et la structure porteuse (120) sur la surface faciale (220) ; dans lequel le gel électrolytique adhésif est recouvert au moins par endroits d'un film protecteur amovible, et
dans lequel le matériau adhésif est recouvert par le film protecteur amovible,
dans lequel le film protecteur comporte une pluralité de sections de film protecteur, lesquelles sont détachables indépendamment les unes des autres de la structure porteuse (120) et de la pluralité d'électrodes.

5. Dispositif (100) selon l'une des revendications 1 à 4, dans lequel une entretoise de connexion (124₂) parmi les entretoises de connexion ramifiées est courbée à une extrémité, de sorte que la structure porteuse (120) comporte une partie d'extrémité courbée, qui est formée pour être placée derrière une oreille (230) de l'utilisateur (210) et dans lequel l'entretoise de connexion est formée pour s'étendre depuis le visage de l'utilisateur (210) au-dessus de l'oreille (230) jusqu'à la partie d'extrémité courbée et dans lequel au moins une électrode de la pluralité d'électrodes est disposée sur la partie d'extrémité courbée.

6. Dispositif (100) selon l'une des revendications 1 à 5, dans lequel une forme de la structure porteuse (120) est configurée pour positionner la pluralité d'électrodes à une pluralité correspondante de positions sur la surface faciale (220) ; la pluralité de positions étant agencée pour l'acquisition des signaux électriques sur la surface faciale (220) pour au moins une combinaison linéaire des signaux électriques afin de représenter des signaux provenant d'une région de la tête prédéterminée de l'utilisateur (210).

7. Dispositif (100) selon l'une des revendications précédentes, dans lequel la structure porteuse (120) comporte, comme matériau de support, un matériau en polyuréthane, un matériau polymère, et/ou un matériau en silicone,
dans lequel le matériau de support est élastique ou extensible ;
dans lequel les électrodes sont reliées à des pistes conductrices, et
dans lequel les pistes conductrices présentent un tracé sinueux sur ou dans la structure porteuse (120) afin de s'allonger de manière correspondante lors d'un étirement de la structure porteuse (120), et
dans lequel la structure porteuse (120) est formée pour augmenter une distance entre deux électrodes de l'agencement d'électrodes (110) de 10 % au maximum.

8. Dispositif (100) selon l'une des revendications précédentes, dans lequel la structure porteuse (120) comporte, sur un côté opposé à l'agencement d'électrodes (110), un élément de rigidification amovible (122) qui est formé pour rigidifier au moins localement la structure porteuse (120).

9. Dispositif (100) selon l'une des revendications précédentes, dans lequel la structure porteuse (120) comporte au moins un prolongement (130) avec un matériau adhésif à une position à laquelle une électrode de l'agencement d'électrodes (110) est disposée.

10. Dispositif (100) selon l'une des revendications précédentes, dans lequel la pluralité d'électrodes comporte des électrodes actives qui comportent un circuit formé pour effectuer une conversion d'impédance du signal électrique mesuré, et/ou
amplifier le signal électrique mesuré.

11. Système (500) comprenant:
un dispositif (100) selon l'une des revendications 1 à 10 ; et
un dispositif d'évaluation (510) formé pour évaluer les signaux électriques mesurés par la pluralité d'électrodes.

12. Système (500) selon la revendication 11, dans lequel le dispositif d'évaluation (510) est formé pour, sur la base des signaux électriques
fournir une information (520) sur une architecture du sommeil de l'utilisateur (210) ; ou
fournir une information (520) sur un comportement épileptique de l'utilisateur (210) ; ou
indiquer (520) une maladie neurologique de l'utilisateur (210) ; ou fournir une information cognitivo-psychologique (520).

13. Système (500) selon la revendication 11, comprenant
un appareil auditif (530) ;
dans lequel le dispositif d'évaluation (510) est formé pour commander, sur la base des signaux électriques, l'appareil auditif (530),
dans lequel le dispositif d'évaluation (510) est formé pour corréler les signaux mesurés par le dispositif (100) avec une enveloppe d'un signal audio capté par l'appareil auditif (530) afin de commander un dispositif de formation de faisceau de l'appareil auditif (530).

14. Procédé (400) d'utilisation du dispositif (100) selon l'une des revendications 1 à 10, comprenant les étapes suivantes :
Mise en place (410) du dispositif (100) sur une surface faciale (220) d'une tête (200) d'un utilisateur (210) par l'utilisateur lui-même ou par une autre personne ;
Surveillance (420) de l'utilisateur (210) au moyen des signaux électriques mesurés par la pluralité d'électrodes.

15. Procédé (400) selon la revendication 14, dans lequel la mise en place du dispositif (100) sur la surface faciale (220) de la tête (200) de l'utilisateur (210) comporte les étapes suivantes:
palpation d'une pommette supérieure et mise en place d'une première électrode sur la pommette supérieure ;
mise en place d'une deuxième électrode devant le tragus d'une oreille (230) de l'utilisateur (210) ;
mise en place d'une troisième électrode sur un front à la verticale au-dessus du nasion dans une zone de 1-3 cm au-dessus d'une ligne des sourcils ;
mise en place d'une quatrième électrode derrière l'oreille (230).
